# EUROPÄISCHE PATENTSCHRIFT

(11) **EP 3 921 045 B1**
(45) Veröffentlichungstag und Bekanntmachung des Hinweises auf die Patenterteilung: **22.07.2026**
(21) Anmeldenummer: 19829633.7
(22) Anmeldetag: 23.12.2019
(51) Int. Cl.: A63B 23/03, A61F 5/00, A61C 7/08

(54) **BIOMECHANISCHE TRAININGSVORRICHTUNG FÜR DAS KIEFERGELENK**
BIOMECHANICAL TRAINING DEVICE FOR THE TEMPOROMANDIBULAR JOINT
DISPOSITIF D'ENTRAÎNEMENT BIOMÉCANIQUE POUR L'ARTICULATION MAXILLAIRE

(30) Priorität: 04.01.2019 DE 102019000008; 11.03.2019 DE 102019106170
(43) Veröffentlichungstag der Anmeldung: 15.12.2021
(73) Patentinhaber: Forstgarten International Holding GmbH, 9016 St. Gallen (CH)
(72) Erfinder: SAXLER, Frank, 07160 Paguera (ES); HORNUNG, Frank, 97779 Geroda (DE)
(74) Vertreter: Schmidt, Christian
(86) Internationale Anmeldenummer: PCT/EP2019/086969
(87) Internationale Veröffentlichungsnummer: WO 2020/141134

(56) Entgegenhaltungen:
- EP-A1- 2 630 938
- WO-A2-2014/044783
- DE-T5- 112009 001 742
- US-A1- 2015 210 014

## Beschreibung

### Gebiet der Erfindung

Die Erfindung betrifft eine individuell gefertigte Vorrichtung zum Training von physiologisch und biomechanisch vorteilhaften Kieferpositionen und Kieferbewegungen, insbesondere für dentalmedizinische und kieferorthopädische Anwendungen. Die Vorrichtung besteht aus einem Oberkiefer-Modul für den Zahnbogen des Oberkiefers und einem Unterkiefer-Modul für den Zahnbogen des Unterkiefers. Die Vorrichtung wird in den Mund des Trainierenden eingesetzt und erlaubt eine in zwei Dimensionen nach vorne und seitlich gleitende Bewegung des Unterkiefers. Dabei besteht eine fast reibungsfrei plane und vorwiegend genau transversal gleitend bewegliche Trennfläche zwischen Oberkiefer-Modul und Unterkiefer-Modul. Der Unterkiefer kann also durch die seitwärts zeigende Kraftkomponente bewegt werden und die Senkrecht auf der Trennfläche stehende Kraftkomponente der Beißkraft drückt die beiden Module in der Trennfläche gegeneinander. Das Anwendungsgebiet der vorliegenden Erfindung ist insbesondere das biomechanische neuromuskuläre Training der Muskulatur, die den Unterkiefer relativ zum Oberkiefer bewegt.

### Stand der Technik

In der Patentschrift DE102012220054 A1 (Dirk Wiechmann, 2012) ist eine kieferorthopädische Vorrichtung zur Verlagerung des Unterkiefers eines Patienten beschrieben. Sie benutzt zwei Teleskope, um eine Kraft transversal auszuüben, die zur Verlagerung führt. Solche Vorrichtungen haben den eklatanten Nachteil, dass sie biomechanisch und neurophysiologisch sehr ungünstige Bedingungen im Kiefer schaffen, selbst wenn sie eine kosmetisch schöne Position der Zähne erzeugen. Die Folge sind schwere Störungen in der Biomechanik und in der Neurophysiologie der Kiefermuskulatur und des Kiefergelenks, bis hin zu massiven Beschwerden und degenerativen Erkrankungen.

EP1147745A2 (Thomas Egli) nennt eine Vorrichtung, die transversale Verlagerungen ermöglicht. Die Kräfte wirken allerdings dehnend auf die seitlichen Bereiche des oberen Zahnbogens und des Oberkiefers und werden deshalb zur Gaumenerweiterung vorgeschlagen. Dabei geht es darum, eine ungenügende Breite des Gaumens kieferorthopädisch zu korrigieren. Die Vorrichtung ist zwar mindestens zweiteilig, eine biomechanische und neurophysiologische Verbesserung ist damit aber nicht erreichbar.

In der US 2018 / 0 078 344 A1 wird eine Schiene mit gleitfähiger Auflagefläche gezeigt, in der es zum Kontakt zwischen OK teil und UK Teil kommt. Die Gleitebene richtet sich aber in keiner weise nach der cranialen Symmetrie sondern nach den dentalen Gegebenheiten im zahnbogen. Insbesondere ist die Gleitebene nicht wegen der cranialen Orientierung relativ zur Kauebene geneigt. Die Schiene sitzt zudem fest auf den Zähnen auf und erzeugt einen seitlichen Druck auf die Zähne. Auf diese Weise wird es unmöglich gemacht, dass der therapeutisch wirksame Druckreiz auf den Zahnbogen in der korrekten cranialen Orientierung ausgeübt wird.

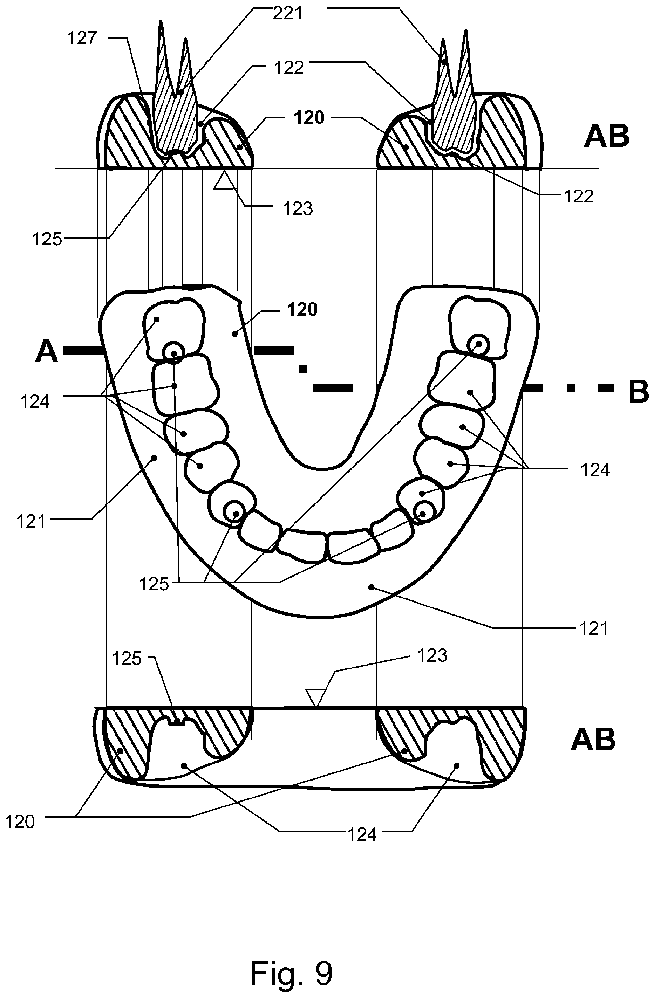

In der EP 3 332 731 A1 wird eine Orientierung an der Innenohrachse vorgeschrieben, doch diese Transversalachse rechts-links allen definiert nicht die genaue Lage der Trennebene in ihrer Schrägneigung von vorne und von der Seite gesehen. Ebenso wenig ergibt sich daraus die Lage der Auflagepunkte 125.1 rechts links bzw. 125.2 rechts links für die Auflagebereiche 125 (siehe Figur 9, vorliegende Anmeldung). Das Dokument nennt die Ausführung von Aligner-Schienen auf die Weise, dass diese eine seitlich wirkende Kraft ausüben um die Zähne des Zahnbogens in ihrer gedachten Transversalachse mehr und mehr in die Orientierung der Innenohrachse bringen. Im Gegensatz dazu benutzt die erfindungsgemäße Vorrichtung einen Randspalt um genau diese seitwärts wirkenden Kräfte auf die Zähne zu vermeiden.

Die Veröffentlichung von H.J. Schindler et al ZEFQ, 2013, Vol. 107, Issue 4-5, Page 297-301 mit dem Titel: "Therapie von Kaumuskelschmerzen mit Okklusionsschienen" zeigt einen Überblick über den Wissensstand der Therapie mit oralen Schienen. Es gibt eine Vielzahl von oralen Schienen, die einen veränderten Abstand der Gelenkknorren im Kiefergelenk herstellen, insbesondere um komprimierte Kiefergelenksseiten zu entlasten. Dabei fehlt jedoch eine Trainingsvorrichtung für die muskulär stabilisierte Transversalbewegung, die die neuromuskuläre Steuerung der Kiefermuskulatur mit hoher Effizienz verbessern kann.

DE 112009001742 T5 betrifft eine verbesserte Unterkieferprotrusionsschiene.

EP 2630938 A1 betrifft eine Vorrichtung zur Verbesserung der Atmung einer Person.

US 2015210014 A1 betrifft die Herstellung von Kiefergelenkschienen.

WO 2014044783 A2 betrifft ein Verfahren zur Simulation der dynamischen Okklusion.

Die bisher üblichen Aufbiss-Schienen, die bei Kiefergelenksverspannungen verordnet werden, haben die ungünstige Eigenschaft, dass sie dauerhaft getragen werden und das kraftvolle dauerhafte Aufbeißen sogar provozieren können. Deshalb klagen Patienten beim Tragen dieser Aufbiss-Schienen oftmals über noch stärkere neuromuskuläre Verspannungen. Die biomechanische und neurophysiologische Funktion der bisher verfügbaren Vorrichtungen ist also sehr unzureichend.

All den bisher vorgeschlagenen Vorrichtungen und Lösungen fehlt der entscheidende Aspekt, die neuromuskuläre Steuerung der Kiefergelenksmuskulatur auf geeignete Weise vorteilhaft und rasch wirkend beeinflussen zu können. Zudem wird durch die Vorrichtungen nach dem bisherigen Stand der Technik die Relativposition des Unterkiefers zum Oberkiefer nicht in der geeigneten Weise neurophysiologisch neu ausgerichtet und stabilisiert. Vielmehr findet oftmals sogar eine Fortsetzung der biomechanisch und neurophysiologisch ungünstigen Situation statt. Deshalb sind die therapeutischen Erfolge durch orale Schienen oder durch andere vorübergehend oder dauerhaft zu tragende Vorrichtungen nur sehr unzureichend.

### Aufgabe der Erfindung

Die Aufgabe der vorliegenden Erfindung ist es, eine schnelle, schonende und nachhaltig wirkende Trainingsvorrichtung bereit zu stellen, um neurophysiologische, biomechanische und kieferorthopädische Verbesserungen zu erreichen. Dabei soll die Vorrichtung ein zeitlich eingegrenztes Training von einigen Minuten ermöglichen, ohne dass die Vorrichtung dauerhaft getragen werden muss. Die Wirkung des Trainings soll sein, dass nach dem Training, insbesondere nach wiederholtem Training die Neurophysiologie und die Biomechanik des Kiefergelenks günstiger ist als zuvor. Ein zusätzliches Ziel ist es, mit Hilfe der erfindungsgemäßen Vorrichtung einen trainierten verbesserten Zustand herzustellen, um dann weiterführende Therapieschritte effizienter und mit noch besserem Ergebnis durchführen zu können.

### Erfindungsgemäße Lösung

Die Aufgabe wird durch den Gegenstand der unabhängigen Ansprüche gelöst. Vorteilhafte Weiterbildungen des Gegenstandes der unabhängigen Ansprüche sind in den Unteransprüchen gekennzeichnet. Der Wortlaut sämtlicher Ansprüche wird hiermit durch Bezugnahme zum Inhalt dieser Beschreibung gemacht. Die Verwendung der Einzahl soll die Mehrzahl nicht ausschließen, was auch im umgekehrten Sinn zu gelten hat, soweit nichts Gegenteiliges offenbart ist.

Im Folgenden werden einzelne Vorrichtungen und Verfahrensschritte näher beschrieben. Bei der Beschreibung der Vorrichtung kann der Bezug zu Oberkiefer OK und Unterkiefer UK vertauscht werden, weil insbesondere die Transversale Gleitebene TGE von Interesse ist.

### Erfindungsgemäße Vorrichtung

Zur Lösung der gestellten Aufgabe wird eine individuell gefertigte Vorrichtung 100 insbesondere zum Training der Kiefermuskulatur vorgeschlagen. Sie beruht in ihren Kontaktflächen zu den Zähnen und zum Zahnfleisch auf den individuellen 3D Daten des Gewebes, weicht aber körpergewebeseitig von der reinen dem Fachmann bekannten 3D digital Abdrucks-Geometrie in erfindungsgemäßer Weise charakteristisch ab, um den Trainingseffekt zu erzielen. Auch außerhalb der Zahnbögen hat sie eine erfinderische und funktionelle Form, die den Trainingseffekt unterstützt.

Die Trainingsvorrichtung ermöglicht es insbesondere, eine genau auf der Trenngleitfläche senkrechte Kraft zu erzeugen und den Kaudruck in die Zahnbögen im OK und UK an definierten Auflagepunkten einzuleiten, wobei dabei möglichst wenige oder gar keine seitlich wirkenden Kräfte auf die Zähne wirken sollen. Dies dient dazu, ein trainingswirksames neurophysiologisches Signal im Kauapparat zu erzeugen und geeignete Trainingseffekte im sensomotorischen Areal zu erreichen, das für die Kieferbewegung und für den Aufbau von Kaukräften zuständig ist.

Die Vorrichtung umfasst mindestens ein Oberkiefer-Modul 120 und mindestens ein Unterkiefer-Modul 130, die durch eine gleitfähig glatte Trenn-Gleit-Ebene 110 getrennt sind. Dabei kann das Oberkiefer-Modul 120 und oder das Unterkiefer-Modul 130 einteilig aus einem Stück bestehen oder mehrteilig sein und aus mehreren Stücken bestehen. Die Stücke des ggfs. Mehrteiligen Moduls bilden zusammen eine ebene Trenn-Gleit-Ebene TGE 110. Die Trenn-Gleit-Ebene 110 ist bei eingesetzter Vorrichtung 100 von vorne gesehen vorwiegend waagrecht orientiert bzw. in der Transversalachse parallel zur Transversalachse zwischen rechts-links-symmetrischen Landmarken des Gesichtsschädels, wie z.B. Jochbein, Orbita-Bogen, etc.. Von der Seite betrachtet neigt sich die Trenn-Gleit-Ebene 110 nach hinten führend schräg nach unten in einem Winkel zwischen 5° und 25° von der Transversalen abwärts führend. Die Trenn-Gleit-Ebene 110 entsteht durch Aufeinanderlegen der beiden Module OKM 120 und UKM 130 genau zwischen den kontaktierenden Modulen. Die Oberfläche hat eine geringe Rauheit, bevorzugt mit RZ unter 5 Mikrometer, besonders bevorzugt ohne sichtbare Riefen und RZ unter 2 Mikrometer.

### Bevorzugte Ausführungsbeispiele der Erfindung

Diese Trenn-Gleit-Ebene 110 der Trainingsvorrichtung liegt bei therapiebedürftigen Patienten nicht parallel zur Kauebene 111, sondern ist in zwei Kipprichtungen links-rechts und hinten-vorne geneigt. Dabei ist diese Trenn-Gleit-Ebene 110 eine parallele zu einer cranialen Symmetrieebene, die an Landmarken des vorderen Gesichtsschädels orientiert ist, wobei sie individuell je nach Geometrie des Craniums ausgerichtet ist und damit in individuellem Ausmaß gegen die individuelle Kauebene 111 geneigt ist. Die Kauebene 111 des Gebisses des Patienten hingegen unterscheidet sich von der Trenn-Gleit-Ebene 110. Die Trainingsvorrichtung umfasst Freiräume für die Zähne der Zahnbögen des Ober- und Unterkiefers, wobei diese Freiräume lokal gezielt weiter bzw. breiter gestaltet sind als die entsprechende Breite der Zähne, so dass keine seitlich wirkenden Kräfte auf die Zähne des Zahnbogens ausgeübt werden, weil die Auflagekräfte nur senkrecht zur Trenn-Gleit-Ebene 110 wirksam sind. Im Fehlen der seitlich wirkenden Kräfte zwischen Trainingsvorrichtung und Zahnbogen unterscheidet die Trainingsvorrichtung ganz wesentlich von allen anderen Schienen und Spangen, die insbesondere für einen festen Sitz und zur kraftvermittelten seitlichen Verlagerung der Zähne nach hinten oder vorne und oder nach rechts oder nach links benutzt werden.

Die Kauebene 111 kann durch ein starren und ebenen Gegenstand, wie beispielsweise ein Blättchen aus Metall, bestimmt werden, der zwischen Oberkiefer und Unterkiefer beim Schließen des Gebisses, eingeklemmet wird. In der eingeklemmten Position entspricht die Ebene des Blättchens der Kauebene 111.

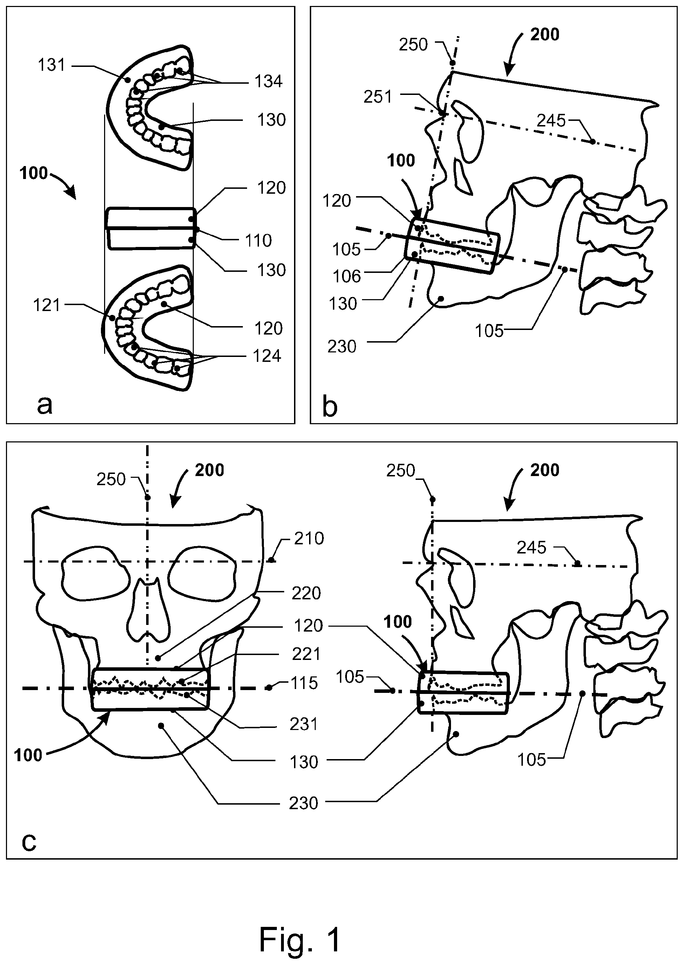

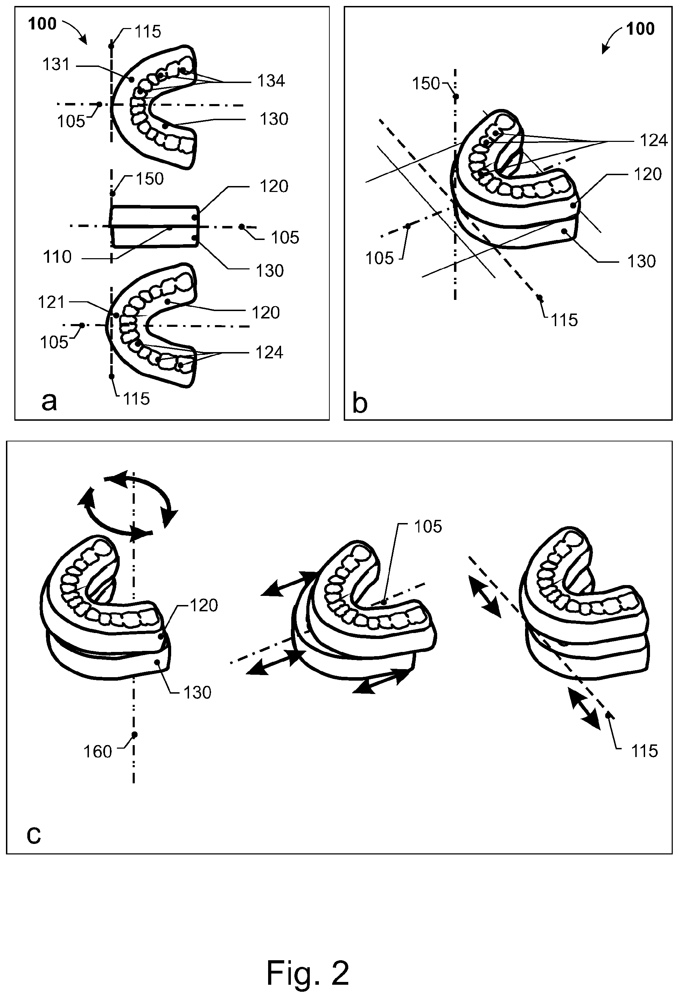

Durch die glatte und besonders bevorzugt polierte Gleitfläche ist auch unter Druck ein fast reibungsfreies Gleiten des Unterkiefer-Moduls 130 zum Oberkiefer-Modul 120 (siehe Fig. 1) in zwei Dimensionen möglich und ebenso eine Rotation um eine zur Trenn-Gleit-Ebene senkrechte Achse 160 (siehe Fig.2). Verkippungen der Module 120 und 130 zueinander im Sinne von Rotationsbewegungen um Achsen 105 und 115, die parallel zur Trenn-Gleit Ebene 110 liegen, sind nicht möglich, sobald die Module durch die Beißkraft zusammengehalten werden. Die physikalische Konsequenz der gleitfähigen glatten und reibungsarmen Trenn-Gleit-Ebene 110 ist, dass die Kontaktkraft bei vernachlässigbarer Reibungskraft senkrecht auf dieser Ebene steht, solange die Bewegungsfreiheit zwischen Unterkiefer-Modul 130 und Oberkiefer-Modul 120 nicht durch seitliche Anschläge eingeschränkt wird. Deshalb wird mit randspalten 122 bzw. 132 gearbeitet, die die kleinräumige aber wichtige seitliche Beweglichkeit in Gleitrichtung gewährleisten. Die Module 120 bzw. 130 weisen dem jeweiligen Zahnbogen 221 bzw. 231 folgend mehrere Vertiefungen 124 im Oberkiefer Modul bzw. 134 im Unterkiefer Modul auf. Vorwiegend im Bereich der Schneidezähne, manchmal aber auch im Bereich der Backenzähne treten die Vertiefungen 124 der oberen Schneidezähne 221 durch das Oberkiefer-Modul 120 hindurch und erreichen das Unterkiefer-Modul 130, wo sie kleine wechselseitige Vertiefungen in der ansonsten planen und glatten Trenn-Gleit-Ebene 133 bilden. Umgekehrt können auch die Zähne des Unterkiefers 231 im Unterkiefer-Modul 130 Vertiefungen 134 beanspruchen, die das Unterkiefer-Modul 130 durchbrechen, wobei flache Vertiefungen 122 im darüber liegenden Oberkiefer Modul entstehen, die ebenfalls einen Randspalt aufweisen, also ohne Kontakt mit den durchtretenden Zähnen sind.

Diese durchtretenden Vertiefungen 132 im Unterkiefer-Modul und 122 im Oberkiefer-Modul führen nicht zur mechanischen Verschlüsselung des Oberkiefer-Moduls 120 mit dem Unterkiefer-Modul 130, denn sie sind durch einen umlaufenden ausreichend breiten Randspalt gekennzeichnet, der in diese flache Vertiefung hinein reichenden Zahn jeweils seitliche Bewegungsfreiheit gibt. Der Randspalt zwischen Rand der flachen Vertiefung und Zahn ist bevorzugt größer als 0,3 mm, liegt besonders bevorzugt zwischen 0,4 und 1,4mm. Die Bewegungsfreiheit des Unterkiefers relativ zum Oberkiefer entlang der Trenn-Gleit-Ebene 110 ist also auf diesen wichtigen Randspalt der flachen Vertiefungen eingeschränkt, sobald solche durchtretenden Vertiefungen 122 bzw. 132 vorhanden sind. Die biomechanische und neurophysiologische Wirkung der als Trainingsgerät nutzbaren Vorrichtung 100 ist von der eingeschränkten transversalen Bewegungsweite unbeeindruckt, denn der Effekt beruht auf der in der Gleichgewichtslage bestehenden seitlichen Beweglichkeit, die ja durch den Randspalt vorhanden ist.

Im Bereich der Kauflächen der Zähne und im Bereich des Zahnfleisches ist ebenso ein Randspalt vorhanden. Um diesen Randspalt auch bei Druckbelastung zu halten, stützen sich die Module auf lokal begrenzten hügelartigen oder Plateauartigen Vorsprüngen 125 bzw. 135 ab, die oberflächlich betrachtet angenähert rund oder oval aussehen, mit einem Durchmesser von 800 bis 6000 Mikrometer und einer Höhe von bevorzugt weniger als 800 Mikrometer. Die Kontaktkraft wird dabei nur an diesen Vorsprüngen 125 bzw. 135 zwischen Modul 120 bzw. 130 und dem jeweiligen Zahnbogen übertragen. Der Vorsprung 125 bzw. 135 selbst ist in der Kontaktfläche zum Zahn formschlüssig am Zahn angepasst. Falls in den Vertiefungen 124 bzw. 134 echte Durchbrüche 126 bzw. 136 vorhanden sein müssen, sind die Auflagepunkte 125 bzw. 135 daneben angeordnet. Insgesamt wird jedes Modul von mindestens 3 Kontaktpunkten 125 bzw. 135 auch unter Druckbelastung beim Zubeißen in einer Position mit Randspalten 122 gehalten. Die Randspalte 122 umgeben alle Zähne und sind mindestens so groß, dass das Aufsetzen und die Entformung der Module hinterschneidungsfrei verläuft und dass eine zusätzliche Beweglichkeit in kleinem maß um bevorzugt weniger als 1200 Mikrometer zwischen Modul und Zahnbogen gegeben ist, besonders bevorzugt weniger als 600 Mikrometer.

Die biomechanische Funktion der Vorrichtung 100 mit Trainingseffekt ergibt sich, nachdem das Oberkiefer-Modul 120 auf das Unterkiefer-Modul 130 gelegt wird und beide Module als Paket im Mund durch Schließen des Mundes in Kontakt mit den Zahnbögen des Oberkiefers 221 und des Unterkiefers 231 gebracht werden. Nun werden Unterkiefer 230 und Oberkiefer 220 in eine durch die individuell gefertigte 3D Geometrie der Module definierte Relativposition gebracht. Gleichzeitig ist aber die Gleitbewegung rechts-links und hinten-vorne sowie die Rotation um die zur Trenn-Gleit-Ebene senkrechte Achse 160 in gewissen Grenzen frei gegeben und fast reibungsfrei möglich. Dadurch beginnt die Wirkung der Orthogonalitätsbedingung für die Kontaktkraft zwischen Oberkiefer 220 und Unterkiefer 230. Das Gleichgewicht ist biomechanisch vergleichsweise instabil oder metastabil, weil die Retentionen der Zähne durch die Gleitfläche 110 eliminiert sind. Und genau dieser instabile oder metastabile Kraftzustand mit ansatzweise möglichem transversalem Gleiten des Unterkiefers nach vorn oder hinten sowie nach links oder rechts bedeutet im Detail eine mechanische Instabilität, die ähnlich wie der Aufrechte Gang neuromuskulär stabilisiert werden muss. Dadurch wird der Balancesinn aktiviert und dies bewirkt ein Training der neurophysiologischen und sensomotorischen Strukturen des Kiefergelenks und des Kauapparates bis hin zu den Muskeln des Kopfes und des Halses.

Für den optimalen Trainingseffekt hat die geeignete Orientierung der Trenn-Gleit-Fläche eine besondere Bedeutung. Von der 3D-Position der Trenn-Gleit-Ebene 110 relativ zum Oberkiefer hängt es im Detail ab, wie sich der Unterkiefer mit aufgesetztem Unterkiefer-Modul entlang der Trenn-Gleit-Ebene bewegen kann. Die Analyse von hunderten von 3D Röntgen Bilddaten des Kopfes hat gezeigt, dass die biomechanisch günstige Bewegungsebene nur selten dort liegt, wo die aktuelle Kau-Ebene erkennbar wird. Die Aktuelle Kau-Ebene liegt dort, wo eine dünne Beißplatte mit Ihrer Raumorientierung und Position liegen würde, wenn man sie zwischen den Zähnen des Oberkiefers und denen des Unterkiefers einklemmen würde. Die biomechanisch günstige Bewegungsebene hingegen hängt von der Symmetrie des Gesichtsschädels im oberen Bereich ab, also kaum vom Unterkiefer, viel mehr vom knöchernen Bereich um die Augenhöhlen rechts und links.

Überraschend stellte sich anhand einer Vielzahl von praktischen Anwendungsversuchen heraus, dass die biomechanisch günstige exakte Orientierung der gleitfähigen Trennebene 110 zwischen OKM 120 und UKM 130 in den meisten Fällen mit Trainingsbedarf deutlich messbar um einen Kippwinkel kappa 112 von der aktuellen Kau-Ebene AKE 111 der Zahnbögen abweicht. Für die Herstellung und für die Positionierung der erfindungsgemäße Vorrichtung 100 wird die Trennebene 110 nicht anhand der Zahnbögen in Oberkiefer 220 und Unterkiefer 230 ausgerichtet, sondern anhand der anatomischen transversalen Symmetrie-Achsen im Gesichtsschädel 200 (im fazialen Cranium). Die transversalen Symmetrieachsen im Gesichtsschädel findet der Fachmann z.B. im Röntgenbild oder im 3D DVT Bild anhand der klar erkennbaren knöchernen Merkmalspunkte am Jochbeinbogen und oder am knöchernen Rand der Augenhöhle. Da die Module für Oberkiefer und Unterkiefer im digitalen virtuellen 3D Modell an dieser Trenn-Gleit-Ebene positioniert werden, ergeben sich aus deren Position relativ zum jeweiligen Zahnbogen die dann herzustellende Vertiefung für die Zähne. Nach Herstellung der Vorrichtung ergibt das Einsetzen der Module auf die Zahnbögen wiederum die geplante Position und Ausrichtung der Trenn-Gleit-Ebene relativ zum Oberkiefer und zum Gesichtsschädel, der ja mit dem Oberkiefer anatomisch verbunden ist. Die Praxis zeigt eine mehr oder weniger deutliche und sehr individuelle Abweichung der vom Gesichtsschädel abgeleiteten Trenn-Gleit-Ebene TGE von der vorzufindenden Aktuellen Kau-Ebene AKE. In diesen Fällen herrscht eine entsprechende Asymmetrie des unteren Gesichtsschädels, des Oberkiefers und des Unterkiefers. Ein Teil der Asymmetrie des Unterkiefers UK relativ zum oberen Gesichtsschädel ist neuromuskulär bedingt und Folge von Verspannungen und Fehlhaltungen in den Kiefergelenken. Hier setzt das Training mit der erfindungsgemäßen Vorrichtung an, um die Symmetrie Schritt für Schritt wiederherzustellen.

Ein neurophysiologisch wichtiger Beitrag zum Trainingseffekt wird durch die wulstig dicke Form der Module erreicht. Die Module tragen außerhalb der Begrenzung des Zahnbogens in deutlichem Unterschied zu Aligner-Schienen oder Aufbeiß-Schienen dicke Materialwülste 121 bzw. 131, die dazu geeignet sind, das Weichgewebe im Lippenbereich zu dehnen. Diese Dehnung durch die scheinbare Übergröße der Module, die kaum in den Mund passen, führt zu einem gesteigerten neuromuskulären Feedback ans sensomotorische System des Kiefers und der mimischen Muskulatur. Da die möglichen Dehnungen individuell sehr unterschiedlich sind, wird die Massivität der umlaufenden Materialwülste 121 am Oberkiefer-Modul und 131 am Unterkiefer-Modul individuell ans Verfügbare Weichgewebe und an den Trainingsbedarf und Trainingszustand angepasst.

Die erfindungsgemäße Vorrichtung wird durch 3D Bearbeitung von geeigneten harten Materialien hergestellt. Die OK teile und die UK teile bekommen im Zuge der Formgebung die individuell charakteristischen Freiräume zur Aufnahme der Zähne bevorzugt mit Randspalt. Die Lage der Trenngleitebene relativ zur Kauebene der Zahnbögen ergibt sich aus der individuellen Symmetrie des Craniums, also des Gesichtsschädels und des Schädels bis zur Schädelbasis des Patienten. Diese Symmetrie kann man bevorzugt anhand von Röntgenaufnahmen erkennen. Erfindungsgemäß wird diese craniale Symmetrie zur Ausrichtung der Trenngleitebene 110 im Lageverhältnis zur Kauebene 111 verwendet.

Die zahlreichen Tests mit Probanden haben gezeigt, dass die Vorrichtung geeignet ist, nach bereits wenigen Minuten im Mund mit aktiv veränderlicher Kaudruckbelastung eine weitgehende Entspannung von zuvor verspannter und schmerzhafter Kiefergelenksmuskulatur zu erreichen.

Eine weitere wichtige Anwendung ist, dass durch die Wirkung des Trainings ein besserer Ausgangszustand für die Vermessung der Kiefergelenkskinematik erreicht wird und damit eine biomechanisch und zahnmedizinisch weitaus günstigere Basis für die Erfassung von physiologisch maßgeblichen Bewegungsdaten des Unterkiefers relativ zum Oberkiefer.

Das Vorstehende hat die Merkmale und technischen Wirkungen der Erfindung breit erläutert. Zusätzlichen Merkmale und technische Wirkungen von Ausführungsbeispielen der vorliegenden Offenbarung werden im Folgenden erläutert, z.B. der Gegenstand der Ansprüche. Es sollte durch den Fachmann anerkannt werden, dass die Konzeption und die spezifischen Weiterbildungen als Basis zum Modifizieren oder zum Entwerfen anderer Strukturen oder Prozesse genutzt werden können, welche gleich oder ähnliche Zwecke haben, wie das hier speziell erläuterte Konzept. Es sollte durch den Fachmann außerdem anerkannt werden, dass sich äquivalente Konstruktionen nicht vom Geist oder Umfang der Offenbarung entfernen, wie sie bspw. in den anliegenden Ansprüchen definiert ist.

Für ein vollständigeres Verständnis der offenbarten Konzepte und deren technischer Wirkungen und Vorteile wird nun auf die folgende Beschreibung in Verbindung mit den beiliegenden Figuren Bezug genommen. Die Figuren sind nicht zum Skalieren gezeichnet. In den Zeichnungen zeigen.

### Liste der Figuren

Weitere Einzelheiten und Merkmale ergeben sich aus der nachfolgenden Beschreibung von bevorzugten Ausführungsbeispielen in Verbindung mit den Figuren. Hierbei können die jeweiligen Merkmale für sich alleine oder zu mehreren in Kombination miteinander verwirklicht sein. Die Möglichkeiten, die Aufgabe zu lösen, sind nicht auf die Ausführungsbeispiele beschränkt.

Die Ausführungsbeispiele sind in den Figuren schematisch dargestellt. Gleiche Bezugsziffern in den einzelnen Figuren bezeichnen dabei gleiche oder funktionsgleiche bzw. hinsichtlich ihrer Funktionen einander entsprechende Elemente.
- Fig. 1: a bis c zeigen die Konturen der Vorrichtung 100 und deren Position anhand der knöchernen Konturen eines Kopfes 200.
- Fig. 2: a bis c zeigen die möglichen relativen Bewegungen, (2 x Translation und 1 x Rotation) zwischen Unterkiefer und Oberkiefer innerhalb der Trenn-Gleit-Ebene TGE.
- Fig. 3: zeigt einen Mund mit eingesetzter Vorrichtung 100 bei deutlich schräg liegender Dental-Kau-Ebene 111 im Vergleich zur Trenn-Gleit-Ebene 110.
- Fig. 4: a und b zeigen die Preforms 20 und 30 für die Herstellung der Module 120 für Oberkiefer und 130 für Unterkiefer für materialabhebende Fertigung.
- Fig. 5: a und b zeigen Varianten mit mehrteiligem Oberkiefer-Modul 120 und mehrteiligem Unterkiefer-Modul 130, dabei ist die Trenn-Gleit-Ebene 110 die Kontaktfläche.
- Fig. 6: zeigt einen Transversalschnitt durch ein individuelles Oberkiefer-Modul 120 mit den Vertiefungen 124 für korrespondierende Zähne des Zahnbogens des Oberkiefers.
- Fig. 7: zeigt die knöchernen Konturen des Schädels mit dessen Symmetrieachse 215 und bevorzugt parallel dazu die Transversale Achse 115 der Trenn-Gleit-Ebene 110 der im Mund eingesetzten Vorrichtung 100, die schräg zur Dental-Kau-Ebene 111 gekippt ist.
- Fig. 8: zeigt den Schnitt AB durch die Vorrichtung 100 mit den Vertiefungen 124 und 134 für die korrespondierenden Zähne, wobei teilweise die Vertiefungen 134 das Modul 130 durchbrechen und Vertiefungen 129 auch in der ansonsten planen und glatten Gleitfläche 123 des Moduls 120 entstehen. Hier sind die Randspalte 122 sichtbar.
- Fig. 9: zeigt ein Oberkiefer-Modul 120 mit im Beispiel 4 Auflagepunkten 125, wobei an den anderen Zähnen ohne Auflagepunkte Randspalte 122 vorhanden sind. Dadurch besteht eine nur punktweise Auflage der Module auf den Auflagepunkten 125.
- Fig. 10: zeigt im Schnittbild die Vorrichtung 100 im bevorzugten Ausführungsbeispiel mit den gleitfähig glatt gearbeiteten Gleitflächen 123 und 133, wobei die Trenn-Gleit-Ebene 110 durch aneinanderfügen der Module für Oberkiefer 120 und Unterkiefer 130 entsteht.
- Fig. 11: a zeigt die Vertiefungen 124 mit den dentalen Zahnpositionen 11 bis 17 sowie 21 bis 27 des Oberkiefers im Oberkiefer-Modul 120 sowie die durchbrechenden Vertiefungen 126. Fig 11 b zeigt im Detail von oben den Zahn des Zahnbogens 221 mit dem umgebenden Zahnfleisch 227 und das Oberkiefer-Modul 120 mit Gleitfläche 123 und Auflagepunkt 125 sowie Randspalt 122.
- Fig. 12: zeigt DVT 3D Röntgenaufnahmen eines Kopfes 200 mit Oberkiefer 220 und Unterkiefer 230 sowie den Symmetrieachsen 250 unten-oben, 105 vorne-hinten und 115 rechts-links anhand der Knochenmerkmale im oberen Gesichtsschädel.
- Fig. 13: zeigt die Erzeugung digitaler bzw. virtueller 3D Bilder von Knochenstrukturen und 3D Strukturmarkierungen.
- Fig. 14: zeigt ein Schema für das Verfahren zur individuellen Anwendung der 3D Strukturmarkierung.
- Fig. 15: zeigt die Kombination von mindestens zwei Ebenenpaketen zu einem 3D Strukturmarker.
- Fig. 16: zeigt Darstellungen des 3D Strukturmarkers mit zusätzlichem sagittalem Ebenenpaket.
- Fig. 17: zeigt eine besonders bevorzugte isotrope Ausführung derje fünf Ebenenpakete.
- Fig. 17a: zeigt eine Vorderansicht von Figur 17.
- Fig. 18: zeigt die Ergänzung der Ebenenpakete durch ein doppeltes Ebenenpaket.
- Fig. 19: zeigt den Transversalschnitt durch die knöcherne Struktur eines Schädels und zeigt, wie die Transversalachse 215 an Landmarken ausgerichtet und damit die Sagittalebene Sm definiert wird.
- Fig. 20: zeigt das median sagittale Schnittbild durch den röntgenologisch dargestellten Kopf mit Ankerpunkten sowie mit eingepasster 3D Strukturmarkierung.
- Fig. 21: zeigt die Strukturmarkierung im Sagittalschnitt vor der Drehung und Skalierung etc.
- Fig. 22: zeigt die Strukturmarkierung in Orientierung entlang der Achse BP - GP.
- Fig. 23: zeigt die Strukturmarkierung mit dem Schritt der Skalierung, um mit Linie F1 den Ankerpunkt NP zu erreichen und wie dadurch der ideale Inzisalpunkt IP definiert wird.
- Fig. 24: zeigt eine alternative Möglichkeit, das gleiche Ergebnis wie in Fig. 8 zu erreichen, allerdings an einem Patienten mit fliehendem Kinn.
- Fig. 25: zeigt einen Transversalschnitt durch den Zahnbogen eines symmetrischen Oberkiefers mit Strukturmarkierung.
- Fig. 25a: zeigt einen Transversalschnitt durch den Zahnbogen eines asymmetrischen Oberkiefers.
- Fig. 26: zeigt einen Transversalschnitt durch den Bereich der Nasenscheidewand mit Asymmetrie.
- Fig. 27: zeigt einen Transversalschnitt durch Landmarken des Innenohrs mit der Innenohrachse.
- Fig. 28: zeigt einen Frontalschnitt durch das Kiefergelenk mit sichtbarer Verkippung des Unterkiefers.
- Fig. 29: zeigt einen Frontalschnitt durch den Backenzahnbereich bei zu hoch liegender Kauebene.
- Fig. 30: zeigt einen Frontalschnitt durch die unteren Schneidezähne.
- Fig. 31: zeigt eine Draufsicht und eine Seitenansicht der Trainingsvorrichtung.
- Fig. 32: zeigt das Detail eines Auflagebereiches im besonders bevorzugten Ausführungsbeispiel.
- Fig. 33: zeigt die Trainingsvorrichtung von der Seite am Gebiß mit starker Schräglage
- Fig. 34: zeigt die Strukturmarkierung im Sagittalschnitt mit eingezeichneter Trenn-Gleit-Ebene
- Fig. 35: zeigt einen digitalen bzw. virtuellen Frontalschnitt durch den Backenzahnbereich und offenbart einen deutlichen Schrägstand der aktuellen Okklusionsebene in diesem Beispiel.
- Fig. 36: zeigt eine Trainingsvorrichtung mit Trenn-Gleit-Ebene in der Position der idealen Okklusionsebene in grob schematischer Darstellung.
- Fig. 37: den Kopf schematisch von der Seite mit einer Raumausrichtung in der das Koordinatensystem des Schädels senkrecht steht.

### Figuren und Ausführungsbeispiele mit Details

### Figur 1

Zu berücksichtigen ist in Fig. 1a die Klapprichtung der abgebildeten Vorrichtung, die dazu führt, dass die Ansicht des Unterkiefer-Moduls 130 von unten in Fig. 1a oben ist. Entsprechend ist die Ansicht des Oberkiefer-Moduls 120 von oben unterhalb gezeichnet. Dazwischen ist die Seitenansicht zu sehen, mit dem Oberkiefer-Modul 120 in direktem Kontakt mit dem Unterkiefer-Modul 130. Die Vorrichtung 100 verfügt über ein Oberkiefer-Modul 120 und ein Unterkiefer-Modul 130. Zwischen den Modulen 120 und 130 bildet sich die glatte, ebene und gleitfähige Trenn-Gleit-Fläche 110. Die dort möglichen Relativbewegungen sind in Fig. 2 beschrieben.

Fig 1b zeigt die eingesetzte Vorrichtung 100 im Mund, wobei die Vorrichtung 100 nur stark schematisiert gezeichnet ist und auch der Kopf ist nur durch die Knochenkonturen 200 angedeutet. Die Achse 250 führt von oben nach unten, wenn der Kopf normal gehalten wird, läuft sie leicht schräg mit meist 5° bis 15° Neigung. Ein bevorzugter Ankerpunkt für die Position ist der Nasionpunkt 251 an der Nasenwurzel. Die Achse von vorne nach hinten 245 im oberen Schädelbereich steht darauf senkrecht. Die Achse 105 für die Orientierung der Vorrichtung verläuft parallel zu 245 durch den Überlappungsbereich der Schneidezähne, den man Inzisionspunkt nennt. Diese Punkte sind dem Fachmann der Dentalmedizin und Kieferorthopädie bekannt. Der Oberkiefer 220 trägt den zahnbogen 221 und der Unterkiefer 230 trägt den zahnbogen 231. Die Trenn-Gleit-Ebene 110 wird definiert durch die Achsen 105 von vorne nach hinten schräg fallend und die Transversalachse 115 rechts-links.

Fig 1c zeigt den Schädel in senkrechter Orientierung. Die vertikale Achse 250 steht genau senkrecht bezüglich der vorn-hinten Achse 105, welche genau waagrecht verläuft. Die Vorrichtung 100 ist genau parallel zur rechts-links-Achse 115 ausgerichtet, die Trenn-Gleit-Ebene 110 ist streng horizontal transversal orientiert. Aus dieser Orientierung der Vorrichtung 100 mit Oberkiefer-Modul 120 und Unterkiefer-Modul 130 ergibt sich die relative Position aller Zähne und damit die Position der Vertiefungen 214 für die korrespondierenden Zähne.

### Figur 2

Fig. 2a zeigt die Vorrichtung aus Fig. 1a mit den orthogonalen Achsen 105, 115 und 150.

Fig. 2b zeigt die drei orthogonalen Raumachsen der Vorrichtung 100, Die vertikale oben-unten Achse 150 , die transversale rechts-links Ache 115 und die vorne-hinten-Achse 105. Die beiden Achsen 115 und 105 spannen die Trenn-Gleit-Ebene 110 auf.

Fig 2 c zeigt die drei Bewegungsfreiheitsgrade, die für die Relativbewegung zwischen Oberkiefer-Modul 120 und Unterkiefer-Modul 130 bestehen, soweit sie nicht durch seitliche Anschläge begrenzt sind. Die Randspalte der Vertiefungen für die Zähne erlauben die erforderliche Bewegungsfreiheit um den labilen Gleichgewichtspunkt herum. Möglich sind angesichts der Biomechanik des Kiefergelenks kleine Drehbewegungen um eine weiter hinten liegende vertikale Achse 160 parallel zur Achse 150 sowie überlagert dazu kleine Translationsbewegungen entlang der vorne-hinten-Achse 105 und zudem auch entlang der rechts-links-Achse 115. Diese freien Bewegungen um die labile Gleichgewichtslage bei Kompression der Vorrichtung 100 zwischen Oberkiefer und Unterkiefer ist wichtig für die neurophysiologische und biomechanische Wirkung der Vorrichtung 100.

### Figur 3

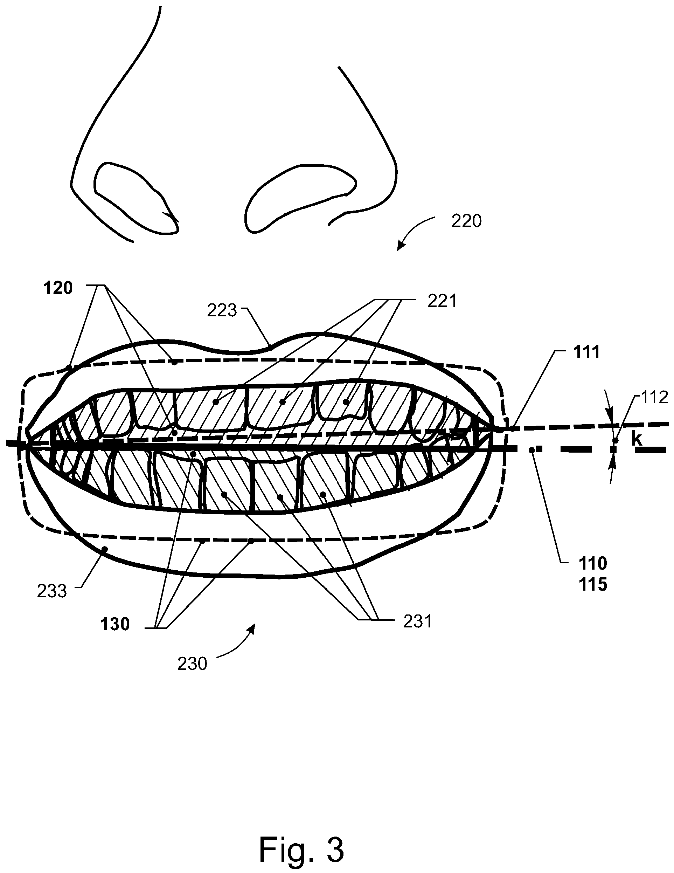

Fig. 3 zeigt die Vorrichtung 100 mit Oberkiefer-Modul 120 und Unterkiefer-Modul 130 teilweise sichtbar, dort schraffiert, aus transparentem druckfestem Material. Durchscheinend zu sehen sind die Zähne 221 und 231 und deren schräg laufende Dental-Kau-Ebene 111. Die Lippen 223 und 233 verdecken den Rest der Vorrichtung 100, die dort gestrichelt angedeutet ist. Die Lage der Dental-Kau-Ebene 111 kann man erfassen, indem man in eine Platte beißen lässt oder indem man im digitalen 3D Bild eine Best Fit Ebene zwischen die Zahnbögen von Oberkiefer und Unterkiefer legt. Wichtiges Maß für die Asymmetrie des Gebisses relativ zum oberen Gesichtsschädel ist der kleine Winkel k 112 zwischen rechts-links-Achse 115 der Trenn-Gleit-Ebene 110 und der Dental-Kau-Ebene 111.

### Figur 4

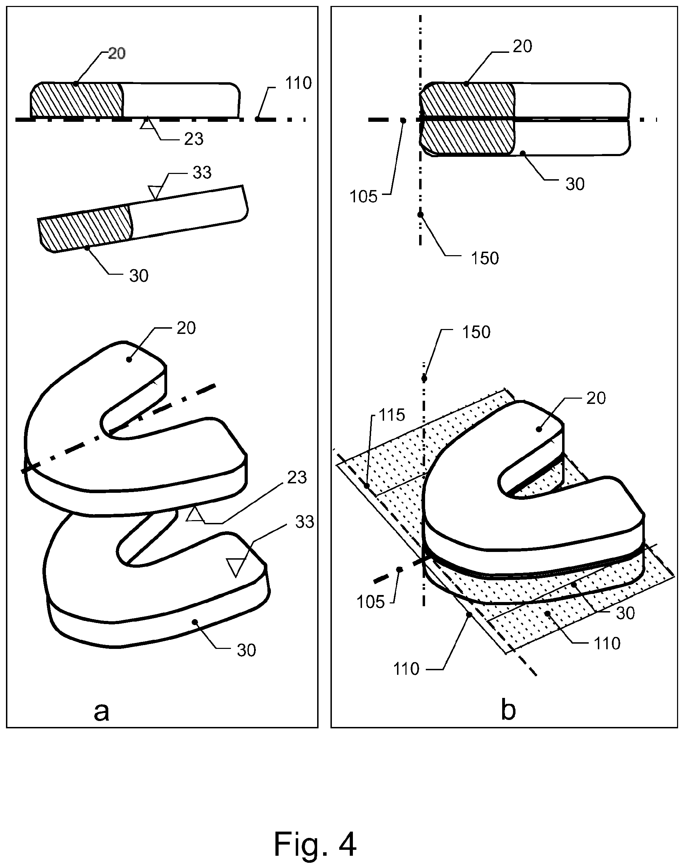

Fig. 4a: In einem bevorzugten Ausführungsbeispiel werden für die Erzeugung der Module 120 und 130 hufeisenförmige Preforms zur weiteren spanabhebenden Bearbeitung verwendet. Die Module können alternativ z.B. aus runden Kunststoff-Scheiben gefräst oder additiv gefertigt werden. Die Verwendung transparenter Kunststoffe wird bevorzugt. Das Material muss druckfest, formstabil und gleitfähig sein, besonders bevorzugt ist es gut polierbar, um an den Kontaktflächen bzw. Trenn-Gleit-Flächen 23 bzw. 33 jeweils eine glatte, plane und fast reibungswiderstandsfreie Gleitfläche herstellen zu können. Geeignet ist z.B. PMMA.

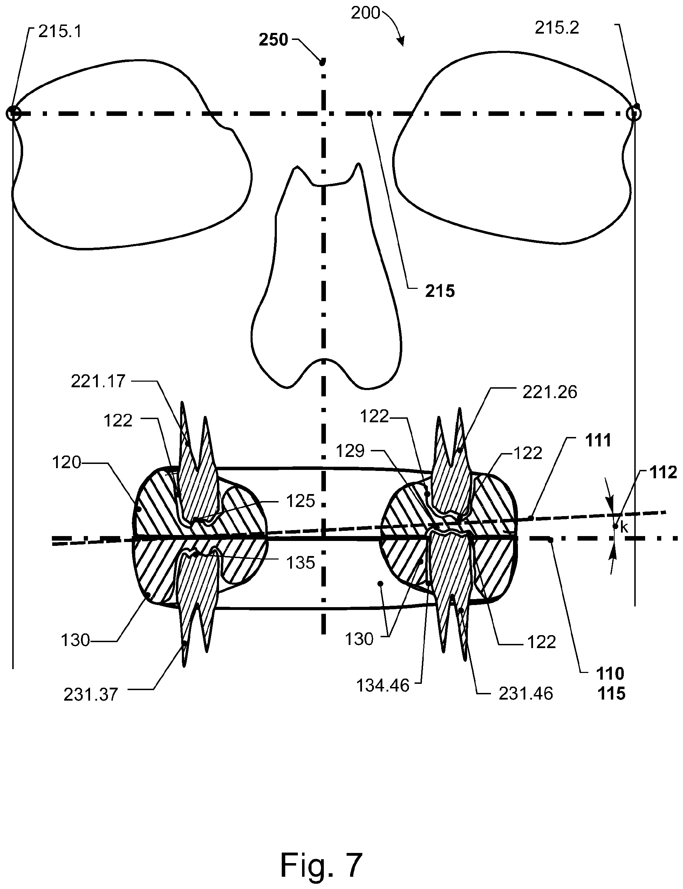

Fig. 4b: Das Oberkiefer-Preform 20 passt geometrisch annähernd oder genau zum Unterkiefer-Preform 30. Legt man die beiden Preforms 20 und 30 an den Trenn-Gleit-Flächen 23 und 33 aneinander, so bilden sie eine plane glatte gleitende Kontaktfläche, die die Ebene 110 definiert. Diese Ebene 110 ist parallel zu den Symmetrieachsen des Schädels vorn-hinten 205 und rechts-links 215. Zur digitalen 3D Fertigung der Module 120 und 130 werden die Preforms 20 und 30 digital relativ zum Schädel 200 und dessen drei orthogonalen Achsen 250, 215 und 205 so positioniert, dass die drei orthogonalen Achsen 150, 115 und 105 der Preforms 20 und 30 parallel zu den Schädelachsen liegen. Die 3D Position der Preforms im Schädel wird anhand der Schädelsymmetrie so ausgerichtet, dass die Achse 105 in etwa gleichen Abstand von den Schneidezähnen des Oberkiefers und Unterkiefers verläuft. Die Schrägneigung aus seitlicher Sicht ergibt sich anhand weiterer anatomischer Landmarken. Nach Festlegung der Position des Pakets aus den beiden Preforms 20 und 30 in der virtuellen 3D Umgebung wird die 3D Kontur der Zahnbögen und des Zahnfleisches über die Preforms gelegt, so dass man die notwendigen Vertiefungen in den Modulen 120 und 130 bekommt. Zusätzlich zur geometrischen Volumen der Zahnbögen werden die Vertiefungen für die Zähne weiter gestaltet als anhand der direkten 3D Daten der Zähne, indem im Preform eine breitere und tiefere Vertiefung für jeden Zahn erzeugt wird und auch für das Zahnfleisch Vertiefungsbereiche erzeugt sind, die etwas tiefer gehen als anhand der direkten 3D Daten des Zahnfleischgewebes für den Formschluss notwendig ist. Durch die weiter ausgedehnten Vertiefungen in den Preforms entstehen zunächst durchgängige Randspalte. Um den Randspalt später im realen auch bei Druck aufrecht erhalten zu können, werden Kontaktpunkte ausgearbeitet, an denen die Randspalte in kleinen lokalen Bereichen massiv durchbrochen sind, indem dort die Vertiefung nur genau so weit ausgearbeitet ist wie es der 3D Form des Zahnes entspricht. Bevorzugt befinden sich die Kontaktpunkte 125 (siehe Fig. 7, 8, 9, 11) an ausgewählten Okklusionsflächen von Zähnen jeweils in einem kleinen Bereich von 1 bis 5 mm Durchmesser, wobei jedes Modul unter einer Beißkraft von mindestens 3 Kontaktpunkten gegenüber dem korrespondierenden Zahnbogen mit einem Randspalt von 0,3 bis 1,3 mm getragen wird.

### Figur 5

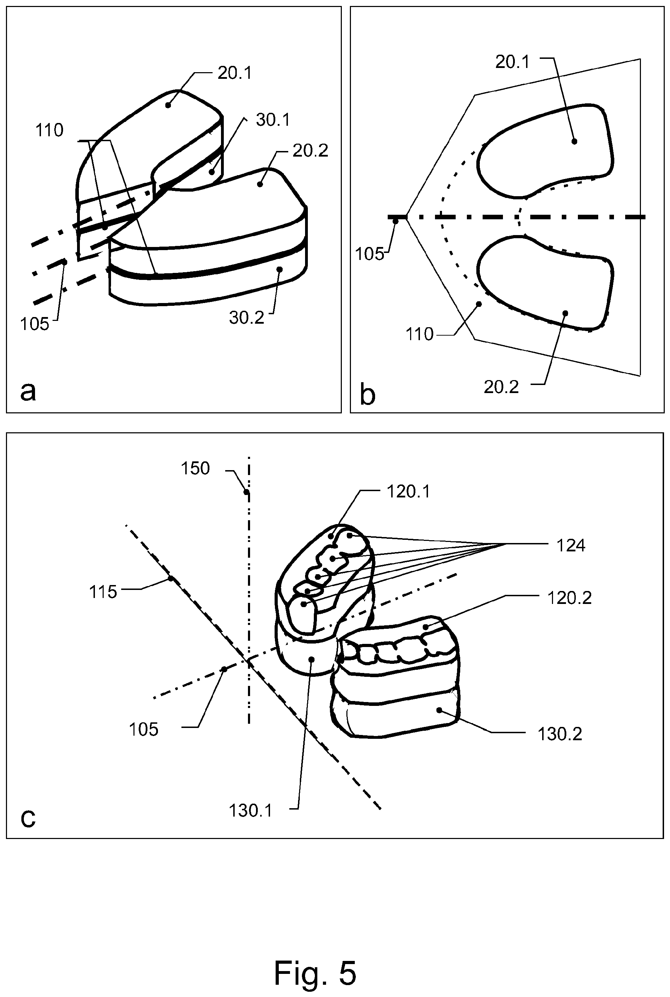

Als besonderes Ausführungsbeispiel werden mehrteilige Preforms 20 und 30 zur Herstellung mehrteiliger Module 120 und 130 verwendet. Fig. 5a zeigt ein Paar von Preforms die jeweils zweiteilig sind: Preforms 20.1 und 20.2 sowie 30.1 und 30.2. Fig. 5b. zeigt andere Formvarianten. Dem Fachmann ist bekannt, dass man bei spanabhebender Bearbeitung das gleiche Ergebnis auch bekommt, wenn man die Bearbeitung von einem einteiligen Preform entsprechend durchführt. Fig. 5c: In diesem besonderen Ausführungsbeispiel handelt es sich um zweiteilige Module für den Oberkiefer 120.1 und 120.2 sowie für den Unterkiefer 130.1 und 130.2. Es kann auch ein zweiteiliges Modul oben mit einem einteiligen Modul unten kombiniert werden und umgekehrt. Grundsätzlich sind mehrteilige Module möglich, wenngleich einteilige und zweiteilige Module bevorzugt sind. Die Anordnung der 3 Raumachsen 105, 115, 150 im 3D Digital Herstellungssystem relativ zum Schädel folgt den gleichen Bedingungen wie bei den einteiligen Modulen.

Die Preforms für die Teile 120 und 130 haben bevorzugt bereits die fast reibungsfreien Gleitflächen und werden anschließend nochmals poliert. Im digitalen Bildraum sind diese Teile vorhanden als 3D Referenzteile. Zunächst wird das kraniale Hauptachsensystem im Kopf individuell bestimmt, das ergibt die Hauptachsen des Craniums hinten vorne 205, rechts links 215, oben unten 250. Die beiden Teile 120 und 130 der Trainingsvorrichtung haben ebenfalls ein Koordinatensystem 305, 315, 350. Zur digitalen Erzeugung der Oberflächen und Volumina der Trainingsvorrichtung werden im 3D Manipulationssystem die Referenzobjekte 120 und 130 so in der Umgebung der Zahnbögen des Oberkiefer und Unterkiefer angeordnet, dass sie in ihren Hauptrichtungen möglichst exakt parallel sind zu den Hauptachsen des Craniums. Da die Hauptachsen des Kraniums nicht parallel sind sondern verkippt zu den Hauptachsen der Zahnbögen und damit des Gebisses und der Kauebene, ergibt sich aus der leicht verkippten und verdrehten Anordnung der Preforms für 120 und 130 relativ zu den Zahnbögen, dass die Freiräume 124 und 134 für die Zahnbögen, zuzüglich der Randspalte 122 und 132 relativ zu den Preforms verdreht und verkippt stehen. Daran kann man erkennen, wie weit im individuellen Fall das craniale System der Hauptrichtungen 205, 215, 250 verkippt und verdreht ist gegen das dentale System der Kauebene 111 mit den Hauptrichtungen 105, 115, 150.

### Figur 6

Das Ausführungsbeispiel zeigt ein einteiliges Unterkiefer-Modul 130. Es wurde im Beispiel aus einem einstückigen Preform 30 gefräst, das gleiche Ergebnis kann der Fachmann jedoch auch unter Verwendung anderer Fertigungstechniken erhalten, z.B. 3D Druck oder andere für individualisierte Produkte besonders geeignete additive Fertigungstechnologien. Das Unterkiefer-Modul 130 zeigt prinzipiell gleich dem Oberkiefer Modul 120 eine Mehrzahl von dem korrespondierenden Zahnbogen 231 folgende Vertiefungen 134. Die Vertiefungen 134 können auch flacher verlaufen als hier gezeigt, z.B. wenn das Zahnfleisch bis nahe an die Okklusionsflächen der Zähne reicht. Wichtig ist, dass im bevorzugten Fall die Vertiefungen 134 überall bis auf ganz wenige Punkte weiter ausgeformt sind als die Zähne, so dass ein Randspalt besteht zwischen Zahnmaterial und dem Material der Vorrichtung 100. Im transversalen Schnitt DE sieht man, dass weite Bereiche des Unterkiefer-Teils 130 tief gehende Aussparungen 134 haben, um sich an Zahnfleisch anzupassen. Im Außenbereich befindet sich eine biomechanisch und neurophysiologisch bedeutsame wulstförmige Materialanhäufung 131, die nicht abgefräst wird. Sie ist oft im Unterkiefer-Modul noch deutlicher ausgeprägt als im Oberkiefer-Modul. Dieser umlaufende Ringwulst 131 im Zusammenwirken zum entsprechenden Ringwulst 121 des Oberkiefer-Moduls 120 dient insbesondere der symmetrischen Dehnung und Streckung des Weichgewebes der Lippen, der mimischen Gesichtsmuskulatur und der Faszien. Der analoge Ringwulst 121 findet sich im Oberkiefer-Modul 120 (hier nicht gezeigt).

### Figur 7

In einem besonders bevorzugten Ausführungsbeispiel wird die Vorrichtung zum vorbereitenden Training der Kiefergelenksmuskulatur benutzt wen die zunächst vorhandene Dental-Kau-Ebene 111 biomechanisch korrigiert werden soll. Die Dental-Kau-Ebene steht in diesem Fall asymmetrisch schräg zur Transversalebene 110 und bildet zu dieser den Kippwinkel k 112. In der Ansicht von Vorn sieht man die Winkelabweichung zwischen den gezeichneten Achsen 115 rechts-links und der Dental-Kau-Ebene die zwischen den Zähnen die planare Best-Fit-Kaufläche darstellt. Das bedeutet in der Praxis, dass der Unterkiefer relativ zum Schädel schräg steht. Im Ausführungsbeispiel durchdringt wegen des Schrägstandes der Dental-Kau-Ebene 111 unter anderen die Vertiefung 134.46 des Zahnes 231.46 das Unterkiefer-Modul 130 vollständig, weil der Zahn 231.46 soweit nach oben reicht, dass dessen Kaufläche bis in das Oberkiefer-Modul 120 hinein reicht. Um dies zu ermöglichen wird von der Trenn-Gleit-Ebene 110 her eine flache Vertiefung 129 hergestellt, die einen deutlichen Randspalt 122 aufweist, um dem im Beispiel von unten kommenden Zahn 231.46 die seitliche Bewegungsfreiheit zu geben. Der Zahn 221.17 ruht auf dem Kontaktpunkt 125 und der zahn 231.37 ruht auf dem Kontaktpunkt 135. Abgesehen von diesen Kontaktpunkten herrscht in diesen Vertiefungen ebenfalls der Randspalt.

### Figur 8

In der Sicht von unten auf eine erste Gleitfläche 123 des Oberkiefer-Teils 120 sieht man für ein anderes Ausführungsbeispiel eine Reihe mehrerer Durchbrüche der Zähne von unten kommend, die das Unterkiefer-Modul 130 durchbrechen und im Oberkiefer-Modul 120 mehr oder weniger flache Vertiefungen 129 bekommen. Diese Vertiefungen 129 sind durch Randspalte 122 erweitert, die mindestens 0,3mm breit sind, nach oben hin ist die Breite des Randspaltes nur so weit praktisch begrenzt, dass die Gleiteigenschaften der Trenn-Gleit-Ebene 110 durch die Vertiefungen 129 und Randspalte 122 nicht beeinträchtigt werden soll. Für die neurophysiologische und biomechanische Funktion der Vorrichtung reicht ein Randspalt von bis zu 3mm aus, besonders bevorzugt beträgt der Randspalt 122 um die flachen Vertiefungen 129 zwischen 0,3mm und 1,3mm. Das Unterkiefer-Modul 130 hat an der Stelle der flachen Vertiefung 129 einen vollständigen Durchbruch 136. Der Randspalt in der Vertiefung 134 mit Durchbruch 136 im Unterkiefer-Modul darf etwas enger sein als der Randspalt 122 in der flachen Vertiefung 129. Analoges gilt mit Vertauschung von Oberkiefer und Unterkiefer, wenn Zähne von Oben ins Unterkiefer-Modul 130 durchbrechen.

### Figur 9

In einem weiteren Ausführungsbeispiel kommt es zu keinen Durchbrüchen 136 oder 126. Man sieht die Vertiefungen 124 mit Randspalt für die Zähne des Zahnbogens und die Kontaktpunkte 125, in diesem Fall beispielhaft 4 Stück, es können aber auch mehr sein. Mindestens erforderlich sind 3, besser 4 Kontaktpunkte 125. Die Kontaktpunkte 125 haben von oben bzw. unten betrachte angenähert Kreisform und in der Kontaktfläche haben sie lokal die Kontur des Korrespondierenden Zahnes, ohne Randspalt. In diesem Fall ist die Gleitfläche 123 nicht durch Durchbrüche unterbrochen. Der Schnitt oben zeigt das Oberkiefer-Modul mit Zähnen des Zahnbogens 221 von vorne, das Modul ist Hufeisenförmig hinten offen. Das Schnittbild unten zeugt das gleiche Modul 120 von hinten, zur Deutlichkeit ohne die Zähne. Dort sieht man links den Kontaktpunkt 125 deutlich abgesetzt. Was hier für den Oberkiefer-Modul 120 gezeigt wird gilt analog auch für das Unterkiefer-Modul 130.

### Figur 10

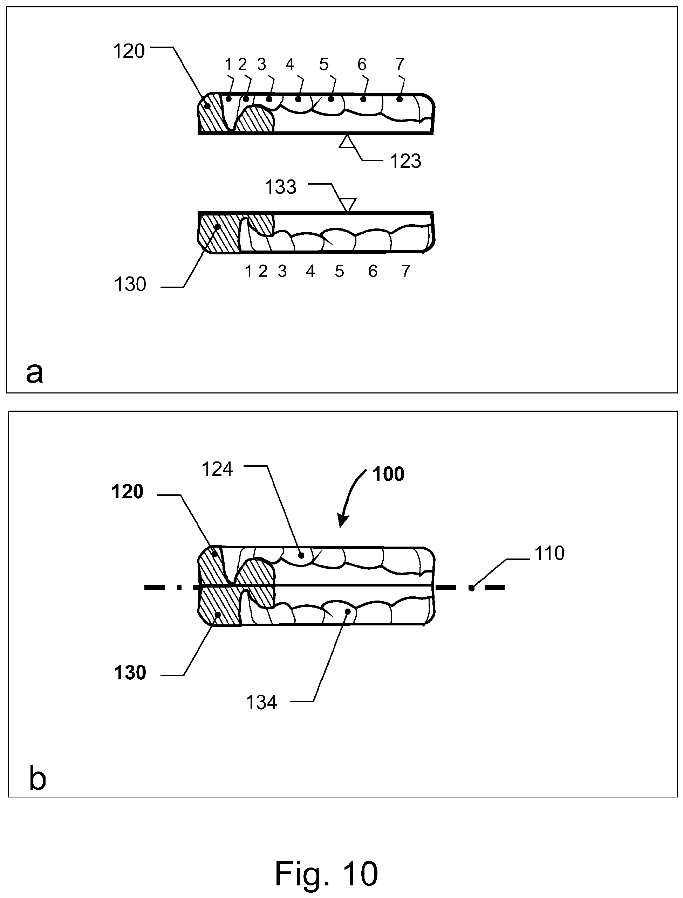

Im besonderen Ausführungsbeispiel in Fig. 10 bleibt die Form des Preforms bei der Einarbeitung der Vertiefungen für Zähne und Zahnfleisch fast erhalten. Wichtig sind die glatten, gleitfähigen ersten und zweiten Kontaktflächen 123 und 133. Zusammen genommen bilden die beiden einteiligen Module 120 und 130 die Vorrichtung 100. Oberkiefer-Modul 120 und Unterkiefer-Modul bilden direkt aneinander liegend die Trenn-Gleit-Ebene 110. Die Trenn-Gleit-Ebene 110 ist besonders bevorzugt so plan und so glatt, dass sich bei Benetzung mit Flüssigkeit der bekannte Hafteffekt zweier glatterPlatten bilden kann. Dabei sind Relativbewegungen in der Ebene 110 fast reibungsfrei möglich, aber die Platten können senkrecht zur Trenn-Gleit-Ebene 110 nur mit erheblicher Kraft auseinander getrennt werden. Das erleichtert insbesondere die Handhabung der Module, wenn man sie vor dem Einsetzen mit Wasser befeuchtet. Die beiden Module fallen also, wenn sie in der Trenn-Gleit-Ebene 110 ausreichend glatt sind, z.B. poliert, feucht sind, in der Praxis im Mund nicht auseinander, sind aber seitlich verschiebbar. In der Schnittansicht sieht man die Vertiefungen 124 und 134 nur teilweise, weil der Innenrand der Module 120 und 130, der innerhalb des jeweiligen Zahnbogens verläuft, die Kauflächen der Zähne verdeckt. Die Zahnpositionen 1 bis 7 entsprechen der im Dentalbereich üblichen Nummerierung.

### Figur 11

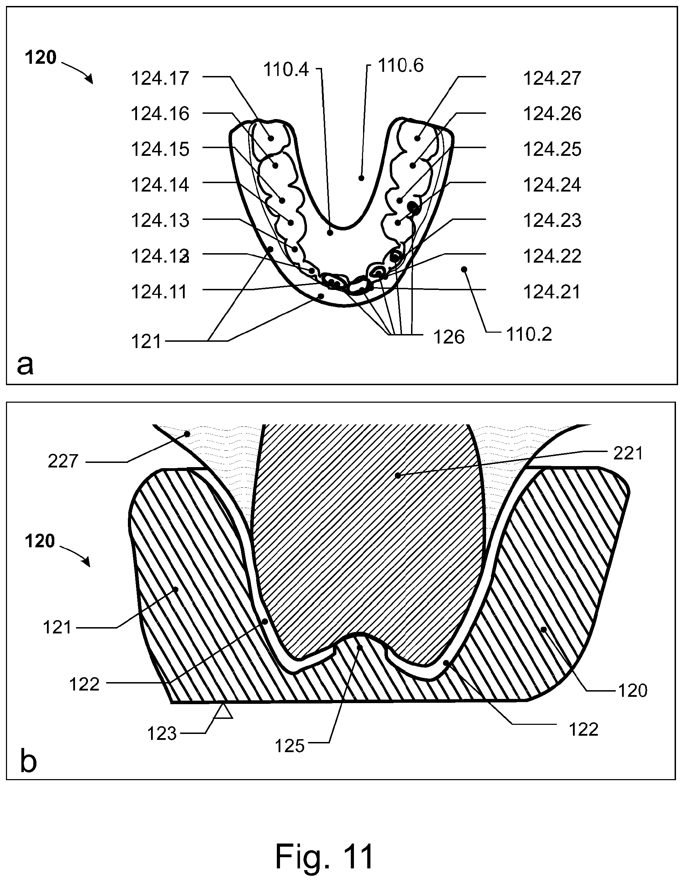

In einer weiteren besonders bevorzugten Ausführungsform ist der Bereich der Schneidezähne deutlich asymmetrisch gestaltet, dem individuellen Zustand der Zahnbögen entsprechend. Fig 11a zeigt die Durchbrüche 126 durch das Oberkiefer-Modul 120 für die Vertiefungen der Zähne 124.11, 124.21, 124.22, 123.23 und 124.25. Deutlich sichtbar ist der funktionell wichtige breite Rand 121 außerhalb des Zahnbogens mit bevorzugt mehr als 3mm Breite, besonders bevorzugt 3 bis 10mm Breite. Der Rand 131 im Unterkiefer-Modul 130 kann insbesondere im Frontzahnbereich noch breiter sein als im Oberkiefer-Modul, besonders bevorzugt 4 bis 15 mm.

Figur 11b zeigt ein Detail eines Kontaktpunktes 125 im Oberkiefer-Modul. Der Zahn des Zahnbogens 221 ist oben umgeben von Zahnfleisch 227. Der Randspalt 122 besteht am Zahnfleisch und am Zahn mit Ausnahme des Kontaktpunktes 125. Im Kontaktpunkt 125 liegt das Material des Oberkiefer-Moduls direkt am Zahn an, die Form korrespondiert mit der 3D Oberfläche des Zahnes an dieser Stelle. Die Dicke des Randspaltes ist dort minimal, fast null, in Abhängigkeit von der Belastung durch Kaudruck etc. Unten im Bild 11b befindet sich die plane erste glatte Gleitfläche 123. Analoges gilt für das Unterkiefer-Modul 130 und dessen Kontaktpunkte 135, jeweils angenähert an der Trenn-Gleit-Ebene gespiegelt.

### Figur 12

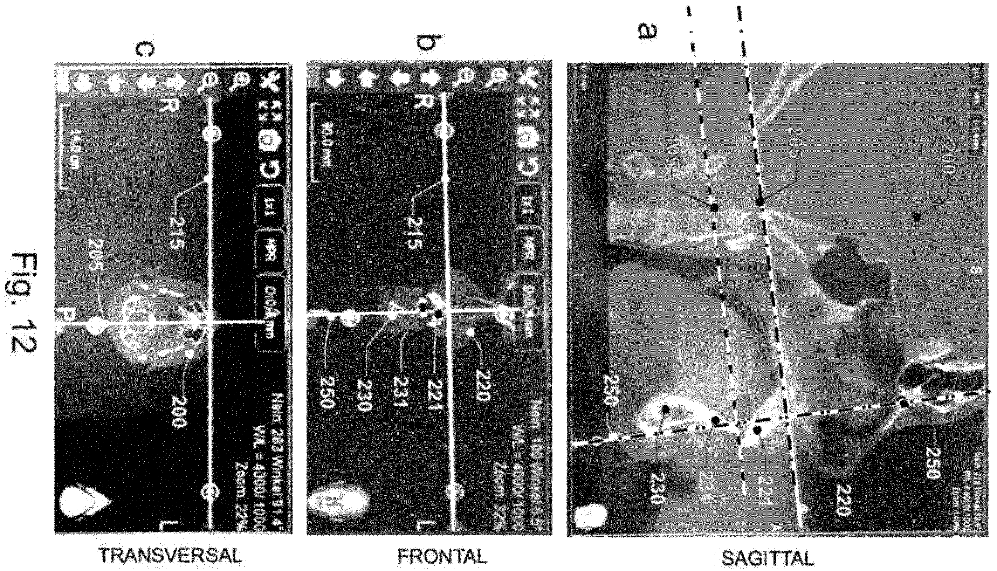

Figur 12 zeigt DVT 3D Röntgenaufnahmen eines Kopfes 200 mit Oberkiefer 220 und Unterkiefer 230 sowie den Symmetrieachsen 250 unten-oben, 105 vorne-hinten und 115 rechts-links anhand der Knochenmerkmale im oberen Gesichtsschädel. Die Positionierung der Vorrichtung im 3D Digitalmodell des Schädels ist Voraussetzung dafür, dass die reale 3D Geometrie der Vorrichtung 100 hergestellt werden kann. Die entstehende Geometrie der Module 120 und 130 ist im Detail so individuell wie die Zahnbögen der Probanden oder Patienten. Die Position der Module 120 und 130 relativ zum Schädel 200, zum Oberkiefer 220 und zum Unterkiefer 230 entscheidet darüber, wie tief und an welcher Stelle die Vertiefungen 124 und 134 für die Zähne entstehen und wie tief die Vertiefungen für das Zahnfleisch und die Weichgewebe ausgearbeitet werden, einschließlich Randspalt. Im Sagittalschnitt Fig. 12a sieht man die fast vertikal stehende Achse 250 von oben nach unten laufend. Die Vorrichtung 100 wird so positioniert, dass deren Vertikalachse 150 parallel zur Achse 250 und bevorzugt ganz oder fast genau in der Achse 250 verläuft. Die frontale Ansicht von vorne zeigt im Schnittbild Fig 12b die hier leicht schräg stehende rechts links Achse des Schädels 215. Diese wird als Bezugsachse verwendet und die Vorrichtung 100 wird digital im 3D Modell so positioniert, dass die Rechts-Links-Achse 115 der Vorrichtung 100 parallel zur Achse 215 verläuft. Die transversale Ansicht Fig 12c zeigt die Position der Achse 215 von oben gesehen. Die Schrägneigung der Achse 205 von vorne nach hinten und schräg nach unten führend ergibt sich aus der Position anatomischer Landmarken des Schädels, wie im Röntgenbild sichtbar. Die Orientierung der Vorrichtung 100 wird digital im 3D Modell so eingestellt, dass die Achse 105 der Vorrichtung 100 parallel zur Achse 205 verläuft und durch den Inzisionsbereich, der Überlappung zwischen den oberen und unteren Schneidezähnen hindurch. Wenn nun anhand des digitalen 3D Modells die Vertiefungen 124 und 134 für die Zähne und für das Zahnfleisch nebst Randspalten 122 und Kontaktpunkten 125 digital erzeugt werden, lassen sich genau diese Konturen auf reale Werkstücke übertragen. Nach der Fertigung liegt dann die der 3D Geometrie des Modells entsprechende Vorrichtung 100 bereit und kann bei dem individuellen Patienten, dessen Daten des Schädels und der Zähne man verwendet hat, eingesetzt werden. Die Vorrichtung 100 passt diesem Patienten so lange wie sich die Zahnpositionen nicht wesentlich verändern, solange also die Beweglichkeit an den Randspalten ausreichend ist.

Die Ausrichtung bzw. Lage der Module 120 und 130 und damit die Festlegung der Trenn-Gleit-Ebene 110 kann durch folgendes Verfahren vorteilhaft und präzise bestimmt werden.

Die nachfolgenden Figuren zeigen Aspekte der Erfindung in vereinfachter und schematisierter Darstellung. Als bevorzugtes Ausführungsbeispiel wird die Anwendung der 3D Strukturmarkierung am Beispiel des Kiefers und der zahnmedizinischen und kieferorthopädischen Behandlungsplanung erklärt und als Mittel zur Herstellung geeigneter Vorrichtungen. Die Einsatzmöglichkeiten sind jedoch nicht auf diese Anwendung begrenzt sondern betreffen allgemein die Biomechanik und die Neurophysiologie des Körpers.

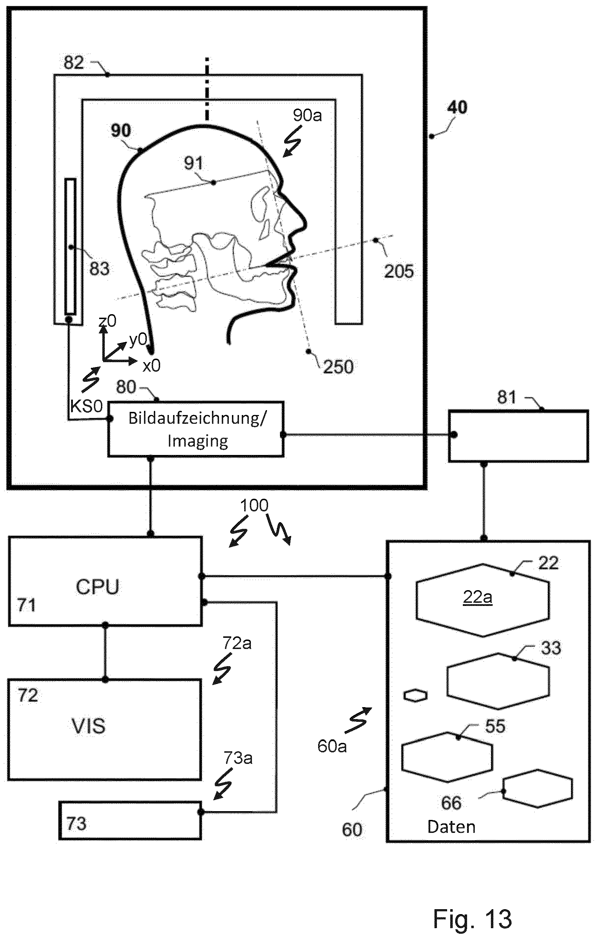

Fig. 13 zeigt eine Vorrichtung zur Erzeugung geeigneter Röntgenaufnahmen. Die 3D Röntgenaufnahmen werden in ein Rechnersystem übertragen und dort können Referenzobjekte in das Abbild des Schädels einkopiert, überlagert und darin ausgerichtet werden. Die Erzeugung dieser Referenzobjekte führt zu virtuellen 3D Bildern von Knochenstrukturen und 3D Strukturmarkierungen. In einem realen 3D Raum 40 befindet sich ein realer Körper mit Körperteil 90a, im Ausführungsbeispiel einem Kopf 90 eines Patienten. Darin befindet sich die knöcherne Struktur 91 des Schädels. Ein Arm 82 bewegt ein strahlensensitives Array 83, das nacheinander mehrere Aufnahmen aus unterschiedlichen Richtungen erstellt. Eine DVT (digitale/dentale Volumentomografie) Imaging Einheit 80 empfängt die Sensorinformationen und leitet sie direkt oder indirekt zu einem Rechnersystem 81, das aus den mehreren strahlensensorischen Bildern ein 3D Bild errechnet. Letztlich entsteht auf diese Weise ein Datensatz 22, der insbesondere die strahlungsabsorbierenden Gewebe und Materialien zeigt, insbesondere vorwiegend Knochen, Zähne und/oder Implantatmaterialien. Der Datensatz 22 enthält somit digitale Raumdaten 22a. Der Datensatz 22 wird in einem Datenspeicher 60 bzw. in einer Speichereinheit 60a abgelegt, ggf. nebst Datensätzen von Vorrichtungen 33 und Datensätzen von Strukturmarkierungen 55 sowie Datensätzen von anderen Hilfsmitteln 66, z.B. Achsen und Ebenen, die im 3D Bildraum positioniert, ausgerichtet und ein- oder ausgeblendet werden können. Über Datentransfer kann eine Recheneinheit 71 auf die Datensätze 22, 33 und 55 etc. zugreifen und aus den virtuellen 3D Bildern visualisierte Ansichten 72 erzeugen, z.B. als perspektivische Ansicht oder als Schnittbild. Die Fachleute verstehen es, mit den Bedienvorrichtungen 73 die Schnittebenen und die Ansichten 72 geeignet zu verändern und Datensätze bzw. Datenobjekte 22, 33, 55 und 66 etc. im 3D Raum geeignet einzublenden, zu verlagern und miteinander zu kombinieren. Angegeben wird die spezifische Technologie der 3D Strukturmarkierung, die hier durch den Datensatz 55 repräsentiert ist und die in den visualisierten Ansichten 72 angezeigt werden und mit den Bedienvorrichtungen 73 verschoben, verdreht und skaliert werden kann. Die visualisierten Ansichten 72 werden bspw. auf einer geeigneten Ausgabeeinheit 72a angezeigt, z.B. auf einem Monitor bzw. auf einem Display. Die Bedienvorrichtungen 73 können auch als Eingabeeinheit 73a bezeichnet werden und sind bspw. eine Computermaus, eine Tastatur, ein Trackball und/oder ein Sprachbefehls-Eingabesystem (Voice-System).

Bestandteile einer Datenverarbeitungsanlage 100 können sein: die Recheneinheit 71, die Ausgabeeinheit 72a, die Eingabeeinheit 73a und Speichereinheit 60a.

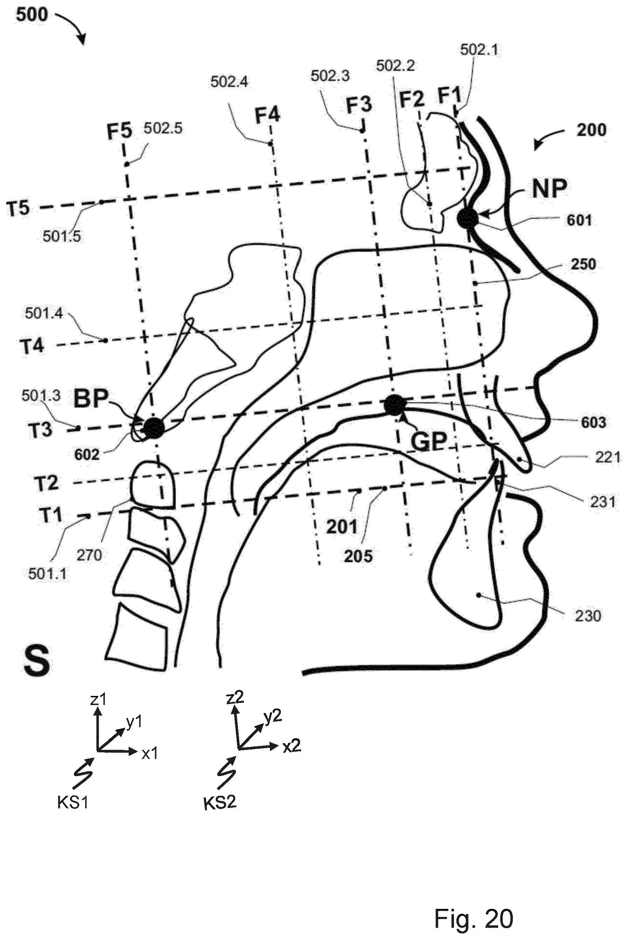

Ein optionales kartesisches Koordinatensystem KS0 kann im realen 3D Raum festgelegt werden. Das Koordinatensystem KS0 kann eine x-Achse x0, eine dazu orthogonale y-Achse y0 und eine zu beiden Achsen x0 und y0 orthogonale z-Achse z0 haben. Mit Bezug auf das Koordinatensystem KS0 kann im Bildraum ein erstes Koordinatensystem KS1 festgelegt werden, das wie das Koordinatensystem KS0 aufgebaut sein kann und dessen Koordinatenachsen in die gleiche Richtung wie die entsprechenden Koordinaten-Achsen x0, y0 und z0 zeigen, siehe z.B. Fig. 20.

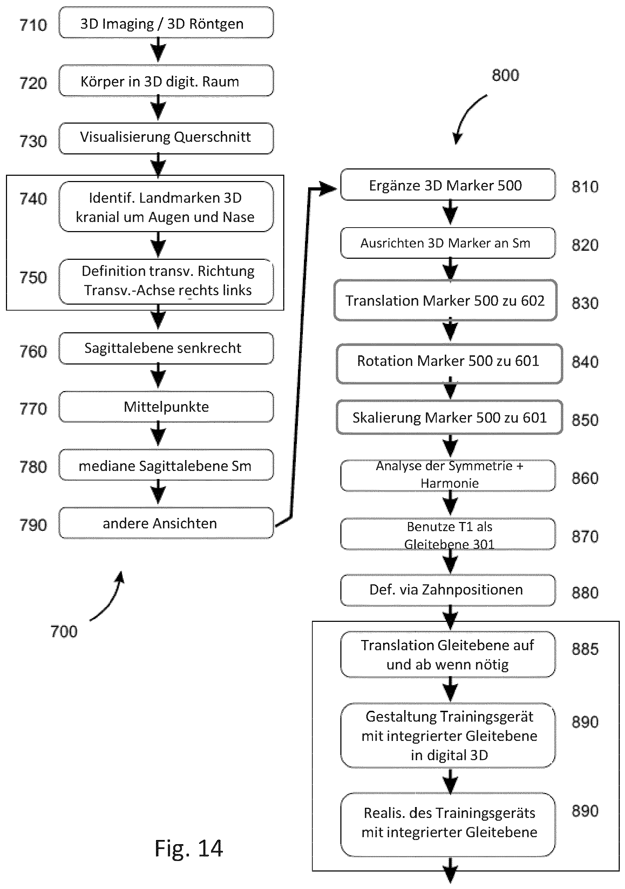

Fig. 14 zeigt ein Schema für das Verfahren zur individuellen Anwendung der 3D Strukturmarkierung. Zuerst kann die mediane Sagittalebene definiert werden, um die Strukturmarkierung danach auszurichten. Da die 3D Strukturmarkierung selbst transversale und frontale Ebenen aufweist, die als Schnittlinien transversal verlaufende Achsen 215 definieren, kann die 3D Strukturmarkierung im Grunde selbst benutzt werden, um die Sagittalebene zu definieren.

Im Folgenden wird ein bevorzugtes Verfahren beschrieben, um erst die Transversalrichtung anhand von Landmarken festzulegen, um dann die weiteren Anpassungsschritte durchzuführen. Dabei kann der Anwender entweder in einer Bildebene eine Achse wie z.B. 215 an eine Landmarke wie z.B. 281 anlegen, oder eine Ebene, die auf der Bildebene senkrecht steht, da im Schnittbild diese Ebene als Linie erscheint.

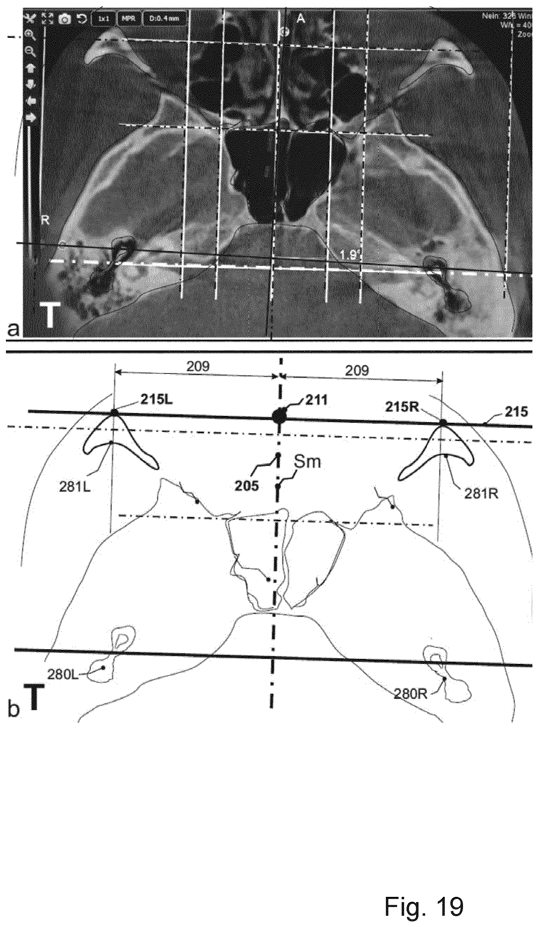

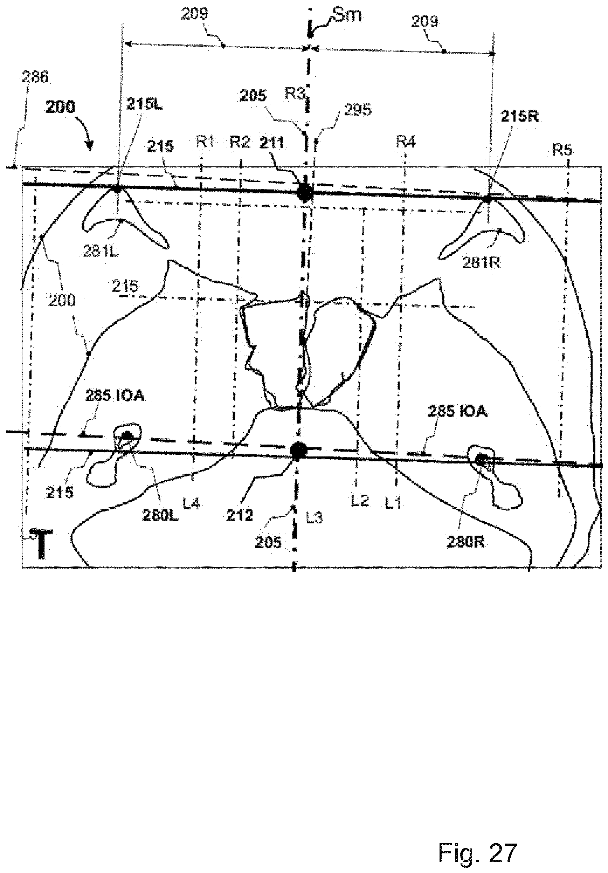

Verfahren 700: In einem ersten Schritt 710 erfolgt die Bilderzeugung eines 3D Bildes des Körperteils 90a, z.B. des Kopfes 90 eines Patienten. Durch die Verarbeitung der Bilder wird in Schritt 720 ein digitaler bzw. virtueller 3D Körper erzeugt. In Schritt 730 werden Schnittbilder dieses 3D Körpers angezeigt (siehe Fig. 19a). In Schritt 740 werden in geeignet gewählten Schnittbildern Landmarken der knöchernen Strukturen identifiziert (siehe Fig. 19b, 281L und 281R). Im Schritt 750 wird mindestens eine transversale Richtung definiert, die zwei symmetriegebende Landmarken verbindet (siehe Fig. 19b, 215). Das kann durch eine Achse (im Ausführungsbeispiel 215) geschehen oder durch eine Ebene und eine dazu gekreuzte zweite Ebene. Im Schritt 760 erfolgt die Definition der Lage der Sagittalebenen 503 (Sm) deren dazu orthogonal stehende Normale die Transversalachse 215 ist. Im Schritt 770 werden die Mitten 211 zwischen geeigneten Landmarken 281 bestimmt, um die mediane Sagittalebene Sm des oberen Gesichtsschädels genau festlegen zu können (siehe Fig. 19 und 27). Dies kann nicht ganz eindeutig sein, weil der Schädel im hinteren Bereich oft eine um bis zu 2 Grad andere Ausrichtung hat als im vorderen oberen Gesichtsschädel (siehe Fig. 27). In Schritt 790 oder einer Vielzahl solcher Schritte werden andere Schnittbilddarstellungen eingenommen, um Positionen und Richtungen zu kontrollieren oder andere Aufgaben und Fragen zu erledigen. Die Reihenfolge der Schritte kann verändert werden, solange das Ergebnis darin besteht, dass mit Hilfe der Landmarken die Orientierung der Sagittalebene definiert wird.

Verfahren 800: In einem ersten Schritt 810 wird die 3D Strukturmarkierung 500 in den 3D Bildraum eingebracht und eingeblendet, so dass man sie in der Anzeige sieht. Die Ebenenpakete erscheinen nun als parallele Linien. In einem Schritt 820 wird die Strukturmarkierung mit ihrer inneren Sagittalebenenstruktur 503 senkrecht zur Transversalachse 215 der knöchernen Strukturen ausgerichtet. In einem davon unabhängigen Schritt 830 wird die 3D Strukturmarkierung 500 auf einen Ankerpunkt 602 in der medianen Sagittalebene gelegt, im Ausführungsbeispiel mit dem Kreuzungspunkt der Linien bzw. Ebenen T3 und F5 in der medianen Sagittalebene auf den Basis-Punkt BP. In einem Schritt 840 wird die 3D Strukturmarkierung 500 durch Drehung um eine zur Transversalachse 215 parallele Achse so orientiert, dass im Sagittalschnittbild die Transversalebenen 501 in Richtung der Verbindungslinie 602-603 verlaufen, also in Richtung der Achse BP-GP. Im Ausführungsbeispiel erfolgt die Drehung um den Fixpunkt 602, auf dem der Kreuzungspunkt T3 und L5 liegt. Die Drehung bringt die Linie T3 in eine Position, dass sie durch Ankerpunkte 602 und 603 verläuft. Damit ist die 3D Strukturmarkierung 500 im Winkel ausgerichtet.

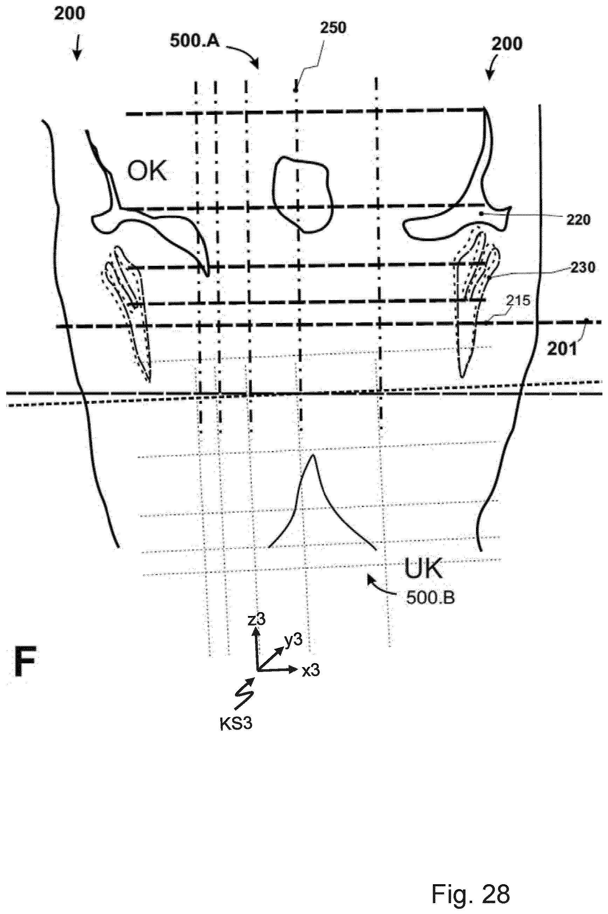

In einem Schritt 850 wird die Größe der 3D Strukturmarkierung 500 so angepasst, dass eine Frontallinie durch einen geeignet gewählten Ankerpunkt 601 verläuft. Im Ausführungsbeispiel ist der Ankerpunkt 601 der Nasen-Punkt NP, siehe Fig. 20, 21, 22. Die individuell richtige Größe ist im Ausführungsbeispiel erreicht, wenn die Frontallinie F1 und damit die Frontalebene 502.1 durch den Nasen-Punkt NP verläuft. In einem nachfolgenden Schritt 860 kann nun optional die Symmetrie und die Harmonie der Proportionen im Körperteil analysiert werden. Dabei zeigen sich oft Verkippungen z.B. der Kiefer und der aktuellen Kauebene (siehe Fig. 27 und 28). In einem weiteren Schritt 870 wird die Transversalebene 501.1 (T1) als idealisierte Okklusionsebene verwendet, um z.B. Zahnersatz oder Vorrichtungen danach auszurichten. Im Fall von trennbaren Vorrichtungen 300 mit Oberteil 320 und Unterteil 330 wird besonders bevorzugt die Trennebene 301 zwischen den Teilen als Trenn-Gleit-Ebene 301 ausgeführt, ohne Retentionen (Überstände) und mit sehr geringer Reibung. Die Position der Trenn-Gleit-Ebene 301 wird im digitalen bzw. virtuellen 3D Raum bei Planung und Formgebung der Vorrichtung möglichst genau auf die Position der idealisierten Okklusionsebene 201 gelegt.

In einem nach dem Verfahrens-Schritt 870 folgenden Schritt 880 wird der Freiraum für die Zähne und oder Zahnimplantate der oberen und unteren Zahnbögen geschaffen, so dass die Vorrichtung später genau auf die individuellen Zahnbögen passt, ohne zu klemmen oder das Zahnfleisch zu verletzen.

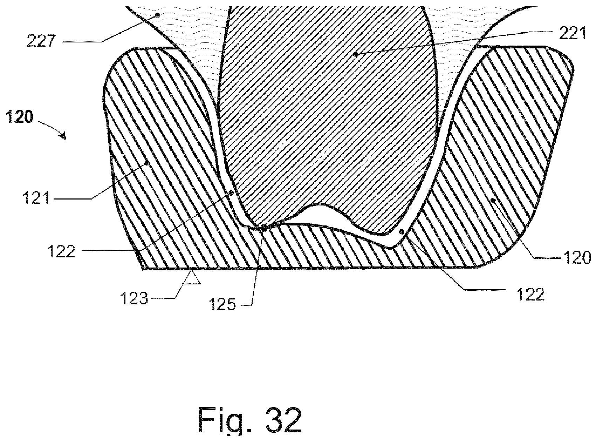

Dabei entsteht durch die Fertigstellung der Form im Schritt 880 mit geplantem Formschluss zwischen Vorrichtungen und Zahnbogen eine exakte Definition der Position der Vorrichtung 300 mit ihren Teilen 320 und 330 im Mund (siehe Fig. 32). In einem nachfolgenden Schritt 890 wird die Vorrichtung 300 in ihren Teilen 320 und 330 für genau diesen individuellen Fall gefertigt und kann anschließend für diesen Patienten verwendet werden.

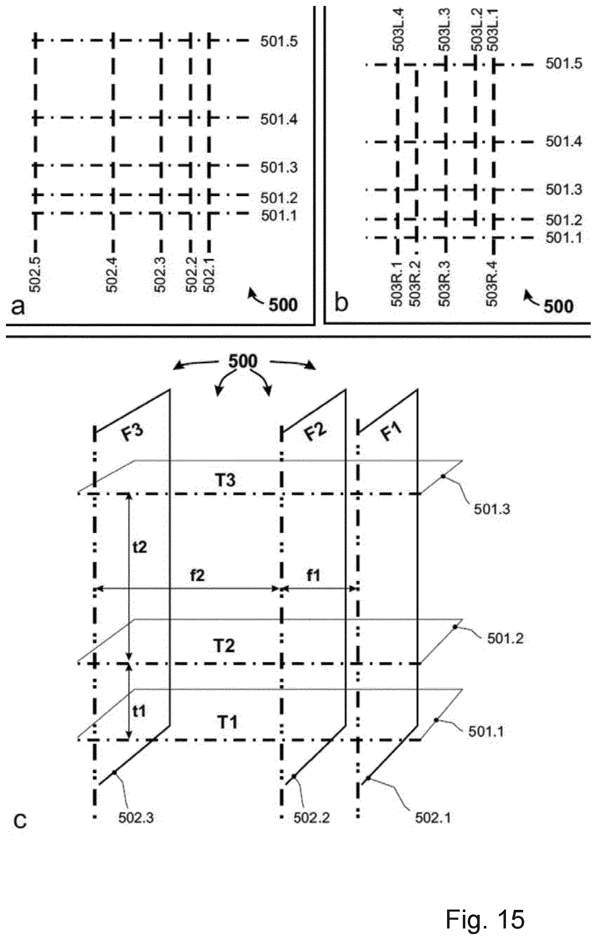

Fig 15. zeigt die Anwendung eines Referenzobjektes 500 als 3D Marker, der aus 3 Stapeln senkrecht aufeinander stehender paralleler Ebenen besteht, wobei die Abstände der parallelen Ebenen einer harmonischen Abstandsbeziehung folgen. Dies ist ein besonders bevorzugtes Ausführungsbeispiel eines Referenzkörpers, der dazu verwendet wird, die craniale Symmetrie und Orientierung im 3D Bildraum zu erkennen und anhand der individuell angepassten Referenzvorrichtung die Trenngleitebene der Trainingsvorrichtung individuell genau korrekt auszurichten, auch wenn diese dann schräg zur individuellen Kauebene stehen mag, was in den allermeisten therapiebedürftigen Fällen der Fall ist.

Fig. 15 zeigt die Kombination von mindestens zwei Ebenenpaketen zum 3D Strukturmarker 500. Fig. 15a zeigt die Ansicht der 3D Strukturmarkierung 500 in einem sagittalen Schnittbild. Fig. 15 zeigt das 3D Referenzobjekt 500 im Schnittbild und unter 15c in halbperspektivischer Darstellung. Während die obere Transversalebene T3 anhand der anatomischen Landmarken in der Gegend der Augenhöhlen ausgerichtet wird, definiert die Lage der unteren Transversalebene T1 die Lage der Trenngleitebene. Weil die sagittalen Ebenen in der Bildebene liegen, erscheinen nur die Schnittlinien mit den transversalen Ebenen des Ebenenpakets 501 und die Schnittlinien mit den frontalen Ebenen 502. Das bevorzugte Ausführungsbeispiel hat je 5 Ebenen pro Ebenenpaket, also T1 bis T5 und F1 bis F5. Die gezeichneten Abstände gehorchen im besonders bevorzugten Ausführungsbeispiel der Fibonacci-Folge und es gilt t1 = 3, t2=5, t3=8, t4=13, jeweils in skalierbaren Einheiten. Das Analoge gilt hier für die Abstände f der frontalen Ebenen F. In den Ansprüchen gilt:
- T ist eine erste Ebene, d.h. eine Transversalebene,
- F ist eine zweite Ebene, d.h. eine Frontalebene, sowie
- S ist eine dritte Ebene, d.h. eine Sagittalebene.

Fig. 15b. zeigt die Struktur 500 in der frontalen Ansicht von vorne. Wieder erkennbar ist die Folge der transversalen Schnittlinien 501.1 bis 501.5 des transversalen Ebenenpakets 501 (T1 bis T5). Die vertikal verlaufenden Schnittlinien sind die sagittalen Ebenen des doppelten Ebenenpaketes 503, zusammengesetzt aus einem Teilebenenpaket 503R und einem Teilebenenpaket 503L, wobei hier jeweils nur 4 Ebenen pro Teilpaket gezeichnet sind, d.h. insgesamt 8 Ebenen. Wegen der Fibonacci-Folge mit den Zahlen 3+5=8 und 5+8=13 fallen die Ebenen zumindest teilweise identisch zusammen. Nur die beiden Ebenen 503R.2 und 503L.2 stehen alleine. Ansonsten gilt z.B. Ebene 503R.4 ist identisch mit Ebene 503L.1. Das sagittale Ebenenpaket 503 ist im besonders bevorzugten Ausführungsbeispiel symmetrisch zur medianen Sagittalebene 503R.3 = 503L.3.

Fig. 15c zeigt ein Ausführungsbeispiel einer 3D Strukturmarkierung 500 in perspektivischer Ansicht. Drei transversale Ebenen 501.1 (T1), 501.2 (T2) und 501.3 (T3) bilden das Ebenenpaket 501. Drei weitere dazu senkrecht stehende Ebenen 502.1 (F1), 502.2 (F2), 503.3 (F3) bilden das Ebenenpaket 502. Die Ebenenabstände sind mit Kleinbuchstaben bezeichnet als f1 zwischen F1 und F2 sowie f2 zwischen F2 und F3, entsprechend als t1 zwischen T1 und T2 sowie t2 zwischen T2 und T3. Diese Abstände sind charakteristisch und ihr Verhältnis verändert sich bei der Skalierung der 3D Strukturmarkierung 500 nicht. Im sagittalen Schnittbild sieht man die Schnittlinien T1 bis T3 und senkrecht dazu die Schnittlinien F1 bis F3. Die Abstände der Ebenen entsprechen im orthogonalen Schnitt den Abständen der Schnittlinien.

Ein optionales kartesisches Koordinatensystem KS2 kann bezüglich der 3D Strukturmarkierung 500 festgelegt werden. Das Koordinatensystem KS2 kann eine x-Achse x2, eine dazu orthogonale y-Achse y2 und eine zu beiden Achsen x2 und y2 orthogonale z-Achse z2 haben. Eine durch die x-Achse x2 und durch die y-Achse y2 aufgespannte Ebene kann parallel zu den transversalen Ebenen 501.1 (T1), 501.2 (T2) und 501.3 (T3) des Ebenenpaketes 501 liegen. Eine durch die y-Achse y2 und durch die z-Achse z2 aufgespannte Ebene kann parallel zu den Frontal-Ebenen 502.1 (F1), 502.2 (F2), 503.3 (F3) des Ebenenpaketes 502 liegen. Eine durch die x-Achse x2 und durch die z-Achse z2 aufgespannte Ebene kann parallel zu Sagittalebenen S bzw. 500R.1 bis 500R.5 des Teilebenenpaketes 503R bzw. zu Sagittalebenen S bzw. 500L.1 bis 500L.5 des Teilebenenpaketes 503L liegen.

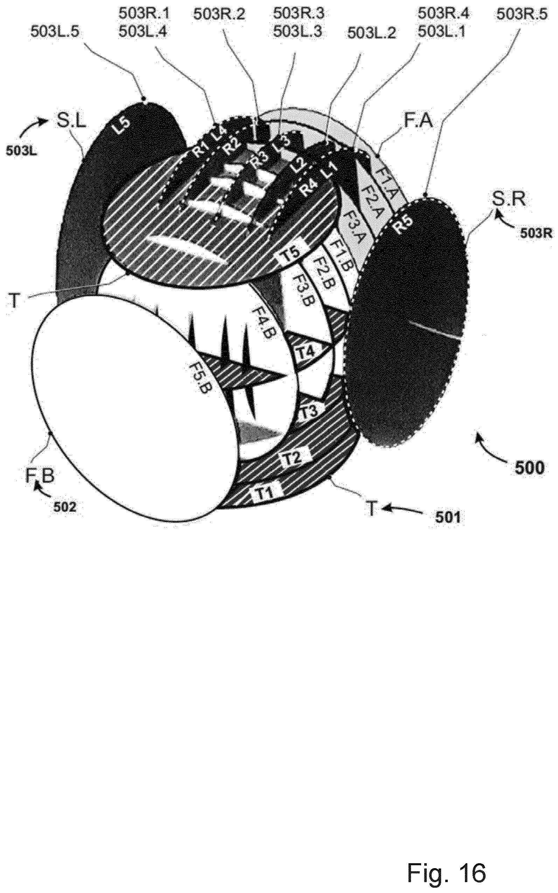

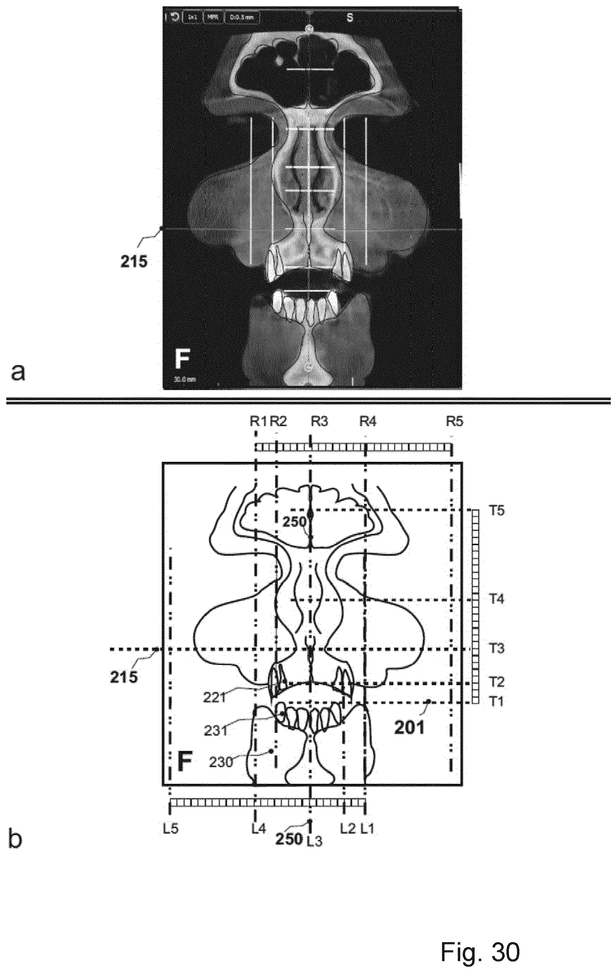

Fig. 16 zeigt Darstellungen des 3D Strukturmarkers mit zusätzlichem sagittalem Ebenenpaket 503. Fig 16. zeigt die komplexe halbperspektivische Darstellung der drei gekreuzten Ebenenstapel 501, 502 und 503, die zur Definition der Lage der Trenngleitebene ganz individuell angepasst wird, ohne dass sich durch die Dehnung und Drehung und Verschiebung die Harmonie der Abstandsverhältnisse ändern würde. Die Harmonie der Abstandsverhältnisse der Ebenen betrifft vor allem auch die Lage der Frontalebenen F, die die Transversalebenen T jeweils in einer Linie schneiden, die von rechts nach links läuft. Diese Schnittlinien geben die Orientierung der Verbindungslinie zwischen den Auflagepunkte 125.1 rechts und links und den Auflagepunkten 125.2 rechts und links in Figur 30 vor. Die perspektivische Darstellung eines besonders bevorzugten Ausführungsbeispiels der 3D Strukturmarkierung zeigt die orthogonale Anordnung der Ebenenpakete 501, 502 und 503, wobei die Einzelebenen jeweils als kreisrunde Scheiben dargestellt sind, die in der Perspektive elliptisch erscheinen. Jedes der Ebenenpakete hat ein anderes Muster. Von unten nach oben aufsteigend horizontal sind die Transversalebenen 501 (T1 bis T5) gezeichnet, mit schwarzweiß gestreiftem Muster. Ganz rechts in schwarz sieht man die fünfte Ebene R5 des nach rechts laufenden Teil-Ebenenpakets 503R, gegenüber die letzte ebene L5 des nach links laufenden Teil-Ebenenpakets 503L. Nochmals senkrecht dazu stehen die frontalen Ebenenpakete, wobei hier zwei Pakete A und B überlagert sind. Auch hier gilt für die Abstände innerhalb der Teil-Pakete die Fibonacci-Reihe 3, 5, 8, 13, 21 etc. Für die Anwendung im Bereich des knöchernen Schädels beträgt der Durchmesser der Ebenenpakete mindestens 6 cm (Zentimeter) bis 30 cm. Ein Höchstmaß für den Durchmesser der Ebenenpakete für die Anwendung im Bereich des knöchernen Schädels kann im Bereich von 35 cm bis 50 cm liegen. Für andere Anwendungen an anderen Körperteilen wie z.B. an Nacken und Rücken sind die Ebenenpakete größer. Die kreisförmigen Ränder haben allerdings nur grafische Bedeutung für die bessere Visualisierung. Mathematisch haben die Ebenen keine Grenze. In der Fig. 16 würde eine linke Seite L des Schädels bzw. des Kraniums auf der linken Seite liegen. Eine rechte Seite R des Schädels bzw. Kraniums würde auf der rechten Seite liegen.

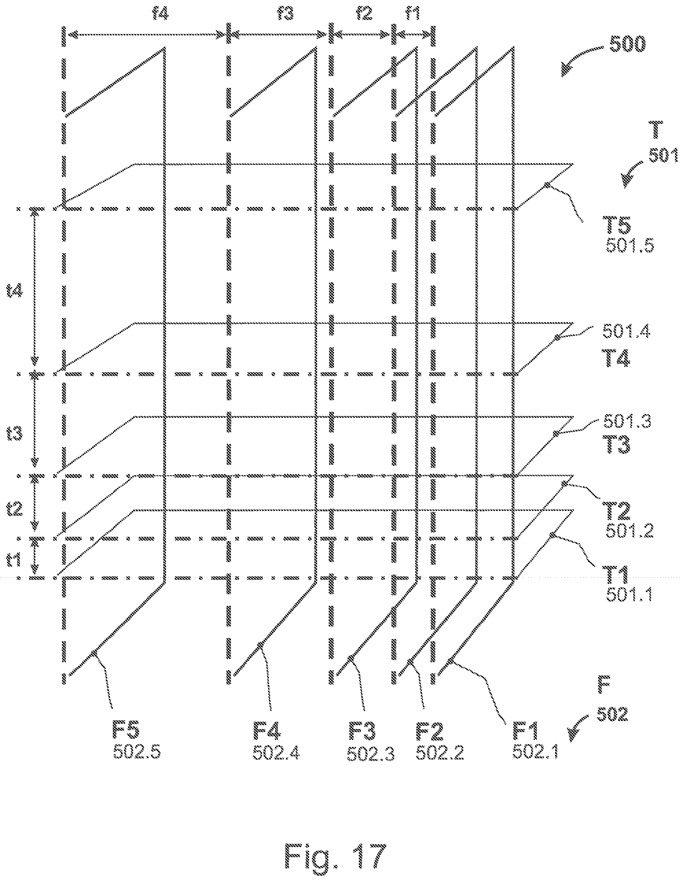

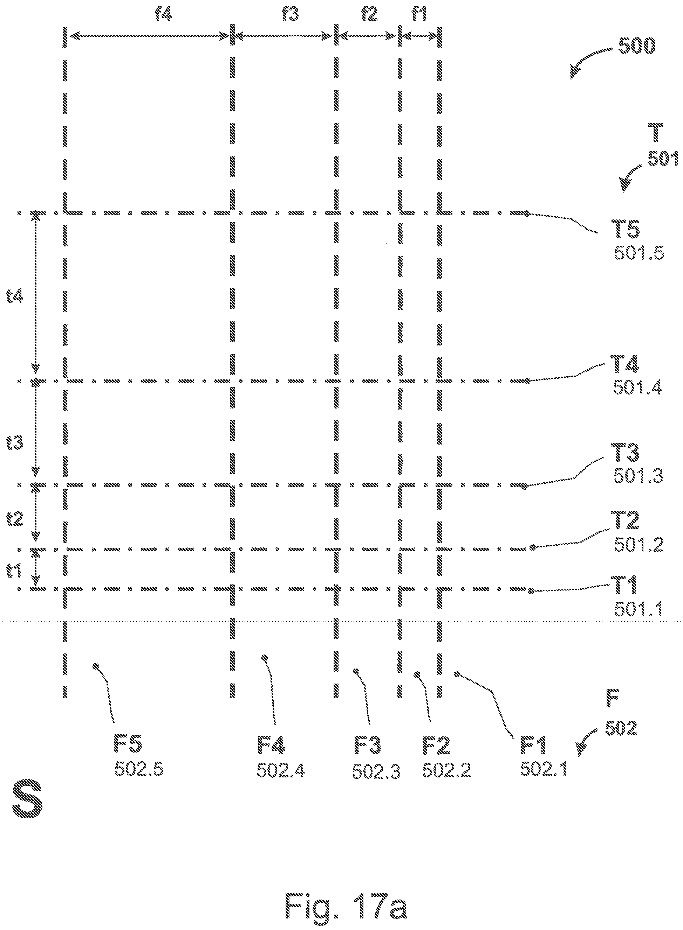

Fig. 17 zeigt eine besonders bevorzugte isotrope Ausführung derje fünf Ebenenpakete 501 und 502. In einer besonders bevorzugten Ausführungsform umfasst das Ebenpaket 501 (T) fünf transversale parallele Ebenen 501.1 (T1) bis 501.5 (T5). Orthogonal dazu gekreuzt steht das Ebenenpaket 502 (F) aus fünf frontalen parallelen Ebenen 502.1 (F1) bis 502.5 (F5). Optional kommen weitere Ebenenpakete hinzu und bilden gemeinsam die 3D Strukturmarkierung. Die Abstände der Ebenen verhalten sich besonders bevorzugt im harmonischen Verhältnis gleicher Proportionalität, die hier abgebildete Abstandsfolge lautet t1=3, t2=5, t3=8, t4=13 und analoges für f1 bis f4. Es können auch andere Abstandsverhältnisse oder andere Abstandsmuster verwendet werden. Kennzeichnend sind die orthogonal gekreuzten Ebenenpakete. Gestrichelt dargestellt sind die orthogonal gekreuzten Linien, die im sagittalen Schnittbild angezeigt werden. In der Richtung Sagittalebene senkrecht auf 501 und 502 stehend geschnitten sieht man die sagittalen Ebenenpakete nicht.

Fig. 17a zeigt eine Vorderansicht von Figur 17, in welcher die fünf transversalen parallelen Ebenen 501.1 (T1) bis 501.5 (T5) dargestellt sind. Orthogonal dazu gekreuzt steht das Ebenenpaket 502 (F) aus fünf frontalen parallelen Ebenen 502.1 (F1) bis 502.5 (F5). Weiterhin sind die Abstände der Ebenen t1, t2, t3 und 4 dargestellt.

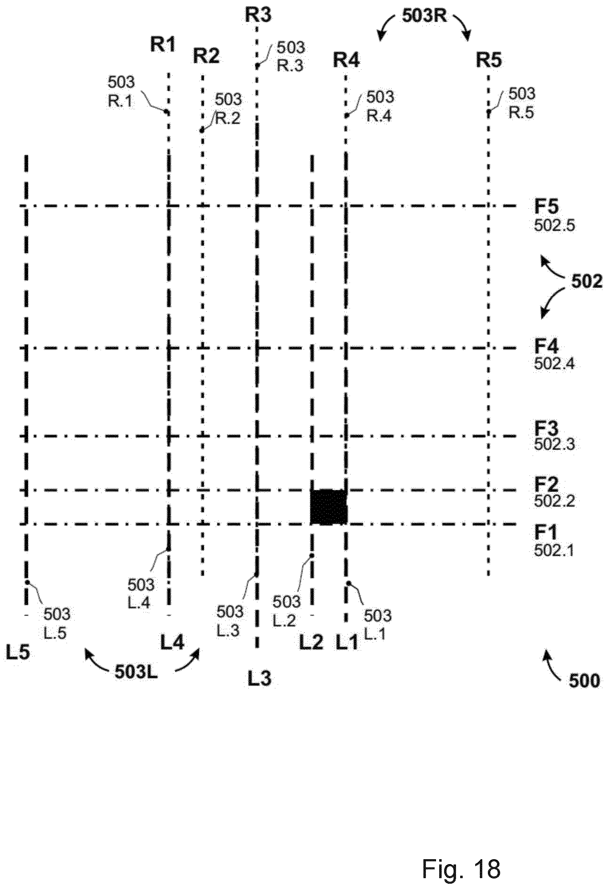

Fig. 18 zeigt die Ergänzung der Ebenenpakete durch ein doppeltes Ebenenpaket 503R und 503L bzw. durch Teilebenenpakete 503R und 503L. Es ergibt sich ein transversales Schnittbild durch die 3D Strukturmarkierung 500. Die Anordnung der sagittalen Ebenen im transversalen Schnittbild zeigt die Frontalebenen 502 (F1 bis F5) in Rechts-Links-Richtung verlaufend und senkrecht dazu die beiden Teilebenenpakete 503R (R1 bis R5) und 503L (L1 bis L5) als Schnittlinien der planparallelen Sagittalebenen in Anterior-Posterior-Richtung verlaufend. Die Sagittalebenen L3 und R3 bilden im hier gezeigten bevorzugten Ausführungsbeispiel die mediane Sagittalebene. Die Abstände der Ebenen verhalten sich besonders bevorzugt im harmonischen Verhältnis gleicher Proportionalität, die hier abgebildete Abstandsfolge lautet I1=3, I2=5, I3=8, I4=13. Es können auch andere Abstandsverhältnisse oder andere Abstandsmuster verwendet werden.

Fig. 19 zeigt den Transversalschnitt durch die knöcherne Struktur eines Schädels bzw. Kraniums 200 und zeigt, wie die Transversalachse 215 an Landmarken ausgerichtet und damit die Sagittalebene Sm definiert wird. Fig. 19 zeigt ein Ausführungsbeispiel wie die Lage des Referenzkörpers 500 anhand anatomischer Landmarken 215L und 215R im Bereich der äußeren Augenhöhlen individuell mit Hilfe der 3D Röntgenabbildung ausgerichtet wird, um dann mit Hilfe des angepassten Referenzkörpers 500 die Ausrichtung der Trenngleitebene 110 115 und die Position der okklusalen Auflagebereiche zu bestimmen. Fig. 19b zeigt am Beispiel, dass die craniale Symmetrie im Bereich der Augenhöhlen eine Transversalachse 215 zeigt, die nicht parallel zur Innenorhrachse liegt. Verschiebt man die im weiteren verwendete craniale Transversalchase parallel 215 zum Innenohr, sieht man, dass das Innenohr nicht rechts und links gleich durchschnitten wird. Die Transversalchse 215 ist also nicht gleich der Innenohrachse nach Hornung. Oben in Fig. 19a ist ein röntgenologisch erzeugter Transversalschnitt durch den Schädel bzw. durch das Kranium 200 einer Person gezeigt. Darin sind überlagert die Konturen näherungsweise eingezeichnet. Die Konturenfindung kann bevorzugt durch 3D Bildsoftware erfolgen. Darunter in Fig. 19b sieht man die knöchernen Konturen des Kraniums 200 und darin die geeigneten Landmarken 281L und 281R, die einen Transversalschnitt durch die Jochbeinbögen zeigen. Die mit 215L und 215R markierten Punkte sind im gezeigten Fall Tangentialpunkte, die sich z.B. eignen, um die Achse 215 in der transversalen Bildebene auszurichten. Eine ähnliche Ausrichtung kann bzw. sollte auch in der Frontalebene erfolgen, damit die Lage der Achse definiert ist. In der Frontalebene bzw. in einem Frontalschnitt können ebenfalls die Jochbeine als Landmarken bzw. Merkmale zur Ausrichtung der Achse 215 dienen. Alternativ können auch andere Landmarken verwendet werden, bspw. die unteren Ränder der Augenhöhlen. Mit der Richtung der transversal zwischen Rechts und Links verlaufenden Achse 215 ist zwar die Richtung der Sagittalebenen definiert. Die Definition der Koordinatenlage der medianen Sagittalebene Sm erfordert jedoch die zusätzliche Angabe des Mittelpunktes 211. Diesen bestimmt man bspw. messtechnisch als Mitte der Strecke zwischen 215L und 215 R oder als Mittelpunkt vergleichbarer Landmarken rechts und links, wie man sie auch z.B. weiter hinten im Bereich des Mittelohrs 280 L und 280R finden kann.

Fig. 20 zeigt ein Ausführungsbeispiel für die Orientierung des Referenzkörpers 500 von der Seite gesehen im Sagittalschnitt. Auch hier erfolgt die Definition des nun von der Seite sichtbaren Kippwinkels der Trenngleitebene, die meist von der Neigung der Kauebene abweicht, durch den Referenzkörper 500, indem dieser an den Basionpunkt BP und an den Gaumendachpunkt GP und an den Nasionpunkt NP individuell angepasst wird, wie es in Fig. 20 gezeigt ist. Dadurch wird die relative Orientierung und Lage der Transversalebene definiert, die senkrecht zur Sagittalebene bzw. Bildebene durch die Linie T1 verläuft. Dargestellt ist das median sagittale Schnittbild durch den in einem digitalen bzw. virtuellen Raum 50 röntgenologisch dargestellten Kopf mit Ankerpunkten 601 (NP), 602 (BP) und 603 (GP) sowie mit eingepasster 3D Strukturmarkierung. Das sagittale Schnittbild geht auch durch die 3D Strukturmarkierung 500 nach individueller Einpassung in die knöchernen Strukturen des Kraniums 200 eines individuellen Kopfes 90, siehe Figur 1. Die Ankerpunkte 601 (Nasen-Punkt NP) und 602 (Basis-Punkt BP) sowie 603 (Gaumen-Punkt GP) dienen dazu, die 3D Strukturmarkierung 500 korrekt an den knöchernen Landmarken zu positionieren. In den Ansprüchen gilt:
- Ankerpunkt 601 (Nasen-Punkt NP) ist ein drittes Ankermerkmal,
- Ankerpunkt 602 (Basis-Punkt BP) ist ein erstes Ankermerkmal, sowie
- Ankerpunkte 603 (Gaumen-Punkt GP) ist ein zweites Ankermerkmal.

Um die Sagittalebene korrekt einzustellen, hat vorher bereits eine Ausrichtung der Transversalachse stattgefunden, die grundsätzlich auf der Sagittalebene senkrecht steht. Die Sagittalebene ist die Bildebene. Sie wird aufgespannt durch die senkrechten Achsen 205 und 250 des Kraniums 200. Der Nasen-Punkt 601 (NP) bezeichnet den Scheitel oberhalb der Nasenwurzel. Dreidimensional definiert wird er durch gleichzeitige Berücksichtigung des Scheitels der Krümmung dieses Bereichs im Transversalschnitt. Der Gaumen-Punkt 603 (GP) wird definiert durch die höchste Stelle der knöchernen Kuppel des Gaumens, die man im Sagittalschnitt von der Seite sieht und im Frontalschnitt von vorne. Genau betrachtet liegt der Gaumen-Punkt 633 (GP) in der etwas weniger dichten Spongiosa (schwammartiges Innengewebe der Knochen) zwischen den kompakten Rändern des Gaumengewölbes und der Schädelbasis. Der Basis-Punkt 602 (BP) ist definiert als der Scheitel des Knochens, der das Foramen Magnum umringt. Man kann ihn im Sagittalschnitt gut sehen und ebenso im Transversalschnitt. Unterhalb des Basis-Punktes 602 (BP) sieht man die Konturen der obersten Ausläufer der Halswirbelsäule 270. Die Gitterlinien entstehen durch den Schnitt der Ebenenpakete T1 bis T5 und F1 bis F5 mit der medianen sagittalen Bildebene. Die 3D Strukturmarkierung wird so positioniert und skaliert, dass folgende Ankerpunkte fixiert sind:
Schnittpunkt der Linie (Ebene) T3 mit der Linie (Ebene) F5 liegt auf Basis-Punkt BP
- Linie T3 verläuft durch Basis-Punkt BP nach vorne durch Gaumen-Punkt GP
- Linie F1 verläuft darauf senkrecht stehend nach oben durch Nasen-Punkt NP.
- Die Position von GP liegt zufällig an der Linie F3, das muss aber nicht so sein.

Die vorliegende Situation in Fig. 19 zeigt, dass der Unterkiefer 230 tendenziell zu klein ausgeprägt ist, folglich liegen die Zähne des Unterkiefers 231 eher zu weit oben und die aktuelle Okklusionsebene liegt etwas zu weit oben. Die physiologisch optimale Okklusionsebene liegt direkt auf T1, die sich mit der Hauptachse 205 deckt. Trainingsvorrichtungen 300 für diesen Patienten bekommen die Trenn-Gleit-Ebene in der Position der Ebene T1, um physiologisch bestmögliche Resultate zu erzielen.

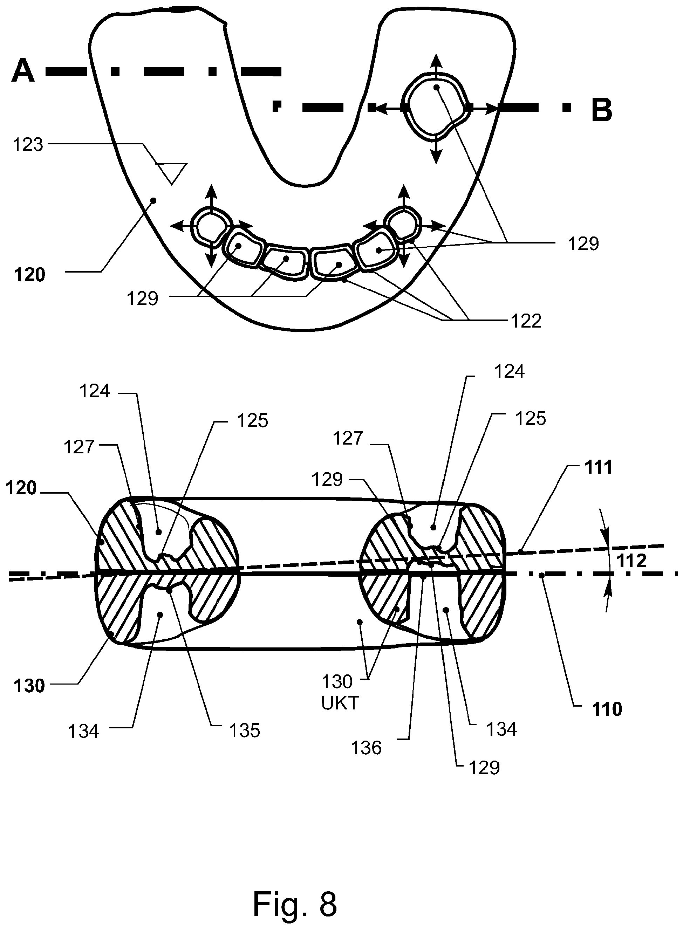

Die Fig. 20 zeigt ein optionales erstes Koordinatensystem KS1 des digitalen Bildraumes 50 und das optionale Koordinatensystem KS2, dass der Strukturmarkierung 500 zugeordnet werden kann. Das Koordinatensystem KS1 kann eine x-Achse x1, eine dazu orthogonale y-Achse y1 und eine zu beiden Achsen x1 und y1 orthogonale z-Achse z1 haben. Fig. 19 zeigt das Ergebnis einer Orientierung der Strukturmarkierung 500, die auch als zweites Bezugssystem bezeichnet werden kann, an den Ankerpunkten 601 bis 603. Die einzelnen Schritte dieser Orientierung werden im Folgenden an Hand der Figuren 20 (Ausrichtung/ Positionierung), 21 (Drehung/ Orientierung) und 22 (optionale Skalierung) näher erläutert. Nach der Orientierung der Strukturmarkierung 500 würde das optionale Koordinatensystem KS2 bezüglich des optionalen Koordinatensystem KS1 die in der Fig. 8 gezeigte Lage haben. Beide Koordinatensysteme KS1 und KS2 wären zueinander in ihrer relativen Lage fixiert, wenn am Ende der Orientierung der Strukturmarkierung 500, die Strukturmarkierung 500 bezüglich der digitalen Bilddaten bzw. Raumdaten 22a fixiert wird.

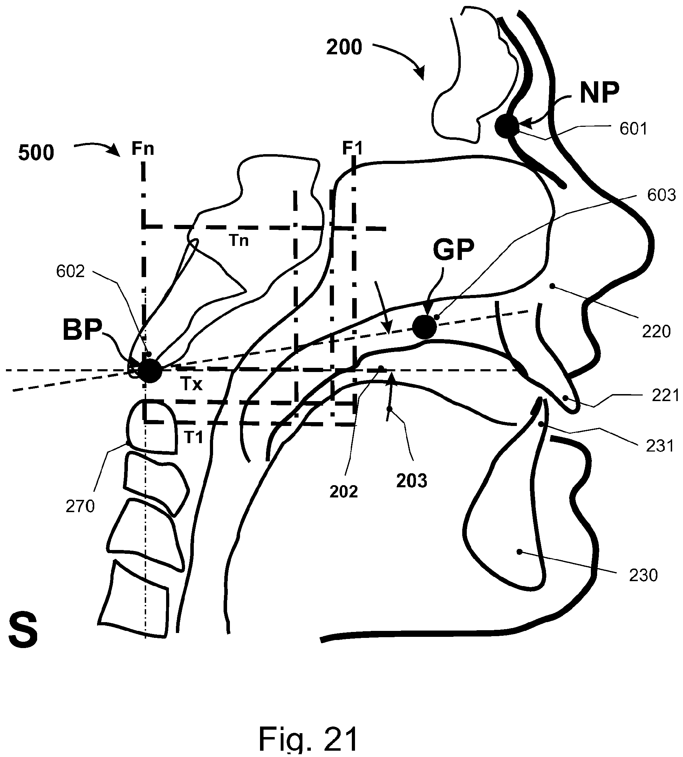

Fig. 21 zeigt die Strukturmarkierung 500 im Sagittalschnitt vor der Drehung und Skalierung etc. Die Strukturmarkierung 500 ist hier in einer vereinfachten Form, weil für die Beschreibung des Verfahrens der Positionierung, Orientierung und Skalierung nur wenige Ebenen erforderlich sind. Das transversale Ebenenpaket 501 umfasst mindestens die Ebenen T1, Tx und Tn sowie F1 und Fn. Im bevorzugten Ausführungsbeispiel aus Fig. 21 ist n=5 und x=3. Die Positionierung bzw. Ausrichtung erfolgt vorzugsweise durch translatorische Bewegungen so, dass der Schnittpunkt zwischen Tx und Fn auf den hinteren Ankerpunkt 602 (BP) gelegt wird. Der vordere Ankerpunkt 603 wird im Ausführungsbeispiel als Gaumen-Punkt GP bezeichnet. Der obere Ankerpunkt 601 wird im Ausführungsbeispiel als Nasen-Punkt NP bezeichnet. Die 3D Strukturmarkierung 500 wird bevorzugt im medianen Sagittalschnittbild so verschoben, dass hier z.B. der Schnittpunkt der Linien Tx und Fn als Drehpunkt im Ankerpunkt liegt, hier also im Basis-Punkt BP. Nimmt man die besonders bevorzugte Ausführungsform der Strukturmarkierung mit je fünf Ebenen aus Fig. 18 und Fig. 21, so ist der Dreh- und Ankerpunkt der Kreuzungspunkt der Linie T3 mit der Linie L5. Ein Winkel 203 zeigt die Neigung einer Linie durch die Ankerpunkte 602 und 603 bezüglich der horizontalen Ebene im Koordinatensystem KS1. Speziell die Schrägneigung der Trenn-Gleit-Ebene 110 ergibt sich im obigen Ausführungsbeispiel aus der Lage der cranialen Punkte BP Basionpunkt und GP Gaumendachpunkt, sobald man im digitalen 3D Raum das individuell durch Dehnung und Drehung anpassbare 3D Referenzobjekt 500 an die craniale Anatomie des individuellen Kopfes anpasst. Liegt z.B. das Gaumendach höher, so verläuft die Trenn-Gleit-Ebene 110 steiler nach oben. In einem alternativen Ausführungsbeispiel werden andere Ebenen angelegt, wobei die craniale Orientierung in der Transversalachse und der Sagittalebene bleibt, wobei sich andere Positionen und Drehwinkel des Referenzobjekt 500 ergeben und folglich eine andere Neigung der Trenn-Gleit-Ebene 110.

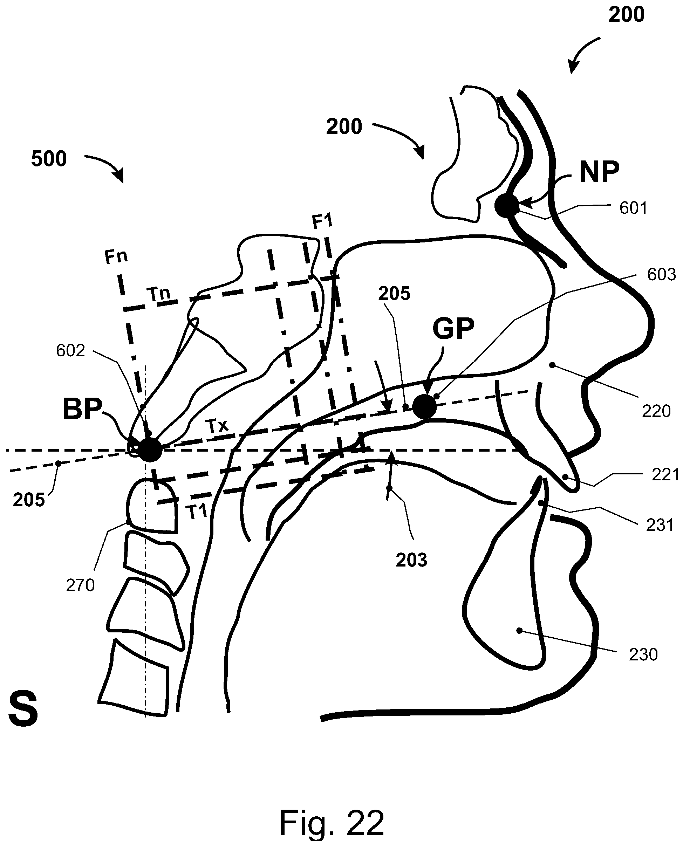

Fig. 22 zeigt die Strukturmarkierung 500 in Orientierung entlang der Achse durch den Ankerpunkt 602 (BP) und den Ankerpunkt 603 (GP). Die Strukturmarkierung 500 wird bevorzugt nach dieser Positionierung bzw. Ausrichtung um den Ankerpunkt 602 gedreht, wodurch sich die Raumrichtung 205 der Transversallinien T1 bis Tn im digitalen bzw. virtuellen 3D Raum ändert. Genauer wird die Strukturmarkierung um einen Drehwinkel W1 bzw. 203 und um eine Drehachse D gedreht, die sich aus der Schnittlinie der Transversalebene Tx mit der Frontalebene Fn ergibt, d.h. bspw. der Transversalebene T3 und der Frontalebene F5. Die Drehung findet ihr Ende, sobald die T-Linie vom Drehpunkt 602 aus durch den vorderen Ankerpunkt 603 verläuft, hier durch den Gaumen-Punkt GP. Alternativ kann man auch drehen bis die Transversallinien parallel sind zur Verbindungslinie zwischen hinterem Ankerpunkt 602 und vorderem Ankerpunkt 603. Das führt zum gleichen Zwischenergebnis. Nun stimmt die Orientierung der Strukturmarkierung 500, aber die Skalierung passt noch nicht, weil die vertikale Schnittlinie, hier der Ebene bzw. Linie F1 noch nicht durch den oberen Ankerpunkt 601 verläuft.

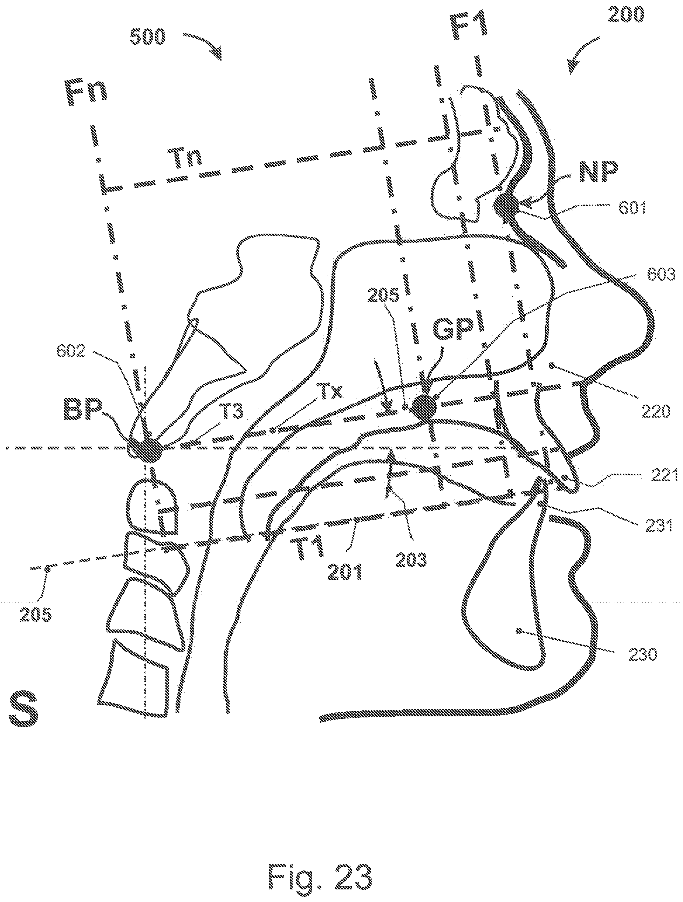

Fig. 23 zeigt die Strukturmarkierung 500 bei einer geeigneten Skalierung, um mit der Linie F1 den anatomischen Ankerpunkt bzw. die Landmarke im individuellen Schnittbild zu erreichen. Die Skalierung kann insbesondere nach der Richtungsorientierung stattfinden, alternativ kann man mehrere Skalierungsschritte verwenden und mit Translationen und oder Rotationen kombinieren. Um die Größe der Strukturmarkierung 500 zu skalieren, vergrößert oder verkleinert man im besonders bevorzugten Ausführungsbeispiel die Strukturmarkierung 500 mit dem hinteren Ankerpunkt 602 als Fixpunkt, bei gleich bleibender Position und Orientierung der Linie T1, bis die dazu senkrecht nach oben führende Linie F1 durch den oberen Ankerpunkt 601 führt, im Ausführungsbeispiel ist dies der Nasen-Punkt NP.

Die Skalierung betrifft die drei Raumrichtungen vorzugsweise gleichermaßen, skaliert also isotrop, vergrößert die Strukturmarkierung also nicht nur in der Sagittalebene, sondern auch in ihrer transversalen Ausdehnung. Ein Neigungswinkel 203 liegt zwischen der kranialen Anterior-Posterior-Achse 205 und der Bildhorizontalen. Der Neigungswinkel 203 dient insbesondere zur Einstellung der Lage der Anterior-Posterior-Achse 205. Eine Achse 205a liegt zur Anterior-Posterior-Achse 205 parallel.

Diese Operationen führen zu einem eindeutigen und individuellen Ergebnis. Die Koordinatenposition, Raumorientierung und Skalierung der 3D Strukturmarkierung 500 wird dadurch festgelegt. Die oben beschriebenen Schritte können in der Reihenfolge variiert und ergänzt werden. Die gleichen Schritte sind auch mit der besonders bevorzugten Ausführung aus Fig. 16 oder anders gestalteten 3D Strukturmarkierungen 500 durchzuführen, solange sie im Sagittalschnitt die Kreuzungspunkte T3 mit F5 und die Linien T1 und F1 aufweisen. Die Linie T1 bzw. die Transversalebene T1 kann als Bezugsebene verwendet werden, siehe bspw. Bezugsebene T1a in der Figur 32.

Die besondere Bedeutung dieser Positionierung und Raumorientierung der Strukturmarkierung 500 (in physikalisch sechs Freiheitsgraden der Bewegung) relativ zum individuellen Schädel eines Patienten liegt darin, dass nun anhand der Strukturmarkierung 500 und ihren Kreuzungspunkten der Linien, die im Schnittbild erscheinen, die Idealpositionen wichtiger biomechanischer, anatomischer und dentalmedizinischer Punkte definiert werden können. Einer dieser wichtigen Punkte ist der sogenannte Inzisalpunkt 604 (IP), d.h. der Berührungspunkt zwischen den beiden unteren mittleren Schneidezähne (mesialer Approximalkontakt). Der Inzisalpunkt 604 (IP) ist der vordere Ankerpunkt, durch den die Idealisierte Kauebene verläuft. Die Schnittlinie der idealisierten Kauebene mit der Sagittalebene ist als Linie 205 gekennzeichnet.

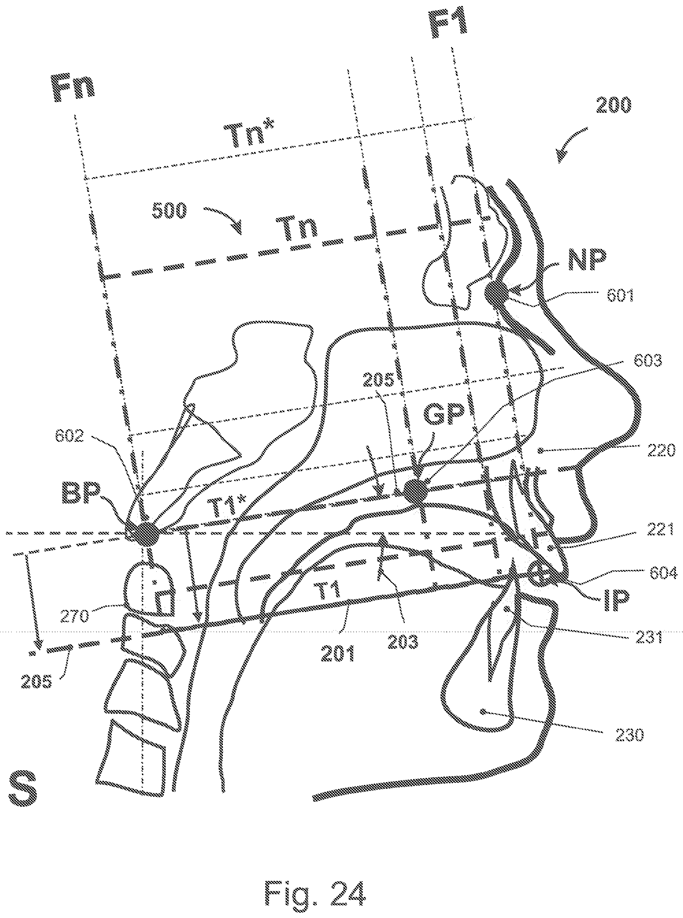

Selbstverständlich ist die Zahnstellung nicht immer so ideal wie in Figur 23 gezeigt, der Mensch kann z.B. auch ein fliehendes Kinn haben wie in Fig. 24 gezeigt und damit zu weit zurück liegende Zähne 231 im Unterkiefer 230 und oder auch ungünstig stehende Zähne 221 im Oberkiefer 220. Zu beachten ist, dass infolge der an kranialen Bezugspunkte 601, 602, 603, die nicht im Bereich des Kiefers liegen, die mittels Strukturmarkierung 500 bestimmten Orientierungspunkte unabhängig sind von einer Fehlstellung der Zähne oder einer Fehlstellung des ganzen Kieferbereiches. Die weitgehende Unabhängigkeit der Bestimmung der idealen Positionen von der Zahnposition hat den besonderen Vorteil, dass auch bei stark lückenhaftem Gebiss, bei verletztem oder deformiertem Kiefer oder auch bei völlig zahnlosem Kiefer die Idealposition z.B. des Inzisalpunktes 604 (IP) mittels Strukturmarkierung erstmals genau definiert werden kann, so dass eine digitale Rekonstruktion und Planung eines biomechanisch, anatomisch und neurophysiologisch idealen Kiefers und Gebisses erstmals möglich wird. Natürlich kann sich der Planer oder Behandler, der ein Gebiss oder einen Zahnersatz oder eine Kieferorthopädie digital mit Hilfe der Strukturmarkierung 500 plant, am kosmetischen Gesamtbild orientieren. Entscheidender Vorteil der erstmals mit Hilfe der Strukturmarkierung 500 erreicht werden kann, ist dabei, dass nun die Idealposition vorab digital bestimmt werden kann und dass die digitale Erzeugung der Vorrichtungen wie z.B. Kronen, Implantate oder Spangen und Retainer (Zahn-Stabilisator) den idealen Zustand zumindest anstreben kann. Die Bedeutung des möglichst idealen Zustandes ergibt sich vor allem daraus, weil dann die Belastungen im Kiefergelenk optimal verteilt sind und weil dann die neuromuskuläre Kopplung zu einer aktiv stabilen Balance führt, die physiologisch sehr vorteilhaft ist.

Fig. 24 zeigt eine alternative Möglichkeit, das gleiche Ergebnis wie in Fig. 20 zu erreichen, allerdings bei einem Fall mit fliehendem Kinn, bei dem die Zähne 231 des Unterkiefers 230 deutlich weiter hinten stehen als ideal. Für die Positionierung (Translation) und Orientierung (Rotation) und die Skalierung (Größeneinstellung) der Strukturmarkierung 500 gibt es mehrere alternative Wege, die zum gleichen Ergebnis kommen.

Man kann z.B. auch zunächst den Kreuzungspunkt der Linie (Ebene) T1 und der Linie (Ebene) Fn auf den hinteren Ankerpunkt 602 legen und dann drehen, bis die Linie T1 durch den Ankerpunkt 603 verläuft. Danach muss man allerdings die 3D Strukturmarkierung 500 in Richtung der F-Linien nach unten verschieben, bis die Transversallinie bzw. die Transversalebene T3 durch den hinteren Ankerpunkt 602 und den vorderen Ankerpunkt 603 verläuft, siehe die nach unten zeigenden Pfeile in der Fig. 24. Dann kann die Skalierung bspw. wie bei Fig. 23 beschrieben folgen. Dieses alternative Verfahren ist weniger direkt und etwas umständlicher, aber ebenso zielführend.

Die Positionierung, Orientierung und Skalierung der Strukturmarkierung kann zum jeweils gleichen Ergebnis führen, wenn man die folgenden Bedingung ansetzt: Die Linie der Frontalebene Fn im medianen Sagittalschnitt soll durch den Basispunkt 602 (BP) verlaufen, im besonders bevorzugten Ausführungsbeispiel mit einer Strukturmarkierung ähnlich zu Fig. 16 und 17 und 20 gilt Fn=F5. Die Linie der Transversalebene T3 im Sagittalschnitt soll durch Basispunkt 602 (BP) und durch Gaumenpunkt GP (603) verlaufen. Und zudem soll die Linie der Frontalebene F1 durch den Nasenpunkt 601 (NP) verlaufen, was eine definierte individuelle Größenskalierung mit sich bringt.

Auch hier kann somit eindeutig der ideale Inzisalpunkt 605 (IP) definiert werden. Allerdings liegt er im Schnittbild nicht genau zwischen den oberen und unteren Schneidezähnen sondern weiter vorne. Grund ist der zu kleine Unterkiefer, der sich im fliehenden Kinn zeigt. Die Strukturmarkierung ist in der Zielposition bei Figur 20 und Fig. 23 und Fig. 24 gleich, weil der kraniale Bereich gleich ist. Damit liegt der Inzisalpunkt in Bezug auf die oberen kranialen Knochenstrukturen BP, GP und NP gleich. Die Definition des Inzisalpunktes 604 (IP) gelingt selbst dann, wenn der Unterkiefer wie z.B. nach einer Trümmerfraktur nicht mehr in der Form definiert ist oder wenn er infolge eines Karzinombefalls entnommen werden musste. Wenn zugleich der Oberkiefer in seiner Zahnkurve beschädigt oder deformiert ist, kann die Anwendung der erfindungsgemäßen Strukturmarkierung 500 erstmalig die Möglichkeit bieten, anatomisch, medizinisch, physiologisch und biomechanisch korrekte Rekonstruktionen digital herzustellen, um sie dann durch Vorrichtungen und Implantate zu realisieren.

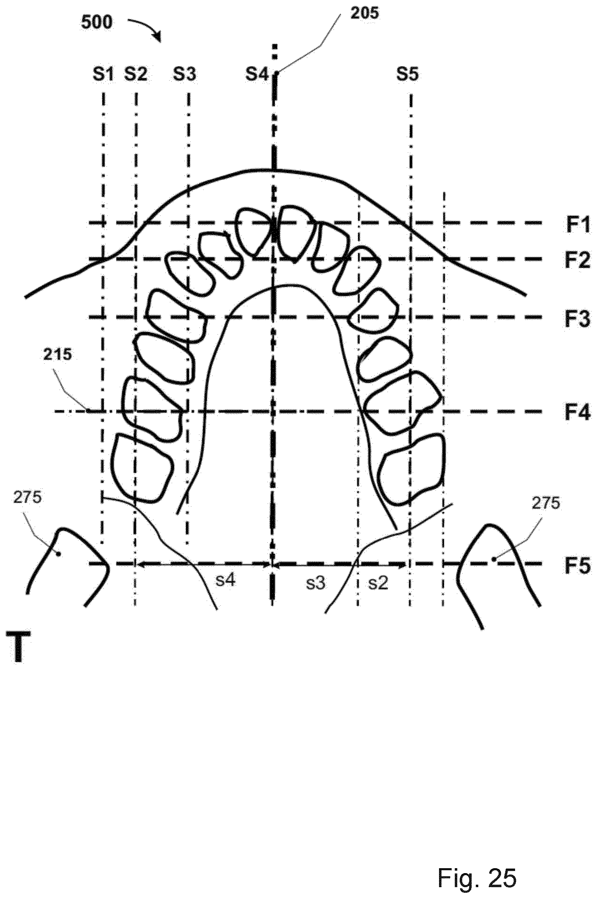

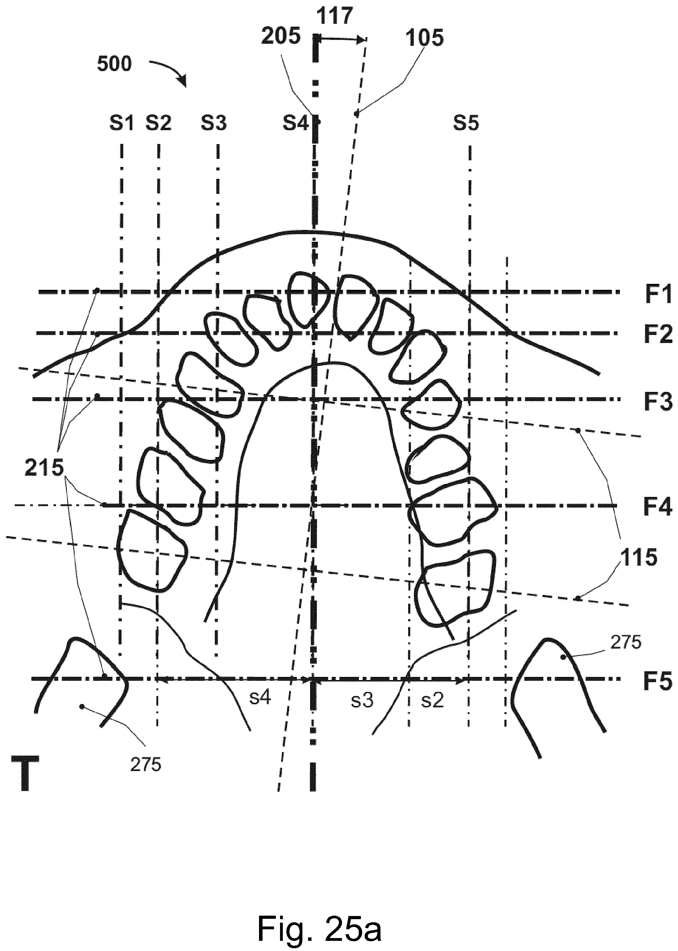

Fig. 25 zeigt einen Transversalschnitt T durch den Zahnbogen des Oberkiefers OK bzw. durch den Bereich des Zahnbogens mit der Sagittalebene, mit Strukturmarkierung 500. Die Figur 25 zeigt einen seltenen fast symmetrischen Zustand. Die meisten behandlungsbedürftigen Patienten hingegen zeigen deutliche Abweichungen von dieser Symmetrie, so dass die craniale Sagittalebene S4 bzw. 205 meist nicht durch die Mitte zwischen den Schneidezähnen verläuft sondern die Schneidezähne aus der sagittalen Mitte heraus versetzt stehen, wie in Fig. 30 gezeigt. Es ergibt sich ein transversales Schnittbild durch den Bereich des Zahnbogens im Oberkiefer OK in der Nähe der Okklusionsebene. Der Zahnbogen ist im vorliegenden Fall vollständig mit je 7 Zähnen je Seite besetzt, die Weisheitszähne wurden früher extrahiert. Im Schnittbild sieht man unten rechts und links die Schnitte durch den gelenknahen Bereich 275 und kann darin die angenäherte Symmetrie des Unterkiefers erkennen. Im Bild vertikal verlaufen die Schnittebenen der Sagittalebenen, die hier mit S1 bis S5 bezeichnet sind. Im Bild horizontal verlaufen die Frontalebenen F1 bis F5. Die Frontalebene F5 bzw. im Schnittbild die Frontallinie F5 verläuft meistens durch den gelenknahen Bereich 275. Die Linie F4 verläuft transversal meist in der Nähe des Backenzahnes mit der Position 6, aber nicht unbedingt genau auf dieser Position. Die Symmetrie ist im vorliegenden Beispiel recht gut, zeigt aber Abweichungen. Die Linie F3 verläuft transversal angenähert durch die Zahnposition 4, also hinter den Eckzähnen. Weitere Details zur Struktur der sagittalen Ebenenpakete siehe Fig. 16 und 19.

Fig. 25a zeigt die Verkippung um den Winkel 117 benannt, projiziert in die Transversalebene T. Die Kauebene steht in ihrer Symmetrielinie 105 schräg im Koordinatensystem der Trainingsvorrichtung, hier übertrieben deutlich schematisch gezeichnet. Das in Fig 25a senkrecht stehende Koordinatensystem mit den Hauptrichtungen 205 und 215 richtet sich nach der Ausrichtung der Transversalachse 215, die durch individuelles anlegen im 3D Bildraum an anatomische symmetrische Merkmale im Kopf des Patienten erreicht wird. Der Winkel 117 zwischen Richtung 205 und Richtung 105 ist der gleiche wie der zwischen Transversalrichtung 215 und 115.

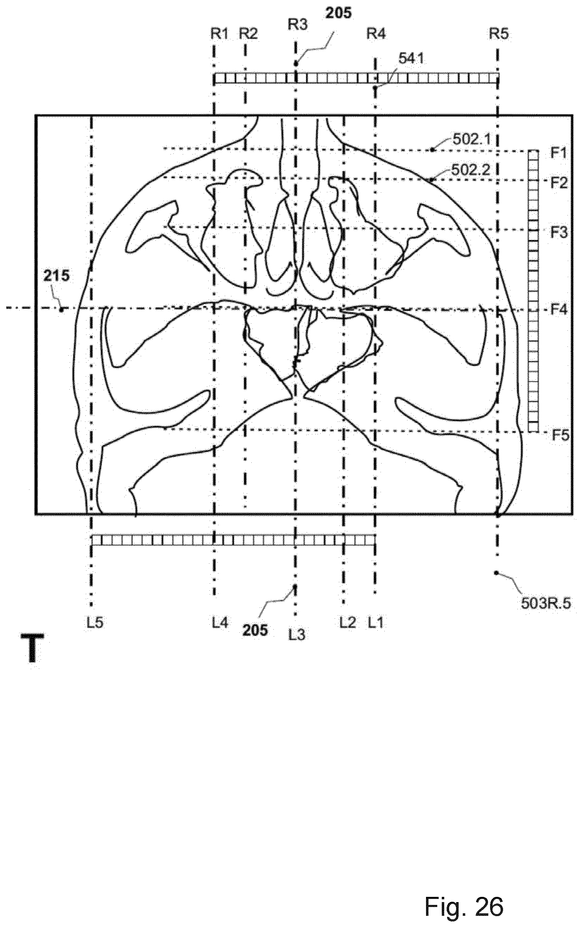

Fig. 26 zeigt einen Transversalschnitt durch den Bereich der Nasenscheidewand mit Asymmetrie. Fig. 26 zeigt die sichtbaren Konturen des Schädels schematisch als Linien. Die linealartigen Streifen oberhalb unterhalb und rechts im Bild zeigen die Harmonie der Ebenenabstände des 3D Referenzkörpers 500. Im Detail sieht man auch trotz scheinbarer Symmetrie die Asymmetrie des Craniums. Die Symmetrie des Zahnbogens im OK und im UK kann in ihrer Mittelebene deutlich von der Symmetrie des Craniums abweichen, d.h. die Kauebene kann verkippt und verdreht sein. Gerade in behandlungsbedürftigen Fällen ist das häufig der Fall. Es ergibt sich ein transversales Schnittbild im digitalen bzw. virtuellen 3D Bildsystem durch das digitale bzw. virtuelle 3D Röntgenbild eines Kopfes mit einem besonders bevorzugten Ausführungsbeispiel der 3D Strukturmarkierung 500 im Bereich des seitlichen Jochbeinbogens. Das Bild zeigt ganz deutlich den sehr unsymmetrischen Bau der knöchernen Strukturen im Bereich der Nasenscheidewand und des Rachens. Die genaue Ausrichtung der transversalen Hauptachse 215 kann im 3D Bildsystem auf mehreren dieser transversalen Schnittebenen anhand mehrerer Rechts-Links-Paare von Landmarken erfolgen. Im Schnittbild eingezeichnet sieht man die Linienpakete R1 bis R5 und L1 bis L5 der sagittalen Ebenenpakete und das Linienpaket F1 bis F5 der frontalen Ebenen der Strukturmarkierung 500. Die Linien 520.1 und 502.2 etc. verlaufen alle in Richtung der transversalen Rechts-Links-Achse 215. Die Linien R und L der Sagittalebenen verlaufen alle in Richtung der Anterior-Posterior-Achse 205.

Fig. 27 zeigt einen Transversalschnitt durch Landmarken / Merkmale des Innenohrs mit der Innenohrachse 285. Fig. 27 zeigt in einem Ausführungsbeispiel die Lage des Referenzkörpers 500 anhand der sichtbaren Achse 215 und der Schnittachse der Sagittalebene 205 und im Vergleich die Innenohrachse 285 IOA nach Hornung, die nicht parallel zur Transversalachse 215 ist. Nur in extrem symmetrisch gebauten Sonderfällen sind die Transversalachse 115 und die Innenohrachse 285 parallel. Es ergibt sich ein transversales Schnittbild im virtuellen 3D Bildsystem durch das virtuelle 3D Röntgenbild eines Kopfes mit einem besonders bevorzugten Ausführungsbeispiel der 3D Strukturmarkierung im Bereich des seitlichen Jochbeinbogens und des Innenohrs, speziell mit Schnitt durch den vertikal stehenden Bogen des Gleichgewichtsorgans 280. Das in der Fig. 27 dargestellte Bild zeigt ganz deutlich die Verbindungslinie 285 (IOA) als Innenohrachse zwischen der Landmarke 280R im Innenohr rechts und der Landmarke 280L im Innenohr links. Im gleichen Bild sieht man die Orientierung der transversalen Achse 215 mit ihren tangentialen Landmarken 215L und 215R. In Bezug zur Hauptachse 215 des vorderen Gesichtsschädels steht die Innenohrachse 285 schräg geneigt. Der Winkel zwischen den beiden Achsen beträgt ca. 2,0 Grad. Figur 15 verdeutlicht damit die hohe Präzision, die mit dem 3D Strukturmarkierung 500 erstmals erreicht werden kann. Denn bisher hatte man angenommen, dass die Innenohrachse 285 und die transversalen Achsen des vorderen Gesichtsschädels parallel sind. In der direkten Folge des Winkels zwischen IOA 285 und Transversalachse 215 entsteht ein Unterschied in der Mitte der Sollposition der Schneidezähne, je nachdem, welche Achse man als transversale Achse heranzieht. Das hier erläuterte Verfahren benutzt nicht die Innenohrachse als transversale Achse sondern die wie oben gezeigt ermittelte Transversalachse 215, die am vorderen Gesichtsschädel ausrichtet werden kann, wie mit den Punkten 215L und 215R gekennzeichnet. Damit kann die Position der optimalen Mitte zwischen den Schneidezähnen in der Frontalansicht für die Zahnbögen auf etwa 0,5 mm bis 1 mm genau bestimmt werden. Diese Mitte 211 wird definiert durch die mediane Sagittalebene, die im gezeigten Ausführungsbeispiel identisch ist mit den Ebenen R3 und L3. Anzumerken ist hierbei, dass sich im Fortschritt einer wirksamen Therapie der knöcherne Schädel und der Kiefer so umbauen können, dass die beiden Achsrichtungen 215 und 285 mehr und mehr parallel werden. Das Ziel einer Therapie kann also anatomisch vergleichbar mit der Orientierung sein, die sich aus der Innenohrachse ergibt. Die Startposition der geeigneten Therapie kann sich jedoch wie hier vorgeschlagen aus der Richtung der Hauptachsen 215 und 205 und aus der Lage der hier bestimmten medianen Sagittalebene des vorderen Gesichtsschädels ergeben.

Fig. 28 zeigt einen Frontalschnitt durch das Kiefergelenk mit sichtbarer Verkippung des Unterkiefers UK. Fig. 28 zeigt den Schrägstand des Unterkiefers 230 in dünn gestrichelten Linien im Vergleich zur cranialen Symmetrie des Oberkiefers 220, die in den dick gestrichelten Linien schematisch dargestellt ist. Es ergibt sich ein frontales Schnittbild durch Oberkiefer OK und Unterkiefer UK eines Patienten. Der Kopf des Menschen umfasst das Kranium 200 mit knöchern fest angebundenem Oberkiefer OK, 220 und gelenkig im Kiefergelenk angekoppeltem Unterkiefer UK, 230. Das virtuelle Schnittbild verläuft durch das Kiefergelenk und zeigt oben rechts und links die digital bzw. virtuell geschnittenen Knochenstrukturen des Oberkiefers OK und darunter länglich stehend die digital bzw. virtuell geschnittenen Knochenstrukturen des Unterkiefers UK. Oben mit fetten Gitterlinien gezeichnet sieht man die Ebenenpakete der Strukturmarkierung 500.A des Kraniums 200. Der Strukturmarkierung 500.A kann das optionale Koordinatensystem KS2 zugeordnet werden. Die mediane Sagittalebene liegt in ihrer Ausrichtung genau in der Richtung der vertikalen Achse 250 des Kraniums, die transversale Achse 215 liegt genau in Richtung der Transversalebenen.

Darunter in feinen Linien gezeichnet liegt verkippt und leicht verschoben eine zweite Strukturmarkierung 500.B, die in den Unterkiefer UK eingepasst ist. Die Strukturmarkierung 500.B kann auch als drittes digitales Bezugssystem bezeichnet werden. Richtet man die zweite untere Strukturmarkierung 500.B nach den Kondylen (knöcherner Teil eines Gelenks) des Unterkiefers UK im Kiefergelenk rechts und links aus, ergibt sich ein Kippwinkel W2 zwischen der Strukturmarkierung 500.B des Unterkiefers UK und der Strukturmarkierung 500. A des Kraniums 200. Die 3D Strukturmarkierungen 500.A und 500.B werden in diesem Ausführungsbeispiel dazu verwendet, Winkelabweichungen zwischen Oberkiefer OK und Unterkiefer UK relativ zur Idealposition präzise und deutlich sichtbar messbar zu machen. Der Strukturmarkierung 500.B kann ein optionales Koordinatensystem KS3 zugeordnet werden.

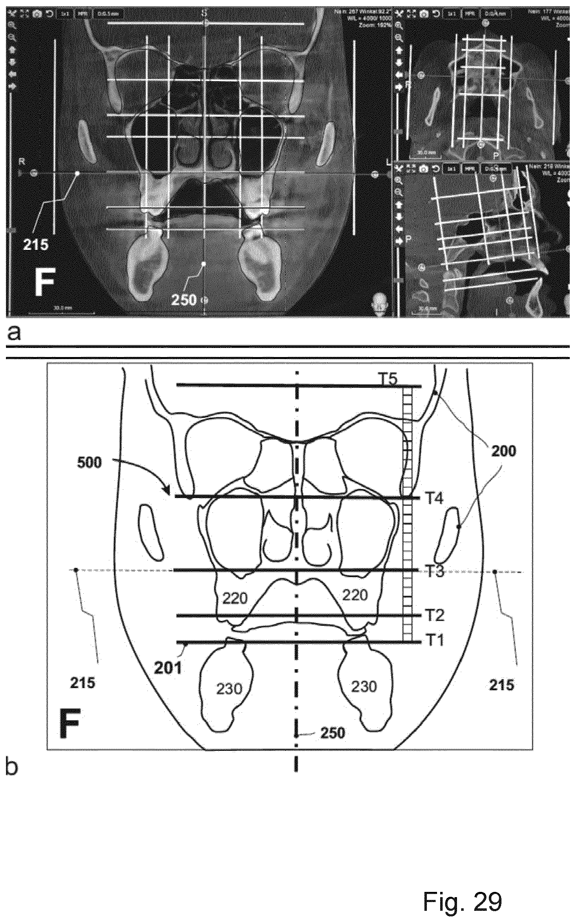

Fig. 29 zeigt einen Frontalschnitt F durch den Backenzahnbereich bei zu hoch liegender Kauebene. Fig. 29 zeigt in der Ansicht von vorn einen 3D Röntgen Frontalschnitt durch den Kopf eines Patienten und darin individuell eingepasst das 3D Referenzobjekt 500 mit seinen Transversalebenen, die im Schnitt als geraden T1, T2, ..., T5 sichtbar sind. Die untere Transversalebene T1 bildet die individuelle Trenngleitebene, die in der 3D Konstruktion der Trainingsvorrichtung berücksichtigt wird. Die Trainingsvorrichtung wird mit OK-Teil und UK-Teil so in 3D erstellt, dass sie die geeignet gelagerte Trenngleitebene enthält. Zudem werden die Randspalte um die Zähne und die okklusalen Auflagebereiche konstruktiv ausgeführt. Anschließend wird die ganz individuell an den Zahnbogen und zudem an die Craniale Symmetrie in der Formgebung angepasste Trainingsvorrichtung gefertigt, z.B. durch Fräsen oder durch additive Fertigung. Oben in Fig. 29a ist die röntgenologische Schnittdarstellung in den drei Hauptebenen F, T und S gezeigt. Das große Bildfenster links zeigt den Frontalschnitt F, das kleine Fenster oben rechts zeigt den Transversalschnitt T und das kleine Bildfenster rechts unten im oberen Bereich zeigt den Sagittalschnitt S.

Unten zeigt Fig. 29b das frontale Schnittbild F schematisch als knöcherne Konturen. Man sieht die knöchernen Strukturen 200a und daran ausgerichtet die Lage der transversalen Linien nicht direkt, weil die Ankerpunkte und Tangentialpunkte hierfür nicht in der Schnittebene liegen. Die im Bild horizontal verlaufenden T-Linien T1 bis T5 entstehen durch den Schnitt der frontalen Bildebene mit den Transversalebenen 501.1 bis 501.5. Hier ist zur Klarheit das vertikale Liniengitter der Sagittalebenen S ausgeblendet. Rechts im Bild befindet sich ein eingeblendeter Maßstab aus kleinen Quadraten zur Verdeutlichung der Abstandsverhältnisse zwischen den Transversalebenen T1 bis T5. T2 ist von T1 3 Kästchen entfernt. T3 ist von T2 5 Kästchen entfernt. T4 ist von T3 3+5=8 Kästchen entfernt. T5 ist von T4 5+8=13 Kästchen entfernt. Das ist die bekannte Fibonacci-Folge, die angenähert proportionale Abstandsverhältnisse aufweist. Auffallend deutlich sieht man zwischen den Transversalebene T1 und T2 den schräg stehenden Unterkiefer 230 und die einseitige Kompression, im Bild auf der rechten Bildseite. Das Bild zeigt auch die Ausrichtung der 3D Strukturmarkierung 500 an den knöchernen Strukturen im oberen frontalen Kranium, also im Bereich der Linien/ Ebenen T4, T5 und darüber. Dort zeigen die äußeren Strukturen der Knochen eine Symmetrie, während die inneren Strukturen der Nasenscheidenwand und deren Umgebung deutlich asymmetrisch und für eine Ausrichtung der Strukturmarkierung unbrauchbar bzw. nicht bevorzugt sind. Die Hauptrichtung 215 in transversaler Rechts-Links-Richtung zeigt sich an jeder der parallelen Transversalebenen und deren Schnittlinien T1 bis T5.

Fig. 30 zeigt einen Frontalschnitt F durch die unteren Schneidezähne. Oben in Fig. 30a ist die röntgenologische Schnittdarstellung eines Frontalschnittbildes F durch die unteren Schneidezähne zu sehen. Man sieht die horizontalen T-Linien als weiße Striche und die vertikalen S-Linien, die symmetrisch um die mediane Sagittalebene angeordnet sind, ebenfalls als weiße Striche. Horizontal ist die Richtung der Transversalachse 215 eingezeichnet.

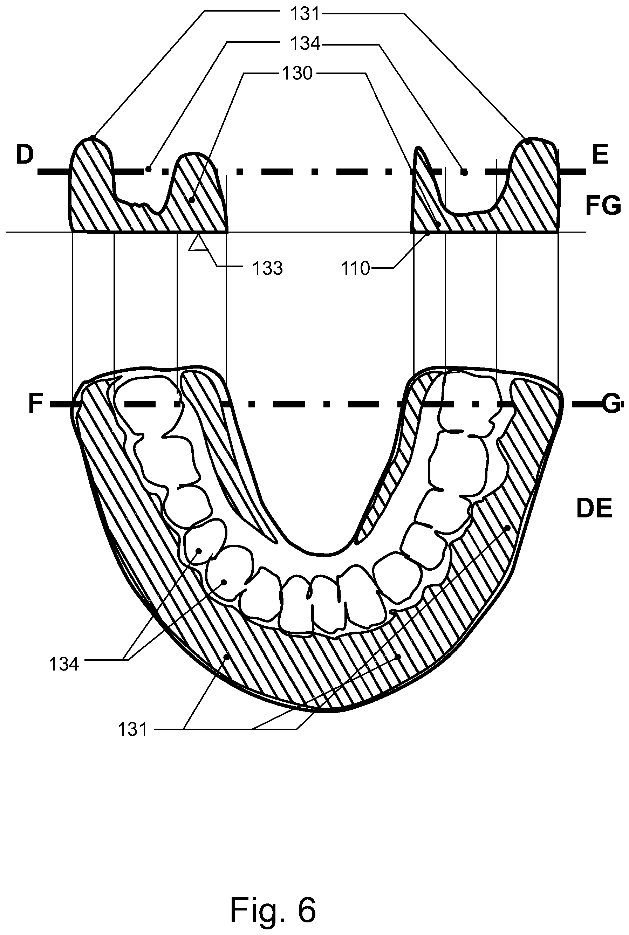

Unten in Fig. 30b sieht man die Konturen des Frontalschnitts F in schematischer Darstellung. Hier sind die T-Linien der transversalen Ebenen T1 bis T5 eingezeichnet und ebenso die vertikalen Schnittlinien der Sagittalebenen R1 bis R5 und L1 bis L5. Weitere Details zum sagittalen Ebenenpaket siehe Figur 3b, 4 und 6. Die Maßstäbe aus quadratischen Kästchen zeigen die Abstandsverhältnisse zwischen den Ebenen, die im vorliegenden bevorzugten Ausführungsbeispiel in beiden Richtungen (horizontal bzw. rechts-links versus oben-unten bzw. vertikal) gleich groß sind. Abweichend hiervon gibt es ein alternatives Ausführungsbeispiel speziell für langgestreckte Kopfformen, in dem die Kästchen in vertikaler Richtung höher sind als die Breite in transversaler Richtung. Die Isotropie der Abstände der Ebenenpakete 501 (T), 502 (F) und 503 (S bzw. R und L) ist keine Notwendigkeit, aber ein sehr bevorzugtes Ausführungsbeispiel.

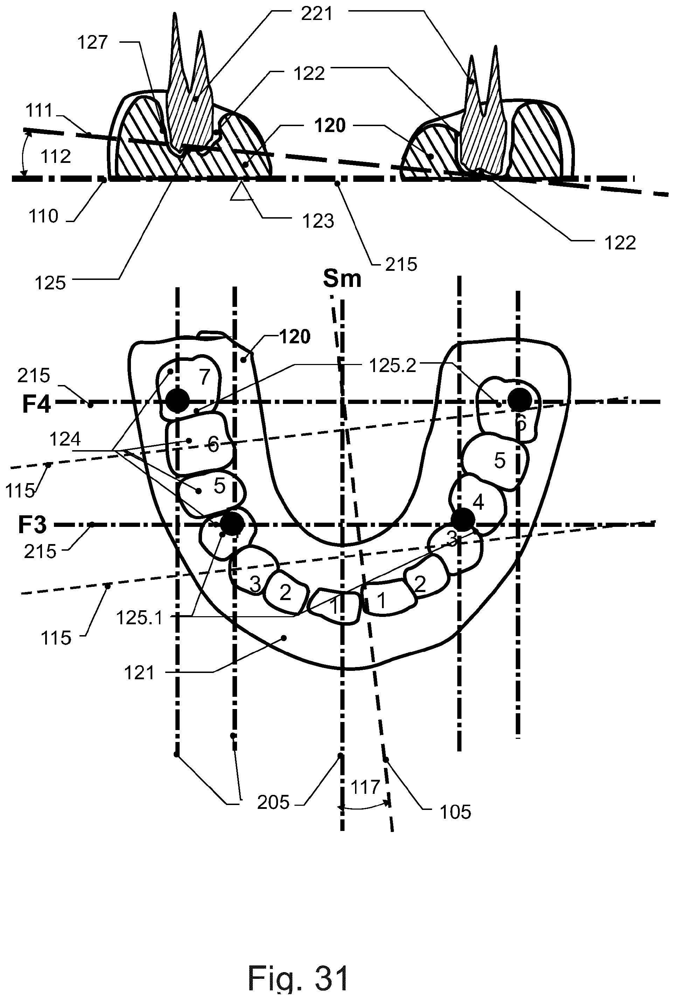

Fig. 31 zeigt oben den Frontalschnitt durch das Oberkieferteil 120 und unten die Aufsicht auf das Oberkieferteil 120, bei der man die Freiräume 124 für die Zähne sieht und die Kontaktstellen 125. In diesem individuell schematisierten Fall haben wir keinen Durchbruch von unten. Die hinten vorne Hauptrichtung 205 des Kranialen Systems bestimmt die hinten vorne Hauptrichtung des OK Teils 120. Die transversale rechts links Hauptrichtung 215 bestimmt die Lage der Trenngleitebene 110. Durch die fast reibungsfrei gleitende glatte Oberfläche 123 wird erreicht dass die resultierende Kraft nur senkrecht auf Trenngleitebene 110 stehen kann, vor allem solange die Zähne seitlich wegen des Randspaltes 122 nicht anstehen. In der Ansicht von oben sieht man dass die genaue Lage der Auflagepunkte 125 sich nach dem gekreuzten Ebenen System mit den Projektionslinien 215 und 205 richtet, insbesondere sind die Frontalebenen F3 und F4 maßgeblich. Da das dentale Symmetriesystem mit den Hauptrichtungen 105 und 115 verkippt ist um drei Raumwinkel, steht der Freiraum 214 für den Zahnbogen im OK teil 120 schräg und die Kauebene 111 ist gegen die Trenngleitebene 110 geneigt.

Figur 31 zeigt schematisch die erste glatte Gleitfläche 123 die möglichst genau mit einer cranial orientierten Transversalebene übereinstimmt, die parallel zu den Achsen 110 und 115 liegt. Die Figur 31 zeigt ebenso die Kauebene 111 im Schnitt von vorn gesehen schräg geneigt zur Richtung der Transversalachse 110. Die Kauebene ist meist auch von der Seite gesehen im Sagittalschnitt schräg gegenüber der ersten glatte Gleitfläche 123 geneigt (in Fig. 31 nicht gezeigt). Betrachtet man die Zähne mit der in der Dentalmedizin üblichen Numerierung 1 bis 7 vom mittleren Schneidezahn ausgehend, so sieht man die deutliche Asymmetrie, die im Beispiel darin begründet ist, dass rechts im Bild der Backenzahn 7 fehlt. Die Auflagepunkte 125 liegen nun nach wie vor an cranialen Symmetrieebenen orientiert, also verläuft die Verbindungslinie zwischen den Auflagepunkten 125.1 rechts 3 und links 3 nicht symmetrisch zum Zahnbogen sondern entsprechend der cranialen Symmetrie, die anders gelagert ist. Analog verläuft auch die hintere Verbindungslinie rechts-links zwischen den Auflagepunkten 125.2 nicht symmetrisch zum Zahnbogen sondern symmetrisch zur cranialen Symmetrie. Die craniale Symmetrie gibt insbesondere die Ansatzpunkte der Muskulatur vor und ist für die neuromuskuläre Funktion mit entscheidend. Betrachtet man die mechanischen Kontaktpunkte und die Kraftübertragung, so sieht man im Schnittbild AB die Randspalte 122 von bevorzugt 0,4mm Breite, die eine direkte Kraftübertragung seitlich auf die Zähne 221 verhindern. Die erfindungsgemäße Vorrichtung bildet wegen der Randspalte 122 mit ihrer teils bogenförmigen Form und den Freiräumen 124 für die Zähne keine stramm sitzende Schiene sondern eine lose sitzende Vorrichtung, die im Ausführungsbeispiel nur auf den Stützbereichen 125.1 und 125.2 aufliegt. Würde man den Randspalt 122 weg lassen und die Schiene 120 direkt an den Zähnen anliegen lassen, wie es andere Schienen tun, so würden die Zähne seitwärts mit Kraft berührt und die resultierenden Kräfte an den Zähnen wären nicht mehr senkrecht zur Trenngleitebene. Da auch die Okklusionsflächen der Zähne 221 eine seitwärts wirkende Verzahnung mit der Schiene 120 darstellen würden, werden sie nicht abgeformt sondern der Randspalt 122 reicht bis zu den Auflagebereichen 125, die bevorzugt keine seitlichen Kräfte sondern nur Druckkräfte übertragen. Neurophysiologisch betrachtet wirkt sich die sehr reibungsarme Transversalgleitfläche 123 dadurch aus, dass die resultierende Kraft nur senkrecht darauf stehen kann, solange kein Anschlag der Gleitflächen des Oberkieferteils und des Unterkieferteils vorliegt. Der Kraftreiz dieser senkrecht stehenden Druckkraft wird über die Haltefasern der Zähne erfasst und an die Sensorik des Kauapparates gemeldet. Gleichzeitig melden auch die Muskelspindeln und die bandspindeln die Belastung und Dehnung der Muskeln, Bänder und sehnen. Dadurch verändert sich die Ansteuerung der Muskulatur durch den sensomotorischen Bereich des Gehirns. Für diese biomechanische und neurophysiologische Trainings-Funktionalität ist es von großer Bedeutung, dass die Trenngleitfläche nicht in der aktuellen Kauebene 111 liegt sondern in der cranialen Transversalebene 110, 115. Durch den Schrägstand der Trenngleitebene 123 im Vergleich zur Kauebene 111 kommt es oftmals zu Durchbrüchen einiger Zähne des Oberkiefers in das Unterkieferteil. In Fig. 11a sind die Durchbrüche 126 schematisch dargestellt, hier allerdings an einem von oben eher symmetrisch wirkenden Zahnbogen.

Fig. 32 zeigt das Detail eines Auflagebereiches 125 im besonders bevorzugten Ausführungsbeispiel. Die Lage des Auflagebereiches richtet sich - wie unter Fig 31 beschrieben - an der cranialen Symmetrie des Schädels des Patienten aus, wie sie z.B. einer DVT Aufnahme des Kopfes zu entnehmen ist. Der Auflagebereich ist möglichst wenig seitwärts verschlüsselt. Zudem besteht ein Randspalt 122. Der Auflagebereich muss nicht notwendigerweise kreisförmig sein und er ist so gestaltet, dass möglichst keine seitlichen Kräfte ausgeübt werden. Eine bevorzugte Ausführung stützt auf einem Höcker in einem kleinen Bereich ab und umliegend beginnt der Randspalt. Damit überträgt die Auflage Kräfte die senkrecht zur Trenngleitebene stehen, sie klemmt aber die Zähne seitlich nicht ein, ganz anders als eine normale Aligner-Schiene, die z.B. zum Verlagern der Zähne verwendet wird. Die Druckkraft durch den Kaudruck wird nur punktuell im eher sehr kleinen Bereich 125 übertragen. Bevorzugt hat der Auflagebereich einen Durchmesser von weniger als 4 mm, besonders bevorzugt weniger als 2,5 mm und der Randspalt ist bevorzugt kleiner als 1mm, besonders bevorzugt kleiner als 0,5mm.

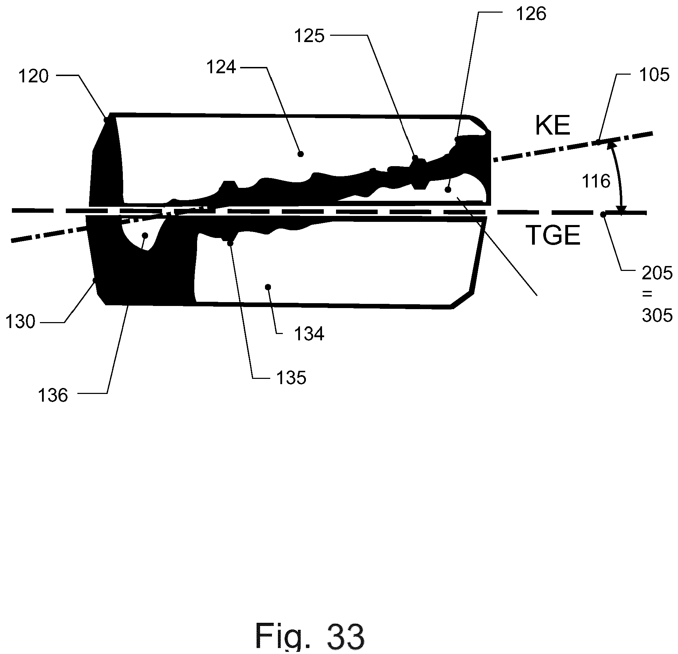

Fig. 33 zeigt die Trainingsvorrichtung von der Seite am Gebiß mit starker Schräglage. Es ist von der Seite gezeigt, dass die Trenn-Gleit-Ebene 110 genau nicht in der Kauebene 111 liegt. Die Trenn-Gleit-Ebene 110 ist im Vergleich zur Kauebene 111 hinten (rechts im Bild) an den Backenzähnen tiefer liegend. Die Backenzähne des Unterkiefers bohren sich also in den Bereich des OK Teiles hinein. Es ist daher eine Korrektur der Trainingsvorrichtung erforderlich. Die Korrektur ist eine Parallelverschiebung der Transversalebene = Trenn-Gleit-Ebene 110 nach oben oder unten, parallel zur Ebene T1, wie auch in den Figuren 16-18 gezeigt, um die Wandstärken an den verbleibenden Resten der ausgefrästen Preforms auf einem machbaren Mindestwert zu bringen bzw. damit der Durchbruch der Zähne der Gegenseite nicht allzu groß wird oder damit die Wandstärken am Trainingsgerät geeignet dick werden. Die Gleitrichtung von vorne und von der Seite gesehen ändert sich durch Verlagerung der Transversalebene nach oben oder unten nicht.

Fig. 33 zeigt ferner ein schematisches Schnittbild durch die Vorrichtung 300 mit OK teil 120 und UK Teil 130. Man sieht schwarz das bevorzugt massive Material der Trainingsvorrichtung und die Freiräume 124 und 134 inklusive Randspalte. Die Auflagepunkte 125 und 135 sind angedeutet. Da die Kauebene KE im seitlichen Schnitt mit Richtung 105 um den Winkel 116 geneigt ist gegen die Trenngleitebene TGE mit Richtung 205 bzw. 305, sieht man den massiv gefüllten Freiraum zwischen den Zähnen schräg von links unten nach rechts oben im Bild verlaufen. Im OK Teil 120 findet sich folglich ein Durchbruch 126 von Unten als Freiraum für die Backenzähne des Unterkiefers. Im Unterkieferteil 130 findet sich ein Durchbruch 136 als Freiraum für die Schneidezähne des Oberkiefers. Die Freiräume umfassen eine Randschicht zur zusätzlichen Bewegungsfreiheit der Zähne zur Freigabe der seitlichen Gleitbewegung des Unterkieferteils 130 relativ zum Oberkieferteil 120.

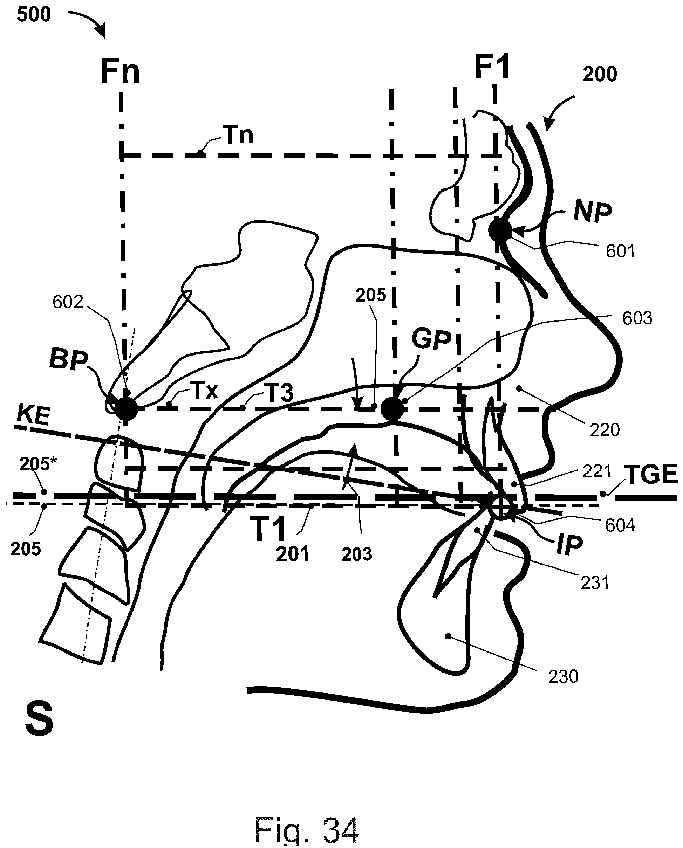

Fig. 34 zeigt die Strukturmarkierung im Sagittalschnitt mit eingezeichneter Trenn-Gleit-Ebene TGE bzw. 110. Gezeigt ist die Konstruktion von der Seite, mit der optionalen Verschiebung der T1 Ebene z.B. um 2 oder 4 mm nach oben, in der Figur entsprechend von der Achse 205 auf 205*, um die Trenn-Gleit-Ebene TGE zu erhalten, die sich auch real bauen lässt. Im schrägen Winkel dazu ist die Kauebene KE bzw. 111 eingezeichnet.

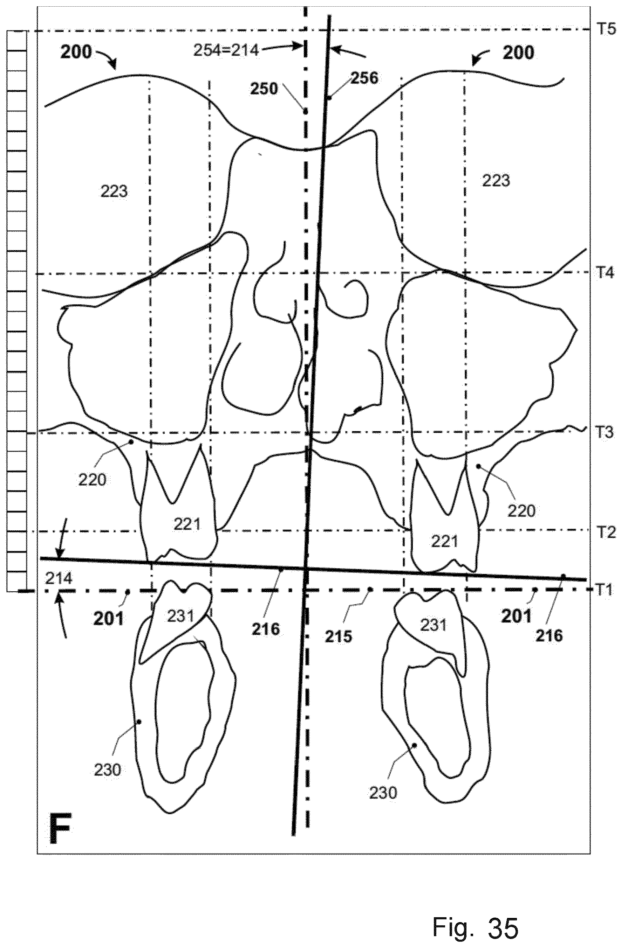

Fig. 35 zeigt einen digitalen bzw. virtuellen Frontalschnitt F durch den Backenzahnbereich und offenbart einen deutlichen Schrägstand der aktuellen Okklusionsebene/ Kauebene 216 in diesem Beispiel. Im hier schematisch gezeigten Frontalschnitt F durch das virtuelle 3D Volumenbild eines Schädels 200 mit Oberkiefer 220 und Unterkiefer 230 und den Zahnbögen 221 oben und 231 unten kann man analysieren, wie weit eine Asymmetrie oder Verkippung des Gebisses und der Kiefer gegeben ist. Gestrichelt fett gezeichnet ist die vertikale Achse 250 des Kraniums 200 und die darauf senkrecht stehende horizontale Achse 215. Zunächst scheint es auf den ersten Blick, als wären die Zähne nach der Transversalachse 215 ausgerichtet. Die genaue Betrachtung zeigt indes, dass die aktuelle Okklusionsebene 216 am Oberkiefer um den Winkel 214 gegenüber der Transversalachse 215 des Kraniums 200 verkippt ist. Entsprechend ist auch die Flächennormale 256 auf die aktuelle Kauebene verkippt, der Winkel 254 ist gleich dem Winkel 214. Augenhöhlen 223 sind ebenfalls knöcherne Strukturen des Kraniums 200. Untere Ränder der Augenhöhlen 223 sind bspw. ebenfalls für die Ausrichtung der Strukturmarkierung 500 geeignet.

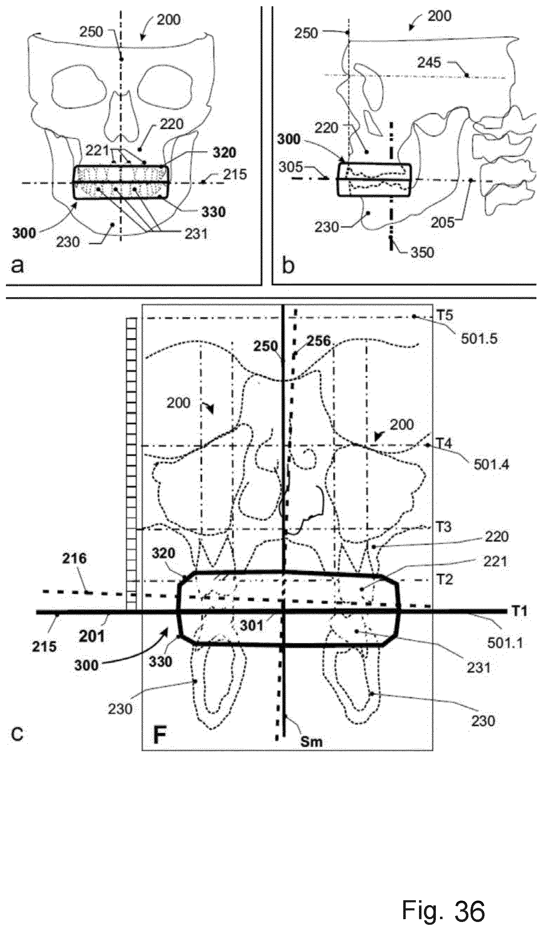

Fig. 36 zeigt eine Trainingsvorrichtung 300 mit Trenn-Gleit-Ebene 301 in der Position der idealen Okklusionsebene 201 in grob schematischer Darstellung. Fig. 32a zeigt schematisch die knöchernen Strukturen 200a des Kraniums 200 mit Oberkiefer 220 und angelagert dem nicht zu Kranium gehörenden Unterkiefer 230. Der Zahnbogen 221 des Oberkiefers und der Zahnbogen 231 des Unterkiefers ist hier von der Vorrichtung 300 verdeckt. Die knöchernen Strukturen 200a des Kraniums insbesondere im Bereich um die Augen, also nicht die im Oberkiefer selbst, können analysiert werden, um die Lage und Richtungsorientierung der Hauptachsen zu definieren. Entscheidend können die Transversalachse 215 und die Vertikalachse 250 sowie die von frontal nicht sichtbare Anterior-Posterior-Achse 205 sein.

Fig. 36b zeigt schematisch die seitliche Ansicht der knöchernen Strukturen eines Kraniums (Schädels) 200 im virtuellen 3D Raum, wobei hier der Kopf so weit nach vorn geneigt ist, dass die Anterior-Posterior-Achse 205 des Kraniums 200 horizontal im Bild verläuft. Die transversale Rechts-Links-Achse 215 läuft in Fig. 32b senkrecht zum Bild, d.h. senkrecht aus der Bildebene hinaus oder senkrecht in die Bildebene hinein. Die idealisierte Okklusionsebene 201 wird durch diese beiden Achsen 205 und 215 definiert und steht senkrecht auf der vertikalen Achse 250 und enthält die Achse 205. Die Trenn-Gleit-Ebene 301 der Vorrichtung 300 ist exakt so positioniert, dass sie mit der Okklusionsebene 201 zusammenfällt. Im Schnittbild oder in der Seitenansicht liegt also die Anterior-Posterior-Achse 305 der Vorrichtung 300 auf der Anterior-Posterior-Achse 205 des Kraniums 200. Die vertikale Achse 350 der Vorrichtung 300 verläuft parallel zur vertikalen Achse 250 des Kraniums 200.

Fig. 36c zeigt die Frontalansicht mit Überlagerung der Konturen der Vorrichtung 300 mit Trenn-Gleit-Ebene 301 über dem Frontalschnitt aus Fig. 36. Die Trenn-Gleit-Ebene 301 liegt möglichst präzise auf der idealisierte Okklusionsebene 201. Diese Abbildung verdeutlicht die Positionierung der biomechanisch und neurophysiologisch wichtigen Lage und Orientierung der Trenn-Gleit-Ebene 301 zwischen dem Oberteil 320 und dem Unterteil 330 der Vorrichtung 300. Diese ergibt sich in der Praxis individuelle aus dem Formschluss der eingesetzten Vorrichtungsteile 320 und 330, wenn diese präzise den Zahnbögen entsprechend gefertigt sind. Bei der Vorrichtung 300 kann es sich zum Beispiel um eine Trainingsvorrichtung oder eine Orthese handeln. Die Vorrichtung 300 mit den mindestens zwei Teilen 320 und 330 kann auch einseitig asymmetrisch und oder mehrteilig gebaut sein.

Als Referenzebene für die Planung und Herstellung der Vorrichtung 300 insbesondere zum Training der Kiefergelenksmuskulatur oder zur Behandlung des Kiefergelenks kann nicht etwa die aktuelle Kauebene mit der Transversalrichtung 216 dienen sondern die Transversalebene T1 des Kraniums mit der Transversalrichtung 215 und der Richtung der Anterior-Posterior-Achse 205. Das ist die idealisierte Okklusionsebene T1a bzw. die Bezugsebene T1a und damit die Ebene, in der die seitliche Gleitbewegung des Unterkiefers 230 mit eingesetzter Vorrichtung 300 relativ zum Oberkiefer 220 möglich ist. In zweiteiligen Vorrichtungen 300 mit Trenn-Gleit-Ebene 301 werden für diesen Zweck bei der Planung im digitalen bzw. virtuellen 3D Raum in einer bevorzugten Vorgehensweise digitale bzw. virtuelle Preforms für die Herstellung der Teile 320 und 330 so orientiert, bzw. die Vorrichtung 300 wird virtuell mit ihrer Trenn-Gleit-Ebene 301 so orientiert, dass diese Trenn-Gleit-Ebene 301 in der Orientierung der idealisierten Okklusionsebene 201 liegt. Im besonders bevorzugten Ausführungsbeispiel ist die Trenn-Gleit-Ebene 301 in ihrer Winkelorientierung und in der vertikalen Position identisch mit der Transversalebene T1 bzw. der Bezugsebene T1a, die anhand der 3D Strukturmarkierung 500 ermittelt wurde.

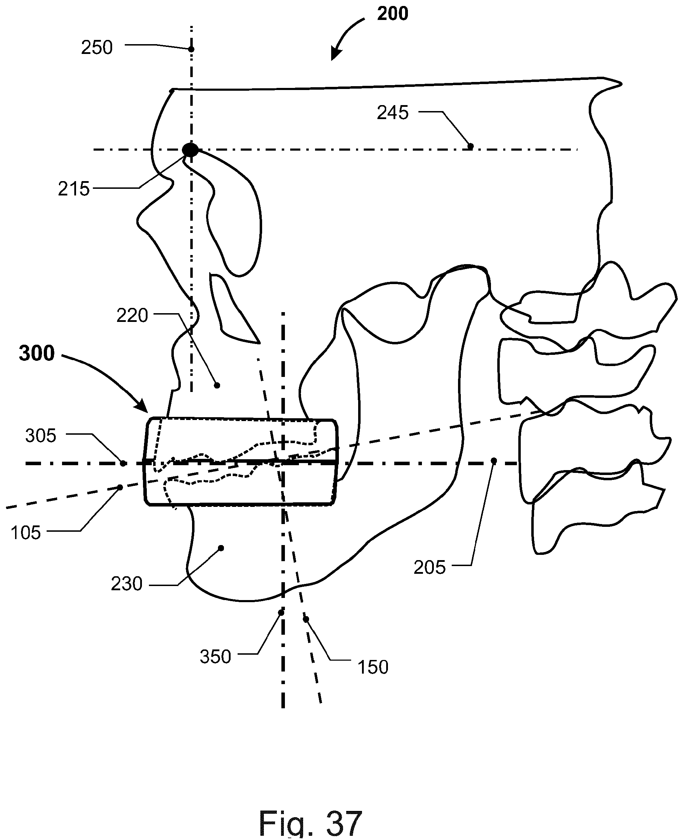

Fig. 37 zeigt den Kopf schematisch von der Seite mit einer Raumausrichtung in der das Koordinatensystem des Schädels senkrecht steht. In dieser Raumorientierung erscheint die vorne hinten Hauptrichtung 105 der Kauebene 110 schräg geneigt gegen die Transversale Hauptrichtung 205 des Schädels, entlang der die Trenngleitebene orientiert ist. Die transversale Hauptrichtung 305 der Vorrichtung 300 ist also durch geeignete Positionierung der Preforms im 3D Bildraum genau in die Orientierung der Hauptrichtung 205 des Schädels gelegt worden, um damit die Trainingsvorrichtung als Überlagerung der Konturen der Preforms und der Konturen der Zahnbögen nebst Luftspalt zu erzeugen. Selbiges gilt in der Ausrichtung in den anderen 2 Raumwinkeln, die man von vorne und von oben sieht, aber nicht von der Seite.

Im Folgenden werden die Strukturmarkierungen 500, die Verfahren und die Verwendung für die Fertigung von realen Vorrichtungen in weiteren Details beschrieben.

Einsatzgebiete sind Analyse, Vermessung, Gestaltung, Planung, Diagnostik, Training und/oder Therapie im Zusammenhang mit beweglichen Körperteilen, insbesondere mit der Position und Bewegung des Unterkiefers UK zum Oberkiefer OK sowie die Definition der Position und Orientierung von Zähnen, Zahnbögen, Implantaten und/oder von biomechanischen und kieferorthopädischen Vorrichtungen, z.B. 300.

Die Beschreibung verwendet anatomische Fachausdrücke, die auch technisch zu verstehen sind:

| | | |
|---|---|---|
| □ | Transversalachse: | zwischen Rechts und Links verlaufend |
| □ | Vertikalachse: | zwischen Oben und Unten verlaufend |
| □ | AP-Achse: | zwischen Vorne (anterior) und Hinten (posterior) verlaufend |
| □ | Frontalebene: | Schnittebene mit Hauptachsen Rechts-Links sowie Oben-Unten |
| □ | Transversalebene: | Schnittebene mit Hauptachsen Rechts-Links und Vorne-Hinten |
| □ | Sagittalebene: | Schnittebene mit Hauptachsen Vorne-Hinten und Oben-Unten |

### Beschreibung der Strukturmarkierung 500

Die 3D Strukturmarkierung 500 umfasst mindestens zwei planparallele Ebenenpakete 501 und 502 die im digitalen bzw. virtuellen 3D Raum 50 aufeinander senkrecht stehen. Fig. 16 zeigt ein bevorzugtes Ausführungsbeispiel mit drei aufeinander senkrechten Ebenenpaketen in perspektivischer Ansicht. Jede Schnittebene zeigt die planparallelen Ebenen als parallele Linien. Durch die senkrecht aufeinander stehenden Ebenenpakete entstehen im Schnittbild gekreuzte Pakete paralleler Linien. In den bevorzugten Ausführungsbeispielen sind die Schnittebenen für die Visualisierung im 3D Bildsystem parallel zu einem der Ebenenpakete 501 oder 502 oder 503 eingestellt. Man sieht dann die Schnittlinien der anderen Ebenenpakete als rechtwinklig gekreuztes Gitter aus parallelen Linien, wobei die Linienpakete ein charakteristisches Abstandsmuster bzw. Abstandsverhältnis aufweisen können.

Jedes Ebenenpaket kann aus mindestens 3 Ebenen (T1, T2, T3 bzw. F1, F2, F3) bestehen. Im bevorzugten Fall der 3D Strukturmarkierung 500 wird ein Ebenenpaket 591 bis 595 aus 5 Ebenen (F1 bis F5) kombiniert mit einem zweiten, darauf orthogonal stehenden Ebenenpaket 581 bis 585 aus 5 Ebenen (T1 bis T5) verwendet, siehe Fig. 17.

In einem weiter entwickelten Ausführungsbeispiel wird die Gruppe aus zwei orthogonal stehenden Ebenenpaketen (F1 bis Fn) und (T1 bis Tn) ergänzt durch mindestens ein senkrecht darauf stehendes Ebenenpaket (S1 bis Sn). Besonders bevorzugt ist das sagittale Ebenenpaket S ein kombiniertes Ebenenpaket aus zwei Teilpaketen (S1R bis SnR) und (S1L bis SnL) die parallel sind und die symmetrisch um die mediane Sagittalebene Sm angeordnet werden, z.B. indem die beiden Ebenen S3L und S3R identisch sind und die mediane Sagittalebene bilden. Weiter alternativ kann auch nur ein Ebenenpaket verwendet werden, bspw. das Ebenenpaket mit den transversalen Ebenen.

Im allgemeinen Ausführungsbeispiel können sich die Abstandsmuster der Ebenenabstände für die Ebenenpakete unterscheiden. Im besonders bevorzugten Ausführungsbeispiel haben alle Ebenenpakete das gleiche Abstandsmuster und die gleichen Abstandsverhältnisse, siehe Fig. 17.

Die Abstände der planparallelen Ebenen innerhalb eines Ebenenpakets können unterschiedlich sein. Im besonders bevorzugten Ausführungsbeispiel verhalten sie sich gemäß einer Fibonacci-Folge wie z.B.

a1=3, a2=5, a3=8, a4=13, a5=21, wobei gilt a3=a1+a2; a4=a2+a3; a5=a3+a4; etc.

Das Abstandsverhältnis kann in einem anderen Ausführungsbeispiel auch eine gleichbleibende Proportionalität sein:
a2/a1 = a3/a2 = a4/a3 = a5/a4 etc.,
wobei als Proportionalitätsfaktor bevorzugt zwischen 1,80 und 1,50 gewählt,
besonders bevorzugt zwischen 1,65 und 1,60.

Im für die kieferorthopädische Anwendung besonders bevorzugten Fall der 3D Strukturmarkierung 500 wird ein Ebenenpaket 501 aus fünf Ebenen mit den Abständen 3, 5, 8, 13 Einheiten kombiniert mit einem zweiten Ebenenpaket 502 aus weiteren fünf dazu orthogonalen Ebenen mit den gleichen Abständen 3, 5, 8, 13 Einheiten. Nochmals senkrecht dazu steht ein drittes Ebenenpaket 503 als doppeltes Ebenenpaket aus zwei Teilpaketen 503R und 503L, die ihrerseits die Abstände 3, 5, 8, 13 aufweisen. Die Teilpakete 503R und 503L können gegenläufig und symmetrisch orientiert sein, entsprechend der grundsätzlichen Symmetrie in der Anatomie des Körpers.

Die 3D Strukturmarkierung 500 hat folgende Möglichkeiten der individualisierten Einstellung:
□ Die Größenskalierung der 3D Strukturmarkierung 500 ist individuell einstellbar, d.h. die Strukturmarkierung kann vergrößert oder verkleinert werden.
□ Die räumliche Winkelorientierung der 3D Strukturmarkierung 500 und ihrer Hauptachsen ist individuell einstellbar, d.h. die 3 Hauptrichtungen der orthogonalen Ebenenpakete können ganz nach Bedarf im digitalen bzw. virtuellen Raum 50 ausgerichtet werden, was den 3 Freiheitsgraden der Rotation entspricht.
□ Die räumliche Position der 3D Strukturmarkierung 500 bzw. ihres Schwerpunktes ist individuell einstellbar, d.h. die 3 Raumkoordinaten (x, y, z) der zueinander feststehenden Gruppe aus den orthogonalen Ebenenpaketen sind einstellbar im Sinne der 3 Freiheitsgrade der Translation.

Für die individuelle Anpassung der 3D Strukturmarkierung 500 an die Gegebenheiten des knöchernen Schädels werden anatomische Landmarken/ Merkmale der Symmetrie und weitere Ankermerkmale, z.B. Ankerpunkte, verwendet, die im virtuellen 3D Bild des Schädels in geeigneten Schnittdarstellungen 72 zu finden.

Zur Anwendung der erfindungsgemäßen 3D Strukturmarkierung 500 wird ein virtuelles 3D Bild von mindestens einem realen Körperteil, z.B. vom Kopf 90a eines Patienten bereitgestellt, im bevorzugten Ausführungsbeispiel z.B. über ein modernes 3D DVT Röntgensystem, siehe Fig. 13.

Die räumliche Auflösung solcher 3D Röntgenbilder kann Werte um 0,1 mm (Millimeter) erreichen, wobei in Zukunft noch bessere Auflösungen zu erwarten sind, d.h. Auflösungen kleiner als 0,1 mm aber bspw. größer als 0,001 mm. Das ist für eine präzise Positionierung und Orientierung ausreichend.

Die virtuellen Schnittbilder werden z.B. mit Hilfe eines Monitors als Ausgabeeinheit 72a visualisiert, der die Ansichten von gewählten Schnittebenen und den dazu senkrechten Orientierungen weiterer Schnittebenen meist in verschiedenen Bildschirmteilbereichen nebeneinander zeigt.

Der Abbildungsbereich und die Orientierung und Lage der Schnittebenen kann durch Bedienelemente bzw. Bedienvorrichtungen 73 verschoben, gedreht und skaliert werden.

Verfahren zur Ausrichtung der 3D Strukturmarkierung
a) Darstellung und 3D Positionsbestimmung von Landmarken/ Merkmalen allgemein
b) Darstellung, Positionierung und Orientierung von Achsen
c) Ausrichtung der Bildebenen auf die Hauptebenen und Hauptachsen des Körperteils
d) Anzeige der 3D Strukturmarkierung im digitalen bzw. virtuellen Bildraum
e) Freiheitsgrade und individuell zu definierende Parameter der 3D Strukturmarkierung
f) Positionierung, Ausrichtung und Skalierung der 3D Strukturmarkierung im digitalen bzw. virtuellen Bildraum
g) Verwenden von mehreren 3D Strukturmarkierungen 500
h) 3D Strukturmarkierung zur Analyse der Harmonie und Symmetrie im betrachteten Körperteil
i) Planung von Zahnersatz oder Zahnveränderungen mit Hilfe der 3D Strukturmarkierung
j) Planung und Fertigung von Trainingsvorrichtungen und Orthesen
k) Spezielle Bedeutung der transversalen idealisierten Okklusionsebene als Trenn-Gleit-Ebene
   a) Darstellung und 3D Positionsbestimmung von Landmarken/ Merkmalen allgemein
      □ Auswahl der Hauptansicht, so dass die Landmarken zu sehen sind, bei symmetrischen Landmarken mindestens eine der beiden und Bestimmung der 3D Position.
      □ Bei angenähert symmetrischen Landmarken, die nicht exakt in einer Bildebene liegen, Verstellen der Bildebene nach Bedarf und 3D Bestimmung der zweiten Landmarke ebenso.
      □ Bei Bedarf Umschalten der Hauptansicht auf die anderen beiden dazu orthogonalen Bildebenen, so dass je nach Situation und aktueller Fragestellung im Verfahren die geeigneten Schnittebenen dargestellt werden können.
      □ Um z.B. rechts links symmetrische Landmarken genau zu definieren, wird die sagittale Schnittebene bei Bedarf in den Bereich dieser Landmarken verschoben und ebenso die transversale und/oder frontale Schnittebene. Damit gelingt die Definition der anatomischen Landmarken mit hoher Präzision.
   b) Darstellung, Positionierung und Orientierung von Achsen
      □ Achsen ergeben sich als fortgesetzte Verbindungslinien zwischen zwei Landmarken, die jeweils eine definierte 3D Position haben.
      □ Achsen sind im 3D Bildsystem meist als digitale Objekte 66 definiert und können im virtuellen 3D Raum 50 eingeblendet und/oder ausgeblendet werden.
      □ Achsen ergeben sich auch als Schnittlinien zwischen zwei zueinander nicht parallelen Ebenen.
   c) Ausrichtung der Bildebenen auf die Hauptebenen und Hauptachsen des Körperteils
      □ Ausrichtung der Schnittebene der Visualisierung 72 so, dass die Flächennormale auf die Schnittebene als Transversalachse 215, siehe Fig. 19, durch rechts links symmetrische Landmarken 281 der knöchernen Strukturen verläuft.
      □ Im bevorzugten Ausführungsbeispiel Ausrichtung an den Scheiteln der Jochbeinbögen, siehe Fig. 19, 215L und 215R. Damit wird die Orientierung aller Sagittalebenen definiert, die grundsätzlich auf der Transversalachse 215 senkrecht stehen. Man kann auch andere symmetrische Landmarken des Kraniums 200 verwenden, soweit sie im oberen und vorderen Gesichtsschädel liegen. Der Bereich der Nasenscheidewand und des Rachens ist allerdings sehr asymmetrisch und kaum bzw. weniger geeignet.
      □ Zudem wird die Mitte 211 des Abstandes zwischen den symmetrischen Landmarken bestimmt, bzw. mehrerer Abstände, um eine geeignete Position für die mediane Sagittalebene Sm zu finden. Fig. 19 und Fig. 27 zeigen den Abstand 209 zur Mitte 211 beiderseits und die mediane Sagittalebene Sm, die durch die durch die Mitte 211 verläuft.
      □ Optional Kontrolle der Position der Mitte 211 durch Abgleich mit der Position des Nasen-Punktes 601, siehe Fig. 20 bis 24, oberhalb der Nasenwurzel. Da sich in Ankerpunkt 601 im hier gewählten Ausführungsbeispiel des Kopfes (Nasen-Punkt NP) geometrisch betrachtet eine Sattelfläche befindet, kann die Lage des Ankerpunktes 601 als Mitte 211 der Transversalachse 215 eher ungenau sein.
      □ Optional Abgleich mit der Mitte 212 der Innenohrachse 285 zwischen den Rechts-Links symmetrischen Landmarken 280L und 280R des Innenohrs bzw. des Gleichgewichtsorgans, siehe Fig. 27. Hier zeigt sich zwar oft eine messbare Abweichung der Achsrichtung 285 von der oben definierten transversalen Achsrichtung 215, aber die Landmarken 280L und 280R am Innenohr eignen sich zur Bestimmung der Mitte 212 und damit zur mittigen Lage der medianen Sagittalebene Sm sehr gut.
      □ Auswahl der Bildebene senkrecht zur oben definierten Transversalachse 215 und durch die oben definierten Mitte 211, 212 und ggf. Berücksichtigen der optionalen zusätzlichen Ankerpunkte 602, 603.
      □ Anzeige der medianen Sagittalebene Sm als Schnittebene mindestens in einem Teilbereich der Visualisierungsvorrichtung 72.
   d) Anzeige der 3D Strukturmarkierung im virtuellen Bildraum
      Nach dieser ersten Ausrichtung der Bildebenen (Sagittalebenen, Frontalebenen, Transversalebenen) für die Visualisierung 72 kann die erfindungsgemäße 3D Strukturmarkierung 500 eingefügt und ausgerichtet werden durch:
      □ Erzeugen eines digitalen bzw. virtuellen Abbildes der 3D Strukturmarkierung 500, die bevorzugt im Datenbereich 60 als Datenobjekt 55 vorliegt, als skalierbares 3D Objekt 500.
      □ Überlagern des virtuellen 3D Bildes des Körperteils bzw. Schädels mit der 3D Strukturmarkierung 500 im digitalen bzw. virtuellen Raum 50.
      □ Anzeige von Ebenenpaketen 501, 502, 503 im Schnittbild, wobei die Ebenen der Ebenenpakete im Schnittbild als parallele Linien erscheinen. Aufeinander senkrecht stehende Ebenen erscheinen bei winkeltreuer Widergabe als rechtwinklig gekreuzte Linien T, F, S (R und L orientiert).
      □ Bei Bedarf Einblenden und/oder Ausblenden der Ebenenpakete oder Einblenden und/oder Ausblenden einzelner Ebenenpakete.
   e) Freiheitsgrade und individuell zu definierende Parameter der 3D Strukturmarkierung Die exakte individuelle Skalierung, Positionierung und Orientierung der 3D Strukturinformation 500 kann in der Regel mindestens 7 Freiheitsgrade umfassen:
      □ 3 Freiheitsgrade (Parameter) der Translation auf den drei Koordinatenachsen.
      □ 3 Freiheitsgrade (Parameter) der Rotation der Raumorientierung der Hauptachsen.
      D Mindestens 1 Freiheitsgrad (Parameter) der Skalierung der 3D Strukturmarkierung 500.
   f) Positionierung, Ausrichtung und Skalierung der 3D Strukturmarkierung 500 im digitalen bzw. virtuellen Bildraum 50

Der erste Hauptzweck der erfindungsgemäßen Strukturmarkierung kann in der Analyse der Symmetrie und Harmonie des Körperteils liegen, insbesondere des Schädels und der Kieferbereiche des Oberkiefers OK und des Unterkiefers UK. Dazu kann die 3D Strukturmarkierung 500 zuerst an der sagittalen Hauptebene ausgerichtet und im Weiteren mit Hilfe der Strukturmarkierung 500 selbst die transversale Hauptebene definiert werden durch:
□ Einbringen der virtuellen 3D Strukturmarkierung 500 als digitales oder virtuelles Objekt in den virtuellen Bildraum 50. Im Sagittalschnitt ist das Gitter der parallelen Linien T1 ... Tn und F1 ... Fn zu sehen.
□ Ausrichten der 3D Strukturmarkierung 500 mit ihrer medianen Sagittalebene direkt in die mediane Sagittalebene Sm des kontrastreich abgebildeten Körperteils, im bevorzugten Ausführungsbeispiel insbesondere des knöchernen Schädels 200.
D Anzeige der Ankerpunkte 601, 602, 603 für die Ausrichtung der 3D Strukturmarkierung 500.
D Im Ausführungsbeispiel am Kopf für dentalmedizinische und kieferorthopädische Anwendungen ist der Ankerpunkt601 (Nasen-Punkt NP) im Nasion positioniert ist und der Ankerpunkt 602(Basis-Punkt BP) ist im Basion positioniert und der Ankerpunkt 603 (Gaumen-Punkt GP) ist im Knochenbereich zwischen Gaumendach und Schädelbasis positioniert. Andere Ankerpunkte bzw. mehr oder weniger Ankerpunkte sind ebenfalls möglich.
□ Positionierung der noch nicht orientierten und noch nicht skalierten 3D Strukturmarkierung 500 mit einem Drehpunkt bspw. am Ankerpunkt 602. Bevorzugt ist als Drehpunkt bzw. Drehachse D ein Kreuzungspunkt von T und L Linien geeignet. Im Ausführungsbeispiel Positionierung des Kreuzungspunktes der Linie F5 mit T3 direkt auf den Ankerpunkt 602 (BP).
□ Drehung der 3D Strukturmarkierung 500, bevorzugt um den Ankerpunkt 602, so dass die Raumrichtung der T-Linien parallel ist zur Linie durch die Ankerpunkte 602 und 603. Im Ausführungsbeispiel Drehung um GP so weit bis Linie T3 tangential durch Ankerpunkt 603 (GP) verläuft. □ Optional Skalierung der 3D Strukturmarkierung 500 durch Verkleinern oder Vergrößern soweit, dass eine definierte F Linie durch den Ankerpunkt 601 (NP) verläuft. Im Ausführungsbeispiel Skalieren mit Fixpunkt 602 (BP) ohne Rotation, bis Linie F1 tangential durch Ankerpunkt 601 (NP) verläuft. Die Linie T3 verläuft auch nach der Skalierung durch Ankerpunkt 602 (BP) und tangential durch Ankerpunkt 603 (GP). Alternativ kann die optionale Skalierung auch vor der Rotation erfolgen.
□ Damit ist die Position der 3D Strukturmarkierung 500 in den drei Raumkoordinaten ebenso definiert wie die Raumorientierung in den drei Raumwinkeln und zudem kann nun auch die Größe der 3D Strukturmarkierung 500 definiert sein. Durch die bspw. isotrope Skalierung in die drei Raumrichtungen ergeben sich damit auch die Ebenenabstände aller Ebenenpakete 501 (T transversal), 502 (F frontal), 503 (S bzw. R und L sagittal), siehe Fig. 19 bis 22.

Die oben beschriebenen Schritte zur Positionierung, Orientierung und Skalierung können alternativ zeitlich anders aufeinander folgen und oder durch zusätzliche Translationen, Rotationen und Skalierungen erweitert werden. Es kann auch eine der Operationen Positionierung, Orientierung und Rotation bzw. Drehung weggelassen werden. Alternativ können mehrere dieser Operationen weggelassen werden. Entscheidend kann sein, dass zum Abschluss der Ausrichtung die 3D Strukturmarkierung 500 individuell skaliert ist und dass die Landmarken/ Merkmale und Ankerpunkte in geeigneter Weise erreicht sind, d.h. geeignet überlappt werden. Das obige Ausführungsbeispiel ist einer der direktesten Wege, dies zu erreichen.

Mit geeigneten technologischen Mitteln der Mustererkennung und der Automation lassen sich diese Schritte und sowie Parameterzuweisungen teils bzw. teilweise oder vollständig automatisch gestalten.

### g) Verwenden von mehreren 3D Strukturmarkierungen 500

Die erfindungsgemäße 3D Strukturmarkierung 500 kann einfach oder auch mehrfach in den betrachteten digitalen bzw. virtuellen 3D Raum 50 hineinkopiert werden:
□ Die mehrfache Verwendung kann z.B. dazu dienen, mehreren relativ zueinander beweglichen Körperteilen je eine 3D Strukturmarkierung zuzuweisen, z.B. 500.A und 500.B.
□ So kann sowohl dem Kranium 200 als auch dem Unterkiefer UK je eine 3D Strukturmarkierung zugewiesen werden, siehe Fig. 28.
□ Auch innerhalb eines Körperteils können mehr als eine 3D Strukturmarkierung 500 zur Anwendung kommen.
□ Für viele Anwendungsfälle kann eine zusätzliche 3D Strukturmarkierung 500 eingebracht, um sie an unphysiologischen, unsymmetrischen oder unharmonischen Merkmalen auszurichten und deren Abweichung von der Symmetrie und Harmonie der Gestalt damit sichtbar und messbar zu machen.

### h) 3D Strukturmarkierung 500 zur Analyse der Harmonie und Symmetrie im betrachteten Körperteil

Die eingepasste 3D Strukturmarkierung 500 kann die Symmetrie und die geometrische Harmonie des Körperteils zeigen:
□ Oben im Bereich des oberen vorderen Kraniums 200 kann sich die Symmetrie zeigen, nach der die Transversalachse 215 und die mediane Sagittalebene Sm definiert wurden.
□ Weiter unten im Schädelbereich, d.h. im Bereich der Nasenscheidenwand und des Rachens, können sich deutliche Asymmetrien zeigen, siehe bspw. Fig. 19, 26, 27.
□ Bei der Vermessung der Innenohrachse 285 anhand der Innenohr-Landmarken 280L und 280R kann sich eine Raumorientierung der Innenohrachse 285 (IOA) ergeben, die nicht immer parallel ist zur Transversalachse 215, die sich aus den knöchernen Landmarken des vorderen oberen Gesichtsschädels ergibt, insbesondere aus anatomischen Landmarken. Dies kann ein Zeichen für die Asymmetrie des vorderen Kraniums 200 im Vergleich zum hinteren Kranium 200 sein.
□ Bei der Einpassung einer Strukturmarkierung 500 an die Anatomie der Zahnbögen 221 und 231, insbesondere an den Zahnbogen 221 des Oberkiefers OK, siehe Fig. 29 bis 32, kann sich oft ein Kippwinkel 214 im Frontalschnitt ergeben, mit dem die aktuelle Okklusionsebene gegen die kraniale Transversalebene verkippt sein kann.
□ Die aktuelle Achse 216 kann gegenüber der Transversalachse 215 sowohl verkippt als auch verlagert sein. In vielen Fällen kann die aktuelle Okklusionsebene zu weit oben stehen, z.B. wenn der Unterkiefer UK zu klein ist und zudem die Zähne bereits abgeschliffen sind, siehe bspw. Fig. 29.
□ Wenn man eine Strukturmarkierung 500.A ins Kranium 200 einpasst wie oben beschrieben und eine zweite Strukturmarkierung 500.B in den Unterkiefer UK am Kiefergelenk, so kann die Verkippung und Verlagerung des Unterkiefers UK sehr gut sichtbar und direkt messbar sein, siehe Fig. 28.

### i) Planung von Zahnersatz oder Zahnveränderungen mit Hilfe der 3D Strukturmarkierung 500

Die Transversalebene 501.1 (T1), siehe Fig. 20, 23, 24, 29, 30, 31, 32, bzw. die Bezugsebene T1a, siehe Figur 20, kann bei eingepasster 3D Strukturmarkierung 500 die idealisierte Lage der Okklusionsebene 201 markieren, ausgerichtet an den Landmarken der knöchernen Strukturen des Schädels 200:
□ In den meisten praktischen Fällen kann die aktuelle Lage der Okklusionsebene von der idealen Okklusionsebene 201 abweichen.
□ Die Abweichung kann sowohl in der vertikalen Position als auch in der Neigung seitlich zur Achse 215, als auch in der Neigung nach hinten zur Achse 205 bestehen.
□ Insbesondere bei der Herstellung von Zahnersatz werden die Zähne bevorzugt ausgerichtet an der idealen Okklusionsebene 201 und damit abweichend von der bisherigen Situation so angeordnet und ausgeformt, dass die neue Okklusionsebene näher an der idealen Okklusionsebene 201 liegt.
□ Dabei können kosmetische Rahmenbedingungen eingehalten werden. Die Veränderung anhand der 3D Strukturmarkierung 500 kann aber in den allermeisten Fällen zu sehr deutlichen Verbesserungen auch im optischen Erscheinungsbild führen, weil nunmehr die Symmetrie in Unterkiefer UK und Oberkiefer OK zum oberen Gesichtsschädel besser ist als zuvor.
□ An Stelle von Implantaten und Kronen können auch Veniers (Verblendungsschalen) gefertigt werden, um die Okklusionsebene zu korrigieren, so dass sich in Okklusion der Abstand zwischen Unterkiefer UK und Oberkiefer OK vergrößert. Die optische Wirkung kann ein jüngeres, dynamischeres Erscheinungsbild sein. Die biomechanische Wirkung kann in einer erheblichen Entlastung des Kiefergelenks liegen.

### j) Planung und Fertigung von Trainingsvorrichtungen und Orthesen

Eine besondere Verwendung kann die Strukturmarkierung 500 und die damit ermittelte ideale Lage der Okklusionsebene 201 bei der Planung und Herstellung von Vorrichtungen 300 für das Training und/oder für die Therapie bei Fehlstellungen finden, insbesondere des Kiefergelenks:
□ In einem Schritt kann die Bildebenen ausgerichtet werden, wie oben beschrieben.
D In einem weiteren Schritt kann mindestens eine 3D Strukturmarkierung 500 eingebracht werden.
□ Die 3D Strukturmarkierung kann wie oben beschrieben positioniert und/oder ausgerichtet und/oder skaliert werden.
□ Damit kann die idealisierte Okklusionsebene 201 definiert werden, im bevorzugten Ausführungsbeispiel liegt sie in Ebene 501.1 (T1), siehe bspw. Fig. 31 und 32.
□ Diese idealisierte Okklusionsebene 201 mit ihren Raumrichtungen 205 und 215 kann nun als Grundebene für den Aufbau von Vorrichtungen 300 etc. im digitalen bzw. virtuellen Raum dienen.
□ Für Vorrichtungen 300 mit Oberteil 320 und Unterteilen 330 kann die Trennebene 301 zwischen 320 und 330 in die Lage der idealisierten Okklusionsebene 201 positioniert werden, d.h. insbesondere die Hauptachsen 315 und 305 der Vorrichtung 300 können nach den Hauptachsen 215 und 205 des Kraniums 200 ausgerichtet werden.
□ In dieser Ausrichtung der Vorrichtung 300 relativ zum Kranium 200 kann sich ein fester Bezug zu den Zähnen des Oberkiefers OK ergeben. Weniger fix kann der Bezug zu den Zähnen des Unterkiefers UK sein, weshalb sich hier Gestaltungsmöglichkeiten für Therapie und/oder Training und ggf. Kosmetik bieten können.
□ Die Aussparungen für Zähne, Zahnfleisch und andere volumenbeanspruchende Teile im Mund können sich aus der Überschneidung der im virtuellen Raum abgebildeten Gewebe mit den Materialien der Vorrichtung 300 ergeben. Hier können Boolesche Operatoren eingesetzt werden, insbesondere Subtraktion, AND, NOT, OR bzw. Kombinationen dieser Operatoren.
□ Das Oberteil 320 kann die Überschneidung mit dem Zahnbogen des Oberkiefers 220 zeigen, wobei die Aussparungen an Material im Oberteil 320 so gestaltet werden können, dass es zu keinen Kollisionen kommt und dass die Vorrichtung 320 im Mund auf dem Zahnbogen 221 passgenau sitzt und die weicheren Gewebe im Oberkiefer 220 nicht verletzt werden.
□ Das Unterteil 330 kann die Überschneidung mit dem Zahnbogen 231 des Unterkiefers UK zeigen, nachdem der Unterkiefer UK biomechanisch und physiologisch geeignet im digitalen bzw. virtuellen Raum 50 positioniert worden ist. Dazu kann die idealisierte Okklusionsebene geeignet sein, die bei dem erläuterten Verfahren mit der Ebene T1 bzw. der Bezugsebene T1a übereinstimmt. Auch hier können die Aussparungen an dem Material des Formteils 330 so gestaltet werden, dass es zu keinen Kollisionen kommt und dass die Vorrichtung 330 im Mund auf dem Zahnbogen 231 passgenau sitzt und die weicheren Gewebe im Oberkiefer 230 nicht verletzt werden.
□ Die Vorrichtung 300 mit den Teilen 320 und 330 kann nach der Gestaltung im virtuellen 3D Raum als reale Vorrichtung hergestellt werden, entweder additiv, z.B. durch 3D Druck, oder subtraktiv, z.B. durch 3D Preforms, die mit 3D Fräsmaschinen bearbeitet werden.
□ Die gefertigten Formteile können absolut individuell genau zu den Zahnbögen der Individuen passen und können positionsgenau im Mund des Individuums sitzen.
□ Die realisierte Trennebene zwischen Oberteil 320 und Unterteil 330 kann der Gestaltung entsprechend parallel zu den Transversalebenen T und im Idealfall auf der Transversalebene T1 bzw. auf der Bezugsebene T1a liegen.

### k) Spezielle Bedeutung der transversalen idealisierten Okklusionsebene als Trenn-Gleit-Ebene

Die eingepasste 3D Strukturmarkierung 500 kann die Lage und die Orientierung der Transversalebenen definieren:
□ Die ideale Okklusionsebene bzw. Kauebene kann eine solche Transversalebene sein und damit orthogonal zur Vertikalachse liegen.
□ Die Muskulatur, die den Unterkiefer UK anhebt und gegen den Oberkiefer OK presst, setzt oben im Kranium 200 rechts und links symmetrisch an.
□ Wenn die Bewegung des Unterkiefers UK durch eine geeignete Vorrichtung in seitlicher Richtung genau in der Transversalebene frei gegeben wird, kann das unter Druck ein labiles Gleichgewicht der Lage bedeuten.
□ Dieses labile Gleichgewicht kann sich ergeben, weil bei freier seitlicher Bewegung die resultierende Kraft zwischen Unterkiefer UK und Oberkiefer OK senkrechts steht wie bei einem Tänzer bzw. Eiskunstläufer, der auf einem Bein balanciert.
□ Eine solche Instabilität kann das regulative bzw. vegetative Nervensystem trainieren und dies kann große und nutzbare Auswirkungen auf die neurophysiologische Steuerung und auf das sensomotorische System des Kauapparates haben.
□ In einem besonders bevorzugten Ausführungsbeispiel kann die Trennebene zwischen Oberteil 320 und Unterteil 330 als glatte, retentionsfreie, d.h. keine Überstände, und/oder reibungsarme Trenn-Gleit-Fläche 301 ausgeführt werden, so dass die Kontaktkraft senkrecht auf dieser Trenn-Gleit-Fläche steht, solange keine seitlichen Anschläge erreicht werden.
□ Im besonders bevorzugten Ausführungsbeispiel kann eine so gestaltete Vorrichtung genau so in den virtuellen Raum eingepasst werden, dass die Trenn-Gleit-Ebene 301 genau in der idealisierten Okklusionsebene 201 liegt oder zumindest angenähert dort angeordnet ist.
□ Wenn solche Vorrichtungen mit Trenn-Gleit-Ebene 301 bei individualisierter Ausrichtung in den Mund eingesetzt werden, ergeben sich nachweislich sehr vorteilhafte Trainingseffekte auf die Kiefermuskulatur und auf das Kiefergelenk, bis hin zu massiven therapeutischen Wirkungen.

Auf diese Weise können mit Hilfe der erfindungsgemäßen 3D Strukturmarkierung 500 hochwirksame Vorrichtungen gestaltet und hergestellt werden, die ausgezeichnete therapeutische Wirkung zeigen.

In den Funktionstests hat sich gezeigt, dass eine 3D Strukturmarkierung, die möglichst exakt in den Ebenenabständen der Fibonacci-Folge 3, 5, 8, 13 gehorcht, besonders gut geeignet ist, um Vorrichtungen herzustellen, die zu sehr guten therapeutischen und auch ggf. auch zu sehr guten kosmetischen Ergebnissen führen. Deshalb benutzen die besonders bevorzugten Ausführungsformen der 3D Strukturmarkierung genau dieses Abstandsverhältnis, siehe Lineale in Fig. 26 und Fig. 29 bis 32.

Umgekehrt kann die Wirksamkeit der Vorrichtungen im Einsatz schlechter werden, wenn sie von der Orientierung der Hauptebenen und Hauptrichtungen des Kraniums 200 und von der harmonischen Symmetrie der Hauptebenen abweichen. Damit konnte die idealisierte Lage der Okklusionsebene 201, siehe bspw. Fig. 19, wie sie sich aus der Anwendung der erfindungsgemäßen 3D Strukturmarkierung 500 ergibt, in der praktischen Anwendung bereits umfassend nachgewiesen werden.

Die Vorrichtungen können bspw. nach mehreren Wochen iterativ angepasst werden, wobei die 3D Strukturmarkierung in einer neuen Aufnahme des Schädels des Patienten positioniert wird, um die Okklusionsebene zu verändern, die nun an anderer Stelle als zu Beginn der Handlung liegen kann.

Es gibt zahlreiche 3D Bildbetrachtungssysteme im Markt, die das Einpassen von Objekten in andere 3D Bilddaten ermöglichen, z.B. unter Verwendung einer Computermaus, eines sogenannten Trackballs oder auch einer Sprachsteuerung, d.h. halbautomatisch mit einem gewissen manuellen Anteil. Die Objekte können Stifte, Implantate o.ä. sein. Alternativ kann das Objekt aber auch eine Strukturmarkierung sein, die vorher mit dem 3D Bildbetrachtungssystem oder einem anderen Programm, z.B. zur Bildbearbeitung oder ein CAD (Computer Aided Design) festgelegt worden ist. Beispielsweise kann das Programm Extraoral-3D-Family verwendet werden oder ein anderes geeignetes Programm, z.B. von der Firma Sirona3D.

In einer weiteren Entwicklungsstufe kann das 3D Bildbetrachtungssystem so angepasst werden, dass die 3D Strukturmarkierung automatisch an den Ankermerkmalen bzw. Ankerpunkten ausgerichtet wird, wobei die Translationen, die Rotation und die Skalierung ebenfalls automatisch bzw. vollautomatisch ausgeführt werden. Ein manuelles Eingreifen oder Nachjustieren kann möglich sein.

Die Ausführungsbeispiele sind nicht maßstabsgetreu und nicht beschränkend. Abwandlungen im Rahmen des fachmännischen Handelns sind möglich. Obwohl die Erfindung im Detail durch das bevorzugte Ausführungsbeispiel näher illustriert und beschrieben worden ist, ist die Erfindung nicht durch die offenbarten Beispiele eingeschränkt und andere Variationen können vom Fachmann hieraus abgeleitet werden, ohne den Schutzumfang der Erfindung zu verlassen. Die in der Einleitung genannten Weiterbildungen und Ausgestaltungen können untereinander kombiniert werden. Die in der Figurenbeschreibung genannten Ausführungsbeispiele können ebenfalls untereinander kombiniert werden. Weiterhin können die in der Einleitung genannten Weiterbildungen und Ausgestaltungen mit den in der Figurenbeschreibung genannten Ausführungsbeispielen kombiniert werden.

Das vorgeschlagene Verfahren und seine Weiterbildungen können nicht für die Behandlung des menschlichen oder tierischen Körpers verwendet werden und können kein Diagnoseverfahren sein, das am menschlichen oder tierischen Körper praktiziert wird. Alternativ, kann das vorgeschlagene Verfahren und seine Weiterbildungen für die Behandlung des menschlichen oder tierischen Körpers verwendet werden und sie können ein Diagnoseverfahren sein, das am menschlichen oder tierischen Körper praktiziert wird.

Offenbart ist eine 3D Strukturmarkierung als neuartiges 3D Werkzeug zur Ausrichtung von virtuellen 3D Objekten in räumlicher Relation zu Körperteilen wie z.B. dem Kopf oder der Wirbelsäule, die ebenfalls nach bildtechnischer 3D Erfassung in 3D Systemen virtuell dargestellten werden. Diese Ausrichtung im virtuellen Raum hat zum Ziel, die individuell an der Knochensubstanz auszurichtenden Zähne, Implantate und Vorrichtungen virtuell in einen physiologisch möglichst hoch funktionalen Zustand zu bringen, um sie anschließend anhand der virtuellen 3D Daten mit dieser ausgezeichneten Eigenschaft zu fertigen. In einer besonders bevorzugten Anwendung am Kopf und Kiefer von Patienten dient die 3D Strukturmarkierung dazu, sehr schnell, sicher und elegant die ansonsten sehr komplexe und langwierige Definition der Hauptachsen und Hauptebenen vorzunehmen. Die Hauptachsen und Hauptebenen des Kraniums sind entscheidend für die Funktionalität von Zähnen, Zahnersatz oder kieferorthopädischen Vorrichtungen. Eine besonders wirkungsvolle Anwendungsgruppe ergibt sich durch die Anwendung der 3D Strukturmarkierung zur Planung und Fertigung von Vorrichtungen, die über eine Trenn-Gleit-Ebene verfügen, die möglichst parallel zur kranialen Transversalebene orientiert ist. Die 3D Struktur der Strukturmarkierung 500 erlaubt überdies die einfache aber präzise vertikale Positionierung der idealen Okklusionsebene, die mit anderen Verfahren gar nicht oder nur schwer durchzuführen wäre. Die 3D Strukturmarkierung kann auch als 3D Ruler (Lineal) bezeichnet werden, insbesondere wenn Größenmaßstäbe mit angezeigt werden, die durch die optionale Skalierung geeignet angepasst werden können.

Die Offenbarung bezieht sich auch auf folgende Merkmale und Kombinationen beliebig ausgewählter Merkmale, wobei die Erfindung durch die Patentansprüche bestimmt ist:

### 3D Werkzeug

Eine 3D Strukturmarkierung 500 als Werkzeug für die Anwendung im virtuellen 3D Raum,
umfassend mindestens zwei Ebenenpakete, jeweils aus planparallelen Ebenen, wobei jedes der Ebenenpakete aus mindestens 3 Ebenen besteht, die in einem charakteristischen Abstandsverhältnis stehen, wobei mindestens zwei Ebenenpakete 501 und 502 in ihrer Orientierung aufeinander senkrecht stehen.

Die 3D Strukturmarkierung 500 kann über eine Transversalachse verfügen, die der transversalen Orientierung und Ausrichtung der Strukturmarkierung im virtuellen Raum dient, insbesondere der Ausrichtung an knöchernen Strukturen von Körperteilen in einem virtuellen 3D Bild der jeweiligen Körperteile.

Bei der 3D Strukturmarkierung 500 können die Abstände zwischen den planparallelen Ebenen innerhalb mindestens eines Ebenenpakets ein definiertes Abstandsverhältnis aufweisen, das dem Bildungsgesetz der Fibonacci Reihe 3, 5, 8, 13, 21 etc. zumindest teilweise entspricht.

Bei der 3D Strukturmarkierung 500 können die Abstände zwischen den planparallelen Ebenen mindestens eines Ebenenpakets ein konstantes Proportionalitätsverhältnis aufweisen, wobei der Abstand der nachfolgenden Ebene zum vorhergehenden Abstand im Verhältnis zwischen 1,8 und 1,5 liegt.

Bei der 3D Strukturmarkierung 500 können die Abstände der planparallelen benachbarten Ebenen ganz oder fast exakt proportional sein und der Proportionalitätsfaktor zwischen 1,60 und 1,65 liegen.

Bei der 3D Strukturmarkierung 500 können mindestens drei jeweils zueinander orthogonal stehende Ebenenpakete zu einer virtuellen 3D Struktur kombiniert sein.

Bei der 3D Strukturmarkierung 500 können die Ebenenpakete im Verbund so orientiert werden, dass ein erstes Ebenenpaket 501 mindestens 3 transversale Ebenen umfasst, ein zweites Ebenenpaket 502 mindestens 3 frontale Ebenen und ein drittes Ebenenpaket 503 mindestens 3 sagittale Ebenen in Bezug auf einen Körperteil.

Die 3D Strukturmarkierung 500 kann im Raum Kreuzungslinien der Ebenen umfassen, die in der dazu senkrechten Schnittebene als Kreuzungspunkte erscheinen, wobei in dieser Bildebene die Schnittlinien der parallelen Ebenen der Ebenenpakete mit der Bildebene als Parallelen erscheinen.

7 Freiheitsgrade (Degrees of Freedom) für die Positionierung sind zusammengesetzt aus der Definition von 3 Freiheitsgraden der Translation und 3 Freiheitsgraden der Rotation und mindestens einem Freiheitsgrad der Skalierung zur Definition der relativen Lage und Orientierung und Größe einer Strukturmarkierung 50 sind dadurch gekennzeichnet dass:
- Frontalebene Fn läuft durch Ankerpunkt 601, und/oder
- Transversalebene Tx läuft durch Ankerpunkte 602 und 603, und/oder
- Frontalebene F1 läuft durch Ankerpunkt 601.

Und damit die Definition eines Ankerpunktes für die Lagepositionierung der idealisierten Kauebene (biomechanisch individuell ideale Lage der Kauebene anhand der Form und Struktur des Schädels) durch einen Schnittpunkt zwischen Transversalebene T und Frontalebene F der Strukturmarkierung in der Sagittalansicht. Raumorientierung der idealisierten Kauebene parallel zur Transversalebenen der Strukturmarkierung.

Besonders bevorzugt ist eine Strukturmarkierung gemäß Fig. 4 erzeugt in Schnittbild gemäß Fig. 8:
- Frontalebene F5 läuft durch Ankerpunkt 601 BP, und/oder
- Transversalebene T3 läuft durch Ankerpunkte 602, BP und 603 GP, und/oder
- Frontalebene F1 läuft durch Ankerpunkt 601 NP, und/oder
- definiert Inzisalpunkt 604, IP als Schnittpunkt F1 mit T1, vorzugsweise in der medianen Sagittalebene.

### 3D Verfahren

In einem Verfahren zur Anwendung einer 3D Strukturmarkierung 500 wird die Strukturmarkierung in ihrer Transversalachse an knöchernen Strukturen ausgerichtet wird, wobei hierzu angenähert symmetrische Landmarken in den knöchernen Strukturen rechts und links verwendet werden, um tangentiale Annäherungen mit transversalen Achsen vorzunehmen und oder um die Achse durch diese Landmarken zu legen, wobei die so ausgerichtete Transversale als Flächennormale der Sagittalebenen dient, die als Bildebenen in einem 3D Bildsystem für die weitere Ausrichtung der 3D Strukturmarkierung benutzt werden.

In dem Verfahren kann zur Ausrichtung der Transversalachse die knöchernen Strukturen des vorderen Gesichtsschädels rechts und links in ihrer Achsverbindung als Transversalrichtung verwendet werden. In dem Verfahren kann die 3D Strukturmarkierung an weiteren Ankerpunkten in mindestens einer Koordinate positioniert und in mindestens einem Raumwinkel ausgerichtet werden, indem Ankerpunkte verwendet werden, die in einer Ebene liegen, die in der Bildebene oder parallel zur Bildebene liegt.

In dem Verfahren kann die 3D Strukturmarkierung soweit vergrößert oder verkleinert werden, dass mindestens zwei gegebene Ankerpunkte von vorgegebenen Linien berührt bzw. durchschnitten werden. In dem Verfahren kann die 3D Strukturmarkierung 500 soweit vergrößert oder verkleinert werden, dass mindestens drei gegebene Ankerpunkte von vorgegebenen Linien berührt bzw. durchschnitten werden. In dem Verfahren kann die 3D Strukturmarkierung 500 in einer Schnittbilddarstellung in einem Schritt um einen Ankerpunkt positioniert werden und in einem anderen Schritt um einen Winkel gedreht werden, so dass eine Hauptachse der Strukturmarkierung in Richtung der Verbindungsachse zweier Ankerpunkte eines Körperteils zeigt und in einem dritten Schritt eine Skalierung erfolgen, so dass mindestens 2 Ankerpunkte durch definierte Linien oder Kreuzungspunkte der 3D Strukturmarkierung im Schnittbild verlaufen.

In dem Verfahren kann die 3D Strukturmarkierung 500 in ihren Sagittalebenen 503 parallel zur Sagittalebene des Körperteils, insbesondere des Kopfes orientiert sein, wobei in mindestens einem Positionierungsschritt die Strukturmarkierung 500 so positioniert wird, dass ein Drehpunkt definiert wird und in einem zweiten Schritt eine Drehung der Strukturmarkierung um einen Winkel erfolgt, so dass die Transversalebene im Sagittalschnitt parallel zur Verbindungslinie zwischen zwei Ankerpunkten 602 und 603 verläuft und in einem weiteren Schritt die Skalierung der Größe und der Ebenenabstände der Strukturvorrichtung 500 so eingestellt wird, dass die Strukturmarkierung mit einer frontalen Ebene einen weiteren Ankerpunkt 601durchschneidet.

In dem Verfahren kann die Positionierung der 3D Strukturmarkierung 500 mindestens eine 3D Position eines Ankerpunktes 602, der in der Sagittalebene liegt, genau berücksichtigen und die Achsenausrichtung der Strukturmarkierung 500 so gelagert sein, dass eine Transversalebene der 3D Strukturmarkierung 500 durch den zweiten Ankerpunkt 602 angenähert tangential verläuft und eine auf der Transversalebene und der Sagittalebene senkrecht stehende Ebene mindestens einen weiteren Ankerpunkt 601 durchschneidet und dass dafür die Skalierung der 3D Strukturmarkierung geeignet eingestellt wird, um diese Bedingungen gleichzeitig zu erfüllen.

In einem Verfahren zur Positionierung und Orientierung und Skalierung einer 3D Positionsmarkierung 500 kann als Ankerpunkt für die Positionierung und Drehung der Basis-Punkt BP am Foramen Magnum verwendet werden und als Ankerpunkt für die Winkelausrichtung der Transversalebene der Gaumen-Punkt GP am obersten Punkt des Gaumendaches in der Mitte der Spingiosa bzw. in der Mitte der konkreten kompakten Knochenränder zwischen Gaumen und Schädelbasis und als Ankerpunkt für die Skalierung der Strukturmarkierung 500 der Nasen-Punkt NP am Nasion über der Nasenwurzel.

### Verwendung

Eine Verwendung der durch Ausrichtung der 3D Strukturmarkierung 500 definierten Hauptebenen und Hauptrichtungen zur exakten Winkelausrichtung und 3D Positionierung der Trenn-Gleit-Ebene von Vorrichtungen, wobei diese Trenn-Gleit-Ebene innerhalb anderweitiger mechanischer Grenzen eine seitliche reibungsarme Beweglichkeit in den Richtungen der Ebene erlaubt und eine Rotation um die Normalenachse, die auf der Trenn-Gleit-Ebene senkrecht steht.

Die Transversalebene T1 kann als Ausrichtungsebene verwendet werden für die idealisierte Trenn-Gleit Ebene von Vorrichtungen oder die idealisierte Okklusionsebene des Gebisses.

Die mittels der 3D Strukturmarkierung definierte idealisierte Trenn-Gleit-Ebene mit Position und Orientierung als Trennebene kann verwendet werden für die Herstellung realer mindestens zweiteiliger Vorrichtungen zum Einsetzen in den Körper oder zum Ansetzen am Körperteil.

Die mittels der 3D Strukturmarkierung 500 definierte idealisierte Trenn-Gleit-Ebene parallel zur kranialen Transversalebene T1 kann verwendet werden zur Herstellung von Zahnersatz oder von Implantaten oder von Vorrichtungen zum Einsetzen in den Mund, insbesondere zum Aufsetzen auf die Zahnbögen.

Obwohl die Erfindung im Detail durch das bevorzugte Ausführungsbeispiel näher illustriert und beschrieben worden ist, ist die Erfindung nicht durch die offenbarten Beispiele eingeschränkt und andere Variationen können vom Fachmann hieraus abgeleitet werden, wobei der Schutzbereich durch die Ansprüche bestimmt ist. Gleiche Bezugszeichen beziehen sich auf gleiche technische Merkmale, wenn nichts anderes gesagt wird. Soweit "kann" in dieser Anmeldung verwendet wird, bedeutet es sowohl, die Möglichkeit der Realisierung als auch die tatsächliche technische Implementation. Konzepte der Offenbarung werden mit Bezug zu bevorzugten Ausführungsbeispielen oben beschrieben in einem spezifischen Zusammenhang, nämlich einer Strukturmarkierung an einem Kopf, insbesondere für die Positionierung von Vorrichtungen im Bereich der Zähne. Die offenbarten Konzepte können jedoch auch auf andere Situationen und/oder Anordnungen angewandt werden, insbesondere auf Strukturmarkierungen an anderen Körperteilen und auf Vorrichtungen, die an diesen anderen Körperteilen angeordnet werden.

### Glossar

- Bukkal:: Zur backenseitigen Seitenfläche eines Zahnes.
- Lingual:: Zur Zunge hin gerichtete Seite
- Okklusal:: Zur Kaufläche gerichtet
- Randspalt:: Spalt zwischen Vorrichtung 100 und Körpergewebe wie Zahn oder Zahnfleisch
- DVT:: Digitales Volumen Tomogramm, stationäre 3D Röntgenaufnahme
- OK:: Oberkiefer 220
- OKP:: Oberkiefer-Preform 20
- OKM:: Oberkiefer-Teil 120 der Vorrichtung 100
- UK:: Unterkiefer 230
- UKP:: Unterkiefer-Preform 30
- UKM:: Unterkiefer-Teil 130 der Vorrichtung 100
- TGE:: Trenn-Gleit-Ebene 110 zwischen OKM 120 und UKM 130
- AKE:: Aktuelle Kau Ebene 111, näherungsweise, meist schräg stehend zur TGE 110
- Anterior-Po+sterior: von vorne nach hinten verlaufend
- BP: Basis Punkt am Basion des Foramen Magnum, bevorzugt als Ankerpunkt 2
- DVT: Digitales Volumen Tomogramm, stationäre 3D Röntgenaufnahme
- f1, f2, f3...: Abstand der Frontalebenen F
- F: erste Ebene
- F1 bis F5: Frontalebene F, Ebene mit Hauptrichtungen Rechts-Links und Oben-Unten
- GP: Gaumen-Punkt, bevorzugt als Ankerpunkt 3
- Kranium: knöcherner Schädel
- L: links
- L1 bis L5: nach links laufende Sagittalebenen , Ebene mit Hauptrichtungen Vorne-Hinten und Oben-Unten
- NP: Nasen-Punkt am Nasion, bevorzugt als Ankerpunkt 1
- OK: Oberkiefer 220
- R: rechts
- R1 bis R5: nach rechts laufende Sagittalebenen
- S: zweite Ebene

- t1, t2, t3...: Abstand der Transversalebenen T
- s1, s2, s3...: Abstand der Sagittalebenen S
- Tb: erste Schnittebene
- T1a: Bezugsebene
- Tx: Transversalebene, die durch die Ankerpunkte 602 und 603 verläuft
- T: dritte Ebene
- T1 bis T5: Transversalebene, Ebene mit Hauptrichtungen Vorne-Hinten und Rechts-Links
- UK: Unterkiefer 230
- KS0: Bezugssystem
- x0, y0, z0: Achse, Koordinate
- KS1: erstes digitales Bezugssystem
- x1, y1, z1: Achse, Koordinate
- KS2: zweites digitales Bezugssystem
- x2, y2, z2: Achse, Koordinate
- KS3: drittes digitales Bezugssystem
- x3, y3, z3: Achse, Koordinate

### Bezugszeichen

- 1 bis 8: Zahnpositionen im Kiefer
- 20: Oberkiefer-Preform OKP
- 22: Datenobjekt als Repräsentation der vorwiegenden knöchernen Strukturen des Körperteils
- 22a: Raumdaten
- 30: Unterkiefer-Preform UKP
- 33: Datenobjekt als Repräsentation der Vorrichtung 300 und ihrer Teile
- 40: realer Raum mit realen Objekten
- 50: digitaler bzw. virtueller Raum der 3D Strukturmarkierung
- 55: Datensatz, Datenobjekt als Repräsentation der 3D Strukturmarkierung
- 60: Datenraum mit Datenobjekten in Speichereinheit
- 60a: Speichereinheit
- 66: Datensatz, sonstige Datenobjekte
- 71: Recheneinheit, 3D Bildsystem mit Prozessor
- 72: visualisierte Ansichten für digitale bzw. virtuelle 3D Objekte
- 72a: Ausgabeeinheit, Monitor
- 73: Eingabemittel für Positionierung, Drehung, Skalierung etc.
- 73a: Eingabeeinheit
- 80: Imaging-Einheit
- 81: Bilddatenspeicher und Erzeugung 3D Bilddatensatz
- 82: Arm eines 3D Röntgenaufnahmegeräts mit mehreren Aufnahmerichtungen
- 83: strahlungssensitives Array zur Bilderzeugung
- 90: Kopf eines Individuums
- 90a: Körperteil
- 91: röntgendichte knöcherne Strukturen im Kopf
- 100: Vorrichtung
- 105: Achse vorne-hinten in der Vorrichtung
- 110: Trenn-Gleit-Ebene TGE zwischen OKM und UKM
- 111: Aktuelle Kau Ebene AKE ohne Korrektur der Lage des UK
- 112: Kippwinkel Kappa zwischen AKE 111 und TGE 110
- 113: Kranialer Symmetriepunkt rechts, bevorzugt im Gesichtsschädel
- 114: Kranialer Symmetriepunkt links, bevorzugt im Gesichtsschädel
- 115: Achse rechts-links in der Vorrichtung 100
- 116: Transversaler Kippwinkel
- 117: Sagittaler Kippwinkel
- 120: Oberkiefer-Teil OKM
- 121: Trainingswulst außen am OKM umlaufend
- 122: Randspalt im Oberkiefer-Modul
- 123: plan glatte Trenn Gleit Fläche am OKM
- 124: Vertiefung im OKM korrespondierend mit Zahn und Zahnfleisch
- 125: Höckerartiger oder Plateauartiger Vorsprung als Auflagepunkt im OKM
- 127: Seitliche Wände der Vertiefungen 124 im OKM bevorzugt mit Randspalt, nicht anliegend
- 129: Einstiche von den Zähnen des Unterkiefers in die Gleitfläche 123 im OKM
- 130: Unterkiefer-Teil UKM
- 131: Trainingswulst außen am UKM umlaufend
- 132: Randspalt im Unterkiefer-Modul
- 133: glatte plane Gleitfläche und Trennfläche am UKM
- 134: Vertiefung im UKM korrespondierend mit Zahn und Zahnfleisch
- 135: Höckerartiger oder plateauartiger Vorsprung als Auflagepunkt im UKM
- 136: Durchbruch der Vertiefung 134 durch die Trenngleitfläche 133
- 137: Seitliche Wände der Vertiefungen 124 im UKM bevorzugt mit Randspalt, nicht anliegend
- 150: Achse oben-unten in der Vorrichtung
- 200: Cranium, knöcherner Schädel
- 200a: knöcherne Struktur
- 201: idealisierte Okklusionsebene, physiologische Lage der Trenn-Gleit-Ebene 203 Neigungswinkel zwischen der kranialen Anterior-Posterior Achse 205 und der Bildhorizontalen, insbesondere zur horizontalen Bildeinstellung der AP-Achse 215
- 205: Achse vorne-hinten in Neigung leicht schräg nach hinten unten bei aufrechter Kopfhaltung
- 205a: zu Anterior-Posterior Achse parallele Achse
- 209: Abstand zur Mitte
- 211: Mitte
- 214: Kippwinkel der aktuellen Okklusionsebene z.B. am Zahnbogen des Oberkiefers gegenüber der kranialen Transversalachse 215
- 215: Achse rechts-links anhand anatomischer Landmarken bevorzugt des Gesichtsschädels
- 216: verkippte aktuelle Okklusionsebene
- 215 R / L: Ausrichtungspunkte für die kraniale Transversalachse 215 im vorderen Gesichtsschädel220 Oberkiefer OK
- 221: Zahnbogen im Oberkiefer
- 223: Augenhöhle
- 228: Zahn aus dem Zahnbogen des Oberkiefers
- 230: Unterkiefer UK
- 231: Zahnbogen im Unterkiefer
- 238: Zahn aus dem Zahnbogen des Unterkiefers
- 245: Orientierungsachse im oberen Gesichtsschädel
- 250: Achse oben unten im Schädel
- 256: verkippte Vertikale (Flächennormale) senkrecht auf aktueller Okklusionsebene 216
- 270: obere Ausläufer der Halswirbelsäule
- 275: Kiefergelenksknochen, gelenknaher Bereich
- 280: Innenohrstruktur, insbesondere Bogen des Gleichgewichtsorgans
- 281 R / L: symmetrische Landmarken
- 285: Innenohrachse IOA
- 295: Senkrechte auf der Innenohrachse
- 301: reibungsarme plane Trenn-Gleit-Ebene der Vorrichtung 300 zwischen Oberteil 320 und Unterteil 330
- 500: 3D Strukturmarkierung als digitales bzw. virtuelles 3D Objekt
- 501: erstes Ebenenpaket, transversales Ebenenpaket,
- 501.1 bis 501.n: Ebene
- 502: zweites Ebenenpaket, frontales Ebenenpaket,
- 502.1 bis 502.n: Ebene
- 503: drittes Ebenenpaket, sagittales Ebenenpaket,
- 503R: nach rechts laufendes sagittales Ebenenpaket
- 503R.1 bis 503R.5: Ebene
- 503L: nach links laufendes sagittales Ebenenpaket
- 503L.1 bis 503L.5: Ebene
- 515: erste Hauptrichtung einer Ebene
- 550: zweite Hauptrichtung einer Ebene
- 601: drittes Ankermerkmal oder erster Ankerpunkt oben kranial im Bereich der Stirn/Nase
- 602: erstes Ankermerkmal oder zweiter Ankerpunkt hinten kranial im Bereich der Basis
- 603: zweites Ankermerkmal oder dritter Ankerpunkt vorne im Bereich des Gaumens
- 604, IP: Idealer Inzisalpunkt zwischen den oberen und unteren Schneidezähnen, der mit Hilfe der Strukturmarkierung 500 bestimmt wird
- W1, W2: Winkel
- D: Drehachse
- 700, 800: Verfahren
- 710 bis 790: Schritt
- 810 bis 890: Schritt

## Patentansprüche

1. Vorrichtung zum Training des muskulären, sensomotorischen und neurophysiologischen Systems des Kiefergelenks und zur Positionierung und Führung des Unterkiefers relativ zum Oberkiefer,
- wobei die Vorrichtung (100) mindestens zwei Module (120, 130) umfasst, die im Bereich einer vergleichsweise großflächigen glatten ebenen Trenn-Gleit-Ebene (110) aneinander liegen, ohne dass Zähne oder Zahnfissuren oder vergleichbare Formen die Seitwärtsbewegung der Module zueinander entlang der Trenn-Gleit-Ebene (110) behindern,
wobei
- mindestens eines der zwei Module (120, 130) als Oberkiefer-Modul (120) ausgeführt ist, das mehrere angenähert einen Bogen bildende Vertiefungen (124) für die Zähne (221) des Oberkiefers (220) sowie eine erste glatte Gleitfläche (123) aufweist und
- mindestens eines der zwei Module (120, 130) als Unterkiefer-Modul (130) ausgeführt ist, das mehrere Vertiefungen (134) für die Zähne (231) des Unterkiefers (220) sowie eine zweite glatte Gleitfläche (133) aufweist,
wobei die glatte ebene erste und zweite Gleitfläche der Module (123; 133) eine gute Ebenheit, eine geringe Rauheit, mit RZ bevorzugt unter 5 Mikrometer, und/oder einen sehr geringen Reibungswiderstand aufweisen, so dass entlang der Trenn-Gleit-Ebene (110) eine seitlich gleitende plan und eben geführte Gleitbewegung in zwei Raumrichtungen und/oder eine Rotation um die Achse senkrecht zur Trenn-Gleit-Ebene (110) stattfinden kann, wobei die Trenn-Gleit-Ebene (110) zusammen mit einer Dental-Kau-Ebene (111) einen ersten Winkel k (112) aufspannt, wobei der Unterkiefer und der Oberkiefer im Zustand des mechanischen Kontakts die Dental-Kau-Ebene (111) ausbilden.

2. Vorrichtung nach Anspruch 1, wobei
a) der erste Winkel k (112) in Draufsicht senkrecht zu einem Sagittalschnitt des Kraniums (200) bestimmt wird, wobei der erste Winkel vorzugsweise <10° ist, und/oder
b) die Trenn-Gleit-Ebene (110) zusammen mit der Dental-Kau-Ebene (111) einen zweiten Winkel (116) aufspannt, welcher in Draufsicht auf einen Sagittalschnitt des Kraniums (200) bestimmt wird, wobei der zweite Winkel (116) durch eine erste Achse (202), welche parallel verschoben zur Dental-Kau-Ebene (111) den Basionspunkt (BP) schneidet, sowie einer zweiten Achse (205), welche den Basionspunkt (BP) und den Gaumendachpunkt (GP) schneidet, aufgespannt wird.

3. Vorrichtung nach Anspruch 2, wobei der zweite Winkel (116) zwischen 0° und 10° groß ist.

4. Vorrichtung nach einem der Ansprüche 1 bis 3, welche in der Trenn-Gleit-Ebene (110) um einen Drehwinkel (117) gedreht ist, welcher ausgehend von einer Sagittalebene, welche durch die Schneidezähne verläuft, einen Winkel <10 °aufspannt.

5. Vorrichtung nach einem der vorhergehenden Ansprüche, wobei
a) die Vertiefungen (134; 124) so weit im Vergleich zur 3D Zahngeometrie in der Tiefe und in der Ausdehnung parallel zur Trenn-Gleit-Ebene (110) erweitert ausgeführt sind, dass beim Aufsetzen der Module auf die Zahnbögen Randspalte (122) zwischen dem Material der Module (120;130) und dem Körpergewebe und oder dem Zahnmaterial bestehen bleiben, und/oder wobei
b) ausgewählte Vertiefungen (124; 134) an einer eng umgrenzten lokalen Stelle, korrespondierend mit der Kaufläche eines Zahnes, höckerartige oder plateauartige Kontaktpunkte (125; 135) aufweisen, wobei direkt an den Kontaktpunkten die 3D Geometrie ohne Randspalt der 3D Oberfläche der Zähne entspricht und die Kontaktpunkte durch Bereiche mit Randspalt (122) umgeben sind, und/oder wobei
c) mindestens ein Modul und darin mindestens eine Vertiefung (124; 134) zumindest teilweise im Bereich der Kaufläche des Zahnes einen offenen Durchbruch (126; 226) durch das Material des Moduls (120; 130) aufweist, wobei die Vertiefung so gestaltet ist, dass der Zahn mit seiner Kaufläche teilweise außerhalb des Moduls (120; 130) liegt, im Bereich des gegenüber stehenden Moduls (130; 120), und/oder wobei
d) mindestens eine Gleitfläche (123; 133) der Module (120;130) durch mindestens eine Vertiefung (129) lokal durchbrochen wird, die mit einem Zahn des entgegen stehenden Zahnbogens korrespondiert, wobei diese Vertiefung (129) mindestens durch einen Randspalt (122) erweitert ist, der die seitliche Beweglichkeit des in die Vertiefung (129) durchtretenden Zahnes der Gegenseite entlang der Trenn-Gleit-Ebene (110) ermöglicht, und/oder wobei
e) das Material der Module (120; 130) außerhalb des Bogens der Vertiefungen (124;134) einen wulstförmigen Rand (121; 131) bilden, der im Mundraum ein so großes Volumen beansprucht, insbesondere so dass die Weichgewebe, Muskeln und Faszien durch diesen Wulstrand (121; 131) definiert gedehnt werden, und/oder wobei
f) die Module (120; 130) angenähert Hufeisenform haben und jeweils einen Zahnbogen umfassen, wobei die Vorrichtung in ihrer Position durch ein orthogonales Achsensystem (105; 115; 150) festgelegt ist, wobei die Module an den Gleitflächen (123, 133) zur Bildung der Trenn-Gleit-Ebene (110) aneinander gelegt werden und in dieser Kontaktlage fast reibungsfrei verschiebbar sind, insbesondere wenn durch den Unterkiefer (230) eine nicht auf die Trenn-Gleit-Ebene (110) exakt senkrechte Kraft in Richtung des Oberkiefers (230) auf die beiden Module im Sinne einer Druckkraft ausgeübt wird.

6. Vorrichtung nach einem der vorhergehenden Ansprüche, wobei in mindestens einem der Module die Dental-Kau-Ebene (111), die durch die Kauflächen der Zähne des Zahnbogens gebildet wird, schräg steht auf der Trenn-Gleit-Ebene (110), die Oberkiefer-Module und Unterkiefer-Module trennt und gleitend aneinander führt, wobei ein deutlich messbarer Kippwinkel k (112) zwischen den Ebenen (111) und (110) von mehr als 0,2 Grad auftritt.

7. Vorrichtung nach Anspruch 5 oder 6, falls abhängig von Anspruch 5, wobei die Vorrichtung im eingesetzten Mund durch die Wulstränder (121; 131) die Lippen mehr als 10% ausdehnt und die Vorrichtung zu maximal 90% von vorn gesehen von den Lippen überdeckt werden kann, wobei die Trenn-Gleit-Ebene (110) von vorne sichtbar ist und die transversale rechts-links Achse (115) in ihrer Achsrichtung messtechnisch erfassbar ist.

8. Vorrichtung nach einem der vorhergehenden Ansprüche, wobei mindestens eines der Module mehrteilig ausgeführt ist, insbesondere zweiteilig, wobei die Module über Vertiefungen (124; 134) verfügen, die vorwiegend mit den Backenzähnen und oder Eckzähnen des jeweiligen Zahnbogens korrespondieren, wobei die Teile eines Moduls zusammen die gleiche Trenn-Gleit-Ebene (110) ausbilden, wenn sie gemeinsam in den Mund eingeführt und am Zahnbogen positioniert werden.

9. Vorrichtung nach Anspruch 5 oder einem der Ansprüche 6 bis 8, falls abhängig von Anspruch 5, wobei der wulstförmige Rand (131) des Unterkiefer-Moduls (130) im Bereich der Frontzähne mehr als 1mm breiter ist als der wulstförmige Rand (121) des Oberkiefer-Moduls (120) und die beiden Module an den Außenbereichen zur Wange hin einen angenähert ebenen Übergang ohne deutliche Kontursprünge aufweisen.

10. Vorrichtung nach einem der vorhergehenden Ansprüche, wobei die Position der Vertiefungen (124; 134) in den Modulen (120; 130) dadurch 6-dimensional eingestellt wird, mit 3 Raumpositionen und 3 Raumrichtungen, indem ein digitales 3D Modell der Vorrichtung so in das digitale 3D Modell des Schädels eingepasst wird, dass die orthogonalen Achsen des Gesichtsschädels (205; 215; 250) parallel sind zu den orthogonalen Achsen (105; 115; 150) der Trenn-Gleit-Ebene (110), wobei die rechts-links-Achsen (115; 215) und die vorne-hinten-Achsen (105; 205) und die oben-unten-Achsen (150; 250) im Sinne der Orthogonalität aufeinander senkrecht stehen und die Position der orthogonalen Achsen des Schädels anhand typischer knöcherner Landmarken des Schädels, insbesondere des Gesichtsschädels im 3D Model definiert wird.

11. Vorrichtung nach einem der vorhergehenden Ansprüche, wobei mindestens eine der Vertiefungen (124; 134) sowohl einen Kontaktpunkt (125; 135) als auch einen Durchbruch (126; 136) umfasst, wobei die Umgebung des Kontaktpunktes mit Randspalt (122; 132) ausgeführt ist.

12. Vorrichtung nach einem der vorhergehenden Ansprüche, wobei die Randspalte (122; 132) in der Umgebung der durchbrechenden Vertiefungen (129, 139) in der Trenn-Gleit-Ebene (110; 123; 133) so weit ausgearbeitet sind, dass eine seitlich gleitende Bewegung in zwei Richtungen rechts-links und oder hinten-vorne und kombiniert um mindestens 0,3 mm möglich ist, besonders bevorzugt mindestens 0,4 bis 1,4 mm.

13. Vorrichtung gemäß Anspruch 12, wobei die rechts-links Position der Vorrichtung (100) relativ zum Schädel (200) durch die Rechts-Links-Symmetrie entlang der rechts-links-Achse (215) der knöchernen Merkmale des Gesichtsschädels und oder der rechts links-Symmetrie des Oberkiefers im Frontzahnbereich bestimmbar ist und zudem die oben-unten-Position der Vorrichtung (100) entlang der oben-unten-Achse (250) des Schädels durch den Überschneidungsbereich der Schneidezähne bzw. der Zahnbögen im Frontalzahnbereich bestimmbar ist und zudem die hinten-vorne-Position der Vorrichtung (100) entlang der hinten-vorne-Achse (205) durch den Bereich zwischen den oberen und unteren Frontzähnen.

14. Vorrichtung gemäß Anspruch 12 oder 13, wobei im 3D Modell der Vorrichtung als Erweiterung der von Zähnen und Zahnfleisch beanspruchten Volumens eine Randspaltschicht (122; 132) erzeugbar sind, die durch vereinzelte Kontaktpunkte (125; 135) auch bei Druck aufrechterhalten wird, auf denen die Vorrichtung (100) an den Zahnbögen (221; 231) in Kaudruckrichtung aufliegt, indem an der Stelle der Kontaktpunkte die Module lokal ohne Randspalt (122; 132) in Formschluss zum korrespondierenden Zahn ausgeprägt werden und anschließend diese Module (129; 130) 3D Geometrie mit Randspalt und Kontaktpunkten und der sich aus der Orientierung ergebenden Position und Lage der Vertiefungen (124; 134) einschließlich Randspalte (122) und Kontaktpunkte (125) für die Zahnbögen hergestellt wird.

15. Verfahren zur Herstellung einer individuell ausgearbeiteten Vorrichtung (100) nach einem der vorhergehenden Ansprüche zum Einsatz in den Mund insbesondere einer Person, die aus mindestens einem Oberkiefer-Modul (120) und mindestens einem Unterkiefer-Modul (130) besteht, wobei die Module eine gemeinsame Trenn-Gleit-Ebene (110) aufweisen, wobei ein digitales 3D Modell der Vorrichtung als Preforms (20; 30) mit seinen orthogonalen Achsen (105; 115;150) in einem 3D Modell des Kopfes mit dessen orthogonalen Achsen (205; 215; 250) so positioniert wird, dass die Achsen der Vorrichtung parallel zu den Achsen des Kopfes sind, wobei sich die Achsen des Kopfes (205; 215; 250) vorwiegend anhand von Landmarken im Bereich des Gesichtsschädels ergeben und dadurch die Position der Vertiefungen definiert wird, die für die Zähne der Zahnbögen des Oberkiefers und des Unterkiefers in den Preforms im 3D Digital Modell erzeugt werden, um danach die Module (120; 130) mit diesen Vertiefungen (124; 134) fertigungstechnisch additiv oder subtraktiv zu erzeugen.

## Claims

1. Device for training the muscular, sensorimotor and neurophysiological system of the temporomandibular joint and for positioning and guidance of the lower jaw relative to the upper jaw,
- wherein the device (100) comprises at least two modules (120, 130) which bear against one another in the region of a comparatively large, smooth and flat separation-sliding-plane (110) without teeth or tooth fissures or comparable forms hindering the lateral movement of the modules with respect to one another along the separation-sliding-plane (110),
wherein
- at least one of the two modules (120, 130) is designed as an upper jaw module (120) having a plurality of approximately arcuate recesses (124) for the teeth (221) of the upper jaw (220) and a first smooth sliding surface (123) and
- at least one of the two modules (120, 130) is designed as a lower jaw module (130) having a plurality of recesses (134) for the teeth (231) of the lower jaw (220) as well as a second smooth sliding surface (133),
wherein the smooth, flat first and second sliding surface of the modules (123; 133) has a good flatness, a low roughness, preferably with an RZ below 5 micrometres, and/or a very low frictional resistance, such that a laterally sliding slide movement, guided in a planar and flat manner in two spatial directions and/or a rotation about the axis perpendicular to the separation-sliding-plane (110) can take place along the separation-sliding-plane (110), wherein the separation-sliding-plane (110), together with a dental-occlusal-plane (111), forms a first angle k (112), wherein the lower jaw and the upper jaw form the dental-occlusal-plane (111) in the state of mechanical contact.

2. Device according to claim 1, wherein
a) the first angle k (112) is determined in plan view perpendicular to a sagittal section of the cranium (200), wherein the first angle is preferably <10°, and/or
b) the separation-sliding-plane (110), together with the dental-occlusal-plane (111), forms a second angle (116), which is determined in a plan view of a sagittal section of the cranium (200), wherein the second angle (116) is formed by a first axis (202), which intersects the base point (BP) when being displaced parallel to the dental-occlusal-plane (111), and a second axis (205), which intersects the base point (BP) and the high point of the palate (GP).

3. Device according to claim 2, wherein the second angle (116) is between 0° and 10°.

4. Device according to one of claims 1 to 3, which is rotated in the separation-sliding-plane (110) by an angle of rotation (117) which, starting from a sagittal plane extending through the incisors, forms an angle <10°.

5. Device according to any of the preceding claims, wherein
a) the recesses (134; 124) are designed to be widened in depth and in extent parallel to the separation-sliding-plane (110) as compared to the 3D tooth geometry to such an extent that edge gaps (122) remain between the material of the modules (120;130) and the body tissue and or the tooth material when placing the modules on the dental arches, and/or wherein
b) selected recesses (124; 134) have bump-like or plateau-like contact points (125; 135) at a closely delimited local point, corresponding to the occlusal surface of a tooth, wherein, directly at the contact points, the 3D geometry corresponds to the 3D surface of the teeth without edge gap and the contact points are surrounded by regions with edge gap (122), and/or wherein
c) at least one module and at least one recess (124; 134) therein has an open aperture (126; 226) through the material of the module (120; 130), at least partially in the region of the occlusal surface of the tooth, wherein the recess is designed such that in the region of the opposite module (130; 120) the tooth with its occlusal surface lies partially outside the module (120; 130), , and/or wherein
d) at least one sliding surface (123; 133) of the modules (120; 130) is locally perforated by at least one recess (129) which corresponds to a tooth of the opposing dental arch, wherein this recess (129) is expanded at least by an edge gap (122) which allows the lateral movability of the tooth of the opposite side passing into the recess (129) along the separation-sliding-plane (110), and/or wherein
e) the material of the modules (120; 130) forms a bead-shaped edge (121; 131) outside the arc of the recesses (124; 134), which takes up a large volume in the oral cavity, in particular so that the soft tissues, muscles and fasciae are stretched in a defined manner by this bead edge (121; 131), and/or wherein
f) the modules (120; 130) have an approximately horseshoe shape and each comprise a dental arch, wherein the position of the device is determined by an orthogonal axis system (105; 115; 150), wherein the modules are placed against one another on the sliding surfaces (123, 133) to form the separation-sliding-plane (110) and can be displaced almost without friction in this contact position, in particular if a force in the sense of a compression force that is not exactly perpendicular to the separation-sliding-plane (110) is exerted on the two modules by the lower jaw (230) in the direction of the upper jaw (230).

6. Device according to any of the preceding claims, wherein in at least one of the modules, the dental-occlusal-plane (111), which is formed by the occlusal surfaces of the teeth of the dental arch, is oblique to the separation-sliding-plane (110), which separates the upper jaw modules and lower jaw modules and guides them slidingly against one another, wherein a clearly measurable tilting angle k (112) of more than 0.2 degrees is formed between the planes (111) and (110).

7. Device according to claim 5 or 6, when dependent on claim 5,
wherein the device in the inserted mouth stretches the lips more than 10% by the bead edges (121; 131) and the device can be covered by the lips to a maximum of 90% when seen from the front, wherein the separation-sliding-plane (110) is visible from the front and the transversal right-left axis (115), in its axial direction, can be detected by measurement.

8. Device according to any of the preceding claims, wherein at least one of the modules is designed in multiple parts, in particular in two parts, wherein the modules have recesses (124; 134) that predominantly correspond to the molars and or the canines of the respective dental arch, wherein the parts of a module together form the same separation-sliding-plane (110) when they are inserted jointly into the mouth and positioned on the dental arch.

9. Device according to claim 5 or any of the claims 6 to 8, when dependent on claim 5,
wherein the bead-shaped edge (131) of the lower jaw module (130) is more than 1 mm wider than the bead-shaped edge (121) of the upper jaw module (120) in the region of the anterior teeth and the two modules have an approximately flat transition on the outer regions towards the cheek without significant contour steps.

10. Device according to any of the preceding claims, wherein the position of the recesses (124; 134) in the modules (120; 130) is set 6-dimensionally, with 3 spatial positions and 3 spatial directions, by fitting a digital 3D model of the device into the digital 3D model of the skull in such a way that the orthogonal axes of the facial skull (205; 215; 250) are parallel to the orthogonal axes (105; 115; 150) of the separation-sliding-plane (110), wherein the right-left axes (115; 215) and the front-rear axes (105; 205) and the up-down axes (150; 250) are perpendicular to one another in the sense of orthogonality, and the position of the orthogonal axes of the skull is defined on the basis of typical osseous landmarks of the skull, in particular the facial skull in the 3D model.

11. Device according to any of the preceding claims, wherein at least one of the recesses (124; 134) comprises both a contact point (125; 135) and an aperture (126; 136), wherein the region of the contact point is designed with an edge gap (122; 132).

12. Device according to any of the preceding claims, wherein the edge gaps (122; 132) in the region of the penetrating recesses (129, 139) in the separation-sliding-plane (110; 123; 133) are worked out to such an extent that a laterally sliding movement in two directions right-left and or rear-front and combined by at least 0.3 mm, particularly preferably at least 0.4 to 1.4 mm, is possible.

13. Device according to claim 12, wherein the right-left position of the device (100) relative to the skull (200) is determinable by the right-left symmetry along the right-left axis (215) of the osseous features of the facial skull and or the right-left symmetry of the upper jaw in the anterior tooth region, and further wherein the up-down position of the device (100) along the up-down axis (250) of the skull is determinable by the intersection region of the incisors and the dental arches in the anterior tooth region, respectively, and additionally the rear-front position of the device (100) along the rear-front axis (205) is determinable by the region between the upper and lower anterior teeth.

14. Device according to claim 12 or 13, wherein in the 3D model of the device, as an extension of the volume occupied by teeth and gums, an edge gap layer (122; 132) can be produced, which is maintained, even under pressure, by individual contact points (125; 135), on which the device (100) rests on the dental arches (221; 231) in the occlusal pressure direction, in that, at the position of the contact points, the modules are formed locally without edge gap (122; 132) in positive fit with the corresponding tooth, and then this module (129; 130) 3D geometry for the dental arches is produced with edge gap and contact points and the position and location of the recesses (124; 134) resulting from the orientation, including edge gaps (122) and contact points (125).

15. Method for producing an individually developed device (100) according to any of the preceding claims for use in the mouth, in particular of a person, comprising at least one upper jaw module (120) and at least one lower jaw module (130), wherein the modules have a common separation-sliding-plane (110), wherein a digital 3D model of the device as preforms (20; 30) with its orthogonal axes (105; 115;150) is positioned in a 3D model of the head with its orthogonal axes (205; 215; 250) in such a way that the axes of the device are parallel to the axes of the head, wherein the axes of the head (205; 215; 250) result predominantly from landmarks in the region of the facial skull, and thereby the position of the recesses is defined, which are produced for the teeth of the dental arches of the upper jaw and the lower jaw in the preforms in the 3D digital model, in order to subsequently produce the modules (120; 130) with these recesses (124; 134) additively or subtractively in terms of production.

## Revendications

1. Dispositif permettant l'entraînement du système musculaire, sensori-moteur et neurophysiologique de l'articulation maxillaire et le positionnement et le guidage de la mâchoire inférieure par rapport à la mâchoire supérieure,
- ledit dispositif (100) comprenant au moins deux modules (120, 130) qui sont adjacents l'un à l'autre dans la zone d'un plan de glissement et de séparation (110) relativement grand, lisse et plat, sans que des dents ou des fissures dentaires ou des formes comparables n'entravent le déplacement latéral des modules l'un par rapport à l'autre le long du plan de glissement et de séparation (110),
- au moins l'un desdits deux modules (120, 130) étant conçu comme un module de mâchoire supérieure (120) qui comporte plusieurs creux (124) formant approximativement un arc pour les dents (221) de la mâchoire supérieure (220) ainsi qu'une première surface de glissement lisse (123) et
- au moins l'un desdits deux modules (120, 130) étant conçu comme un module de mâchoire inférieure (130) qui comporte plusieurs creux (134) pour les dents (231) de la mâchoire inférieure (220) ainsi qu'une seconde surface de glissement lisse (133),
lesdites première et seconde surfaces de glissement lisses et plates des modules (123 ; 133) présentant une bonne planéité, une faible rugosité, avec une RZ de préférence inférieure à 5 micromètres, et/ou une très faible résistance au frottement, de sorte qu'un déplacement de glissement guidé plat et lisse à glissement latéral le long du plan de glissement et de séparation (110) dans deux directions spatiales et/ou une rotation autour de l'axe perpendiculaire au plan de glissement et de séparation (110) peut avoir lieu, ledit plan de glissement et de séparation (110), conjointement avec un plan de mastication dentaire (111), définissant un premier angle k (112), ladite mâchoire inférieure et ladite mâchoire supérieure en état de contact mécanique formant le plan de mastication dentaire (111).

2. Dispositif selon la revendication 1,
a) ledit premier angle k (112) étant déterminé en vue de dessus perpendiculairement à une coupe sagittale du crâne (200), ledit premier angle étant de préférence < 10°, et/ou
b) ledit plan de glissement et de séparation (110), conjointement avec le plan de mastication dentaire (111), définissant un second angle (116) qui est déterminé en vue de dessus sur une coupe sagittale du crâne (200), ledit second angle (116) étant défini par un premier axe (202) qui coupe le point de basion (BP) parallèlement au plan de mastication dentaire (111) décalé, ainsi qu'un second axe (205) qui coupe le point de basion (BP) et le point de voûte palatale (GP).

3. Dispositif selon la revendication 2, ledit second angle (116) étant situé entre 0° et 10°.

4. Dispositif selon l'une des revendications 1 à 3 qui est tourné dans le plan de glissement et de séparation (110) d'un angle de rotation (117) qui, à partir d'un plan sagittal qui passe par les incisives, définit un angle < 10°.

5. Dispositif selon l'une des revendications précédentes,
a) lesdits creux (134 ; 124) étant élargis par rapport à la géométrie dentaire 3D en profondeur et en extension parallèlement au plan de glissement et de séparation (110) de telle sorte que, lors de la mise en place des modules sur les arcades dentaires, des espaces périphériques (122) subsistent entre le matériau des modules (120 ; 130) et le tissu corporel et/ou le matériau dentaire, et/ou
b) lesdits creux sélectionnés (124 ; 134) présentant, dans un endroit local étroitement délimité, correspondant à la surface de mastication d'une dent, des points de contact (125 ; 135) en forme de bosses ou de plateaux, ladite géométrie 3D directement au niveau des points de contact correspondant à la surface 3D des dents sans espace périphérique, et lesdits points de contact étant entourés par des zones comportant un espace périphérique (122), et/ou
c) au moins un module et au moins un creux (124 ; 134) dans celui-ci présentant au moins en partie au niveau de la surface de mastication de la dent un orifice ouvert (126 ; 226) à travers le matériau du module (120 ; 130), ledit creux étant conçu de sorte que la dent se trouve avec sa surface de mastication partiellement à l'extérieur du module (120 ; 130), dans la zone du module opposé (130 ; 120), et/ou
d) au moins une surface de glissement (123 ; 133) des modules (120 ; 130) étant localement percée par au moins un creux (129) qui correspond à une dent de l'arcade dentaire opposée, ce creux (129) étant élargi au moins par un espace périphérique (122) qui permet la mobilité latérale de la dent du côté opposé passant dans le creux (129) le long du plan de glissement et de séparation (110), et/ou
e) ledit matériau des modules (120 ; 130) à l'extérieur de l'arc des creux (124 ; 134) formant un bord en forme de bourrelet (121 ; 131) qui occupe un volume si important dans la cavité buccale, en particulier de sorte que les tissus mous, les muscles et le fascia soient étirés de manière définie par ce bord en bourrelet (121 ; 131), et/ou
f) lesdits modules (120 ; 130) présentant approximativement une forme de fer à cheval et comprenant chacun une arcade dentaire, ledit dispositif étant fixé dans sa position par un système d'axes orthogonaux (105 ; 115 ; 150), lesdits modules étant placés l'un contre l'autre au niveau des surfaces de glissement (123, 133) de manière à former le plan de glissement et de séparation (110) et pouvant se déplacer presque sans frottement dans cette position de contact, en particulier lorsqu'une force non exactement perpendiculaire au plan de glissement et de séparation (110) est exercée par la mâchoire inférieure (230) en direction de la mâchoire supérieure (230) sur les deux modules dans le sens d'une force de pression.

6. Dispositif selon l'une des revendications précédentes, dans au moins l'un des modules, ledit plan de mastication dentaire (111), qui est formé par les surfaces de mastication des dents de l'arcade dentaire, étant oblique par rapport au plan de glissement et de séparation (110), séparant le module de la mâchoire supérieure et le module de la mâchoire inférieure et les guidant par glissement l'un par rapport à l'autre, un angle d'inclinaison k (112) clairement mesurable de plus de 0,2 degré apparaissant entre les plans (111) et (110).

7. Dispositif selon la revendication 5 ou 6, si dépendante de la revendication 5, ledit dispositif, une fois en place dans la bouche, étirant les lèvres (121 ; 131) de plus de 10 % grâce auxdits bords en forme de bourrelet, et ledit dispositif pouvant être recouvert à 90 % au maximum par les lèvres, vu de face, ledit plan de glissement et de séparation (110) étant visible de face et ledit axe transversal droite-gauche (115) pouvant être détecté de manière métrologique dans sa direction axiale.

8. Dispositif selon l'une des revendications précédentes, au moins l'un des modules étant réalisé en plusieurs parties, en particulier en deux parties, lesdits modules comportant des creux (124 ; 134) qui correspondent majoritairement aux molaires et/ou aux canines de l'arcade dentaire respective, lesdites parties d'un module formant ensemble le même plan de glissement et de séparation (110) lorsqu'elles sont introduites ensemble dans la bouche et positionnées sur l'arcade dentaire.

9. Dispositif selon la revendication 5 ou l'une des revendications 6 à 8, si dépendante de revendication 5, ledit bord en forme de bourrelet (131) du module de mâchoire inférieure (130) étant, dans la zone des dents antérieures, plus large de plus de 1 mm que le bord en forme de bourrelet (121) du module de mâchoire supérieure (120) et lesdits deux modules présentant, au niveau des zones externes vers les joues, une transition approximativement plane sans sauts de contour significatifs.

10. Dispositif selon l'une des revendications précédentes, ladite position des creux (124 ; 134) dans les modules (120 ; 130) étant réglée en 6 dimensions, avec 3 positions spatiales et 3 directions spatiales, en ajustant un modèle numérique 3D du dispositif dans le modèle numérique 3D du crâne de sorte que les axes orthogonaux du crâne facial (205 ; 215 ; 250) soient parallèles aux axes orthogonaux (105 ; 115 ; 150) du plan de glissement et de séparation (110), lesdits axes droite-gauche (115 ; 215), lesdits axes avant-arrière (105 ; 205) et lesdits axes haut-bas (150 ; 250) étant perpendiculaires les uns aux autres dans le sens de l'orthogonalité, et ladite position des axes orthogonaux du crâne étant définie à l'aide de repères osseux typiques du crâne, en particulier du crâne facial dans le modèle 3D.

11. Dispositif selon l'une des revendications précédentes, au moins l'un des creux (124 ; 134) comprenant à la fois un point de contact (125 ; 135) et un orifice (126 ; 136), l'environnement du point de contact étant réalisé avec un espace périphérique (122 ; 132).

12. Dispositif selon l'une des revendications précédentes, lesdits espaces périphériques (122 ; 132) au voisinage des creux percés (129, 139) dans le plan de glissement et de séparation (110 ; 123 ; 133) étant élaborés de manière à permettre un déplacement de glissement latéral dans deux directions, droite-gauche et/ou arrière-avant, et combiné d'au moins 0,3 mm, de préférence d'au moins 0,4 à 1,4 mm.

13. Dispositif selon la revendication 12, ladite position droite-gauche du dispositif (100) par rapport au crâne (200) pouvant être déterminée par la symétrie droite-gauche le long de l'axe droite-gauche (215) des caractéristiques osseuses du crâne facial et/ou de la symétrie droite-gauche de la mâchoire supérieure dans la zone des dents antérieures, et en outre la position haut-bas du dispositif (100) le long de l'axe haut-bas (250) du crâne pouvant être déterminée par la zone de chevauchement des incisives respectivement des arcades dentaires dans la zone des dents antérieures, et en outre, la position avant-arrière du dispositif (100) le long de l'axe avant-arrière (205) étant déterminée par la zone entre les dents antérieures supérieures et inférieures.

14. Dispositif selon la revendication 12 ou 13, une couche d'espace périphérique (122 ; 132) pouvant être générée dans le modèle 3D du dispositif en tant qu'extension du volume occupé par les dents et les gencives, laquelle est maintenue par des points de contact isolés (125 ; 135) même sous pression, sur lesquels le dispositif (100) repose sur les arcades dentaires (221 ; 231) dans le sens de la pression de mastication, en formant localement les modules sans espace périphérique (122 ; 132) au niveau de l'emplacement des points de contact, en liaison de forme avec la dent correspondante, puis ces modules (129 ; 130) de géométrie 3D étant réalisés avec un espace périphérique et des points de contact et la position et l'orientation des creux (124 ; 134) résultant de l'orientation, y compris l'espace périphérique (122) et les points de contact (125) pour les arcades dentaires.

15. Procédé de fabrication d'un dispositif personnalisé (100) selon l'une des revendications précédentes, destiné à être utilisé dans la bouche, en particulier d'une personne, qui se compose d'au moins un module de mâchoire supérieure (120) et d'au moins un module de mâchoire inférieure (130), lesdits modules présentant un plan de glissement et de séparation commun (110), un modèle numérique 3D du dispositif étant positionné sous forme de préformes (20 ; 30) avec ses axes orthogonaux (105 ; 115 ; 150) dans un modèle 3D de la tête avec ses axes orthogonaux (205 ; 215 ; 250) de sorte que les axes du dispositif soient parallèles aux axes de la tête, lesdits axes de la tête (205 ; 215 ; 250) étant principalement déterminés à partir de repères situés dans la zone du crâne facial, définissant ainsi la position des creux qui sont créés pour les dents des arcades dentaires de la mâchoire supérieure et de la mâchoire inférieure dans les préformes du modèle numérique 3D, afin de produire ensuite les modules (120 ; 130) avec ces creux (124 ; 134) par des techniques de fabrication additives ou soustractives.
